(19) Europäisches Patentamt
European Patent Office
Office européen des brevets

(11) EP 3 988 174 A1

(12) EUROPEAN PATENT APPLICATION
published in accordance with Art. 153(4) EPC

(43) Date of publication:
27.04.2022 Bulletin 2022/17

(21) Application number: 20827091.8

(22) Date of filing: 17.06.2020

(51) International Patent Classification (IPC):
*A61P 35/00* (2006.01) *C07H 19/14* (2006.01)
*A61K 31/7064* (2006.01)

(52) Cooperative Patent Classification (CPC):
A61K 31/7064; A61P 35/00; C07H 19/14

(86) International application number:
PCT/JP2020/023661

(87) International publication number:
WO 2020/255979 (24.12.2020 Gazette 2020/52)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 18.06.2019 JP 2019113172
29.08.2019 JP 2019157375

(71) Applicant: Taiho Pharmaceutical Co., Ltd.
Chiyoda-ku, Tokyo 101-8444 (JP)

(72) Inventors:
- MIYAKOSHI Hitoshi
Tsukuba-shi, Ibaraki 300-2611 (JP)
- TANAKA Nozomu
Tsukuba-shi, Ibaraki 300-2611 (JP)
- KOBAYAKAWA Yu
Tsukuba-shi, Ibaraki 300-2611 (JP)

(74) Representative: Schiener, Jens
Wächtershäuser & Hartz
Patentanwaltspartnerschaft mbB
Weinstraße 8
80333 München (DE)

# (54) NOVEL CARBONATE COMPOUND HAVING PYRROLOPYRIMIDINE SKELETON OR PHARMACEUTICALLY ACCEPTABLE SALT THEREOF

(57) There is provided a novel carbonate compound of a nucleoside having a pyrrolopyrimidine skeleton having an excellent antitumor effect, or a pharmaceutically acceptable salt thereof and a method for preventing and/or treating a tumor in combination with an alkylating agent and/or a radiation therapy. According to one aspect of the present invention, there is provided a compound represented by the following general formula (1) :

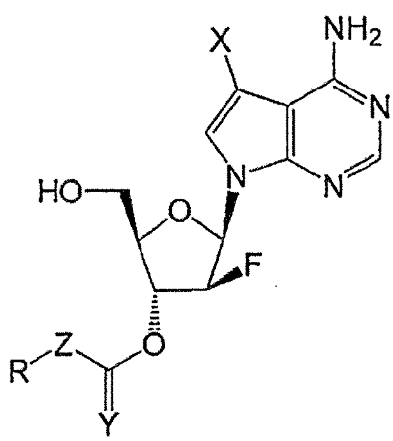

(1)

or a pharmaceutically acceptable salt thereof, and a method for preventing and/or treating a tumor in combination with an alkylating agent and/or a radiation therapy.

EP 3 988 174 A1

Fig. 1

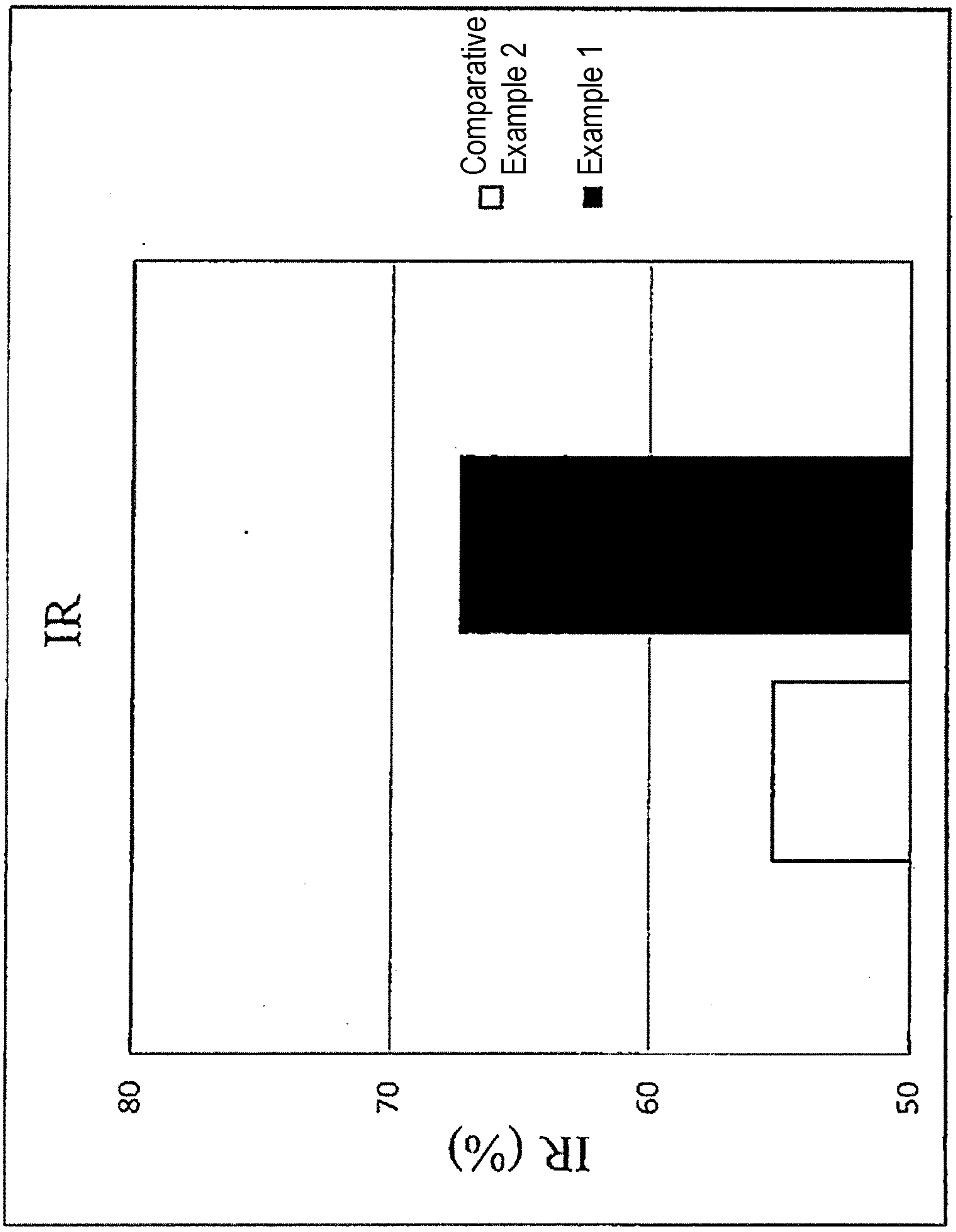

## Description

### Technical Field

The present invention relates to a novel carbonate compound having a pyrrolopyrimidine skeleton having an excellent antitumor effect, or a pharmaceutically acceptable salt thereof. The present invention also relates to combination use of a novel carbonate form having a pyrrolopyrimidine skeleton or a pharmaceutically acceptable salt thereof and an alkylating agent and/or a radiation therapy.

### Background Art

Tumors characterized by abnormal proliferation of cells are intractable diseases for which effective treatments are still desired. For proliferation of tumor cells, biosynthesis of nucleic acids is essential. The compounds which mimic molecules involved in biosynthesis of nucleic acids have been energetically developed as nucleic acid antimetabolites disturbing nucleic acid metabolism of tumors.

However, even if compounds having a highly effective nucleic acid antimetabolic action are found out, most of the compounds may have problems in view of toxicity or pharmacokinetics and cannot be used as clinically useful medical agents. To overcome the problems and maximize the efficacy which these compounds inherently have, the compounds are sometimes converted into prodrugs. If a compound having an antitumor effect is converted into a prodrug, it is expected that the pharmacokinetics of the compound and selectivity of action thereof on a cancer tissue can be improved. However, despite such expectations, it is not easy to convert a compound having a highly effective nucleic acid antimetabolic action into a prodrug serving as a clinically useful medical agent.

In order to solve the aforementioned problems, it has been tried to convert a number of nucleic acid derivatives into prodrugs by introducing a carbonate or an ester into, e.g., a free hydroxy group or an amino group. For example, capecitabine is a prodrug obtained by introducing a n-pentyloxycarbonyl group into the amino group at the 4-position of 5-fluorouracil (5-FU) (Non Patent Literature 1). Also, it is known to obtain a compound by introducing a carbonyl into the hydroxy group of the 2'-postion of a nucleic acid (database name: REGISTRY, CAS No.145388-61-0) and a compound by introducing a carbonyl into the 3'-position of a hydroxy group (Patent Literature 1, compound 28). However, a carbonate compound of a nucleotide having a pyrrolopyrimidine skeleton has not yet been known.

In the meantime, as nucleic acid derivatives having a pyrrolopyrimidine skeleton, deoxyribonucleoside derivatives having a 4-amino-5-halogeno-7H-pyrrolo[2,3-d]pyrimidine skeleton, have been reported (Non Patent Literatures 3, 4, 5, 6). Of them, deoxyribonucleoside derivatives having an iodine at the 5-position have been reported (Non Patent Literature 3, compound 13 of Patent Literature 2). However, there are no descriptions suggesting carbonate forms corresponding to these nucleoside derivatives.

As a compound suggesting a carbonate form corresponding to a nucleoside derivative, deoxyribonucleoside derivatives having a carbonate or a thiocarbonate at the 3'-position have been reported (compound 7 of Non Patent Literature 7, compound 28 of Patent Literature 1). However, these literatures neither describe a deoxynucleoside derivative compound having a halogen atom at the 5-position of a pyrrolo[2,3-d]pyrimidine skeleton nor suggest a prodrug of the corresponding nucleoside derivative.

### Citation List

#### Patent Literature

Patent Literature 1: WO 2004/041203
Patent Literature 2: WO 2013/009735

#### Non Patent Literature

Non Patent Literature 1: Drug Metab Dispos. 2002 Nov; 30(11): 1221-1229
Non Patent Literature 2: J. Org. Chem. 1999, 64(22), 8319-8322
Non Patent Literature 3: Acta Cryst. 2014, E70, o120
Non Patent Literature 4: ChemMedChem, 2013, 8, 832-846 Non Patent Literature 5: Bioorg. Med. Chem, 20, 2012, 5202-5214

Non Patent Literature 6: J Med Chem 2016; 59(14): 6860-6877
Non Patent Literature 7: Synthesis 1992, (5), 477-481

Summary of Invention

Technical Problem

The present invention provides a compound having a pyrrolopyrimidine skeleton, which has more excellent safety than existing compounds having a pyrrolopyrimidine skeleton and exerts a high antitumor effect. The present invention also provides a combination therapy of the compound having such a pyrropyrimidine skeleton and an alkylating agent and/or a radiation therapy.

Solution to Problem

The present inventors found deoxyribonucleoside derivatives having a pyrrolo[2,3-d]pyrimidine skeleton represented by general formula (1). These compounds have a predetermined halogen atom at the 5-position and a carbonate at the 3'-position.
More specifically, an embodiment of the present invention includes the followings.

[1] A compound represented by the following general formula (1):

[Formula 1]

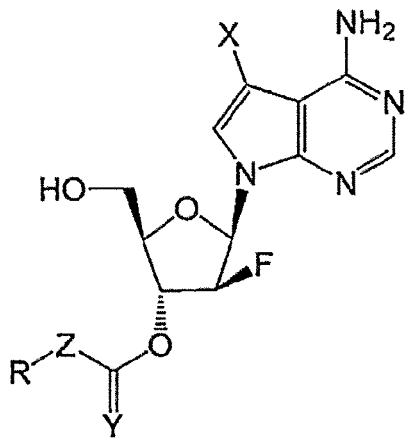
(1)

wherein

X represents a chlorine atom, a bromine atom or an iodine atom,
Y represents an oxygen atom or a sulfur atom,
Z represents an oxygen atom or a sulfur atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent,

or a pharmaceutically acceptable salt thereof.
[2] The compound or a pharmaceutically acceptable salt thereof according to [1], wherein

R represents a linear C1-C6 alkyl group that may have 1 to 3 substituents which are selected from C1-C6 alkyl groups, C1-C6 alkenyl groups, C1-C6 alkynyl groups, C1-C4 alkoxy groups, C1-C4 haloalkyl groups, hydroxy groups, halogen atoms or aromatic hydrocarbon groups;
a C3-C10 cycloalkyl group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic

hydrocarbon groups;
a C4-C10 cycloalkenyl group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic hydrocarbon groups; or
a 4 to 10-membered saturated heterocyclic group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic hydrocarbon groups; or
a 5 to 10-membered unsaturated heterocyclic group that may have 1 to 3 substituents which are selected from C1-C6 alkyl groups, C1-C6 alkenyl groups, C1-C6 alkynyl groups, C1-C4 alkoxy groups, hydroxy groups, or halogen atoms, or aromatic hydrocarbon groups.

[3] The compound or a pharmaceutically acceptable salt thereof according to [2], wherein

R represents a linear C1-C6 alkyl group that may have 1 to 3 substituents which are selected from C1-C6 alkyl groups, C1-C4 alkoxy groups, C1-C4 haloalkyl groups, halogen atoms or phenyl groups;
a C3-C10 cycloalkyl group that may have 1 to 3 substituents which are selected from C1-C6 alkyl groups, C1-C4 alkoxy groups, halogen atoms or phenyl groups;
a C4-C10 cycloalkenyl group that may have 1 to 3 substituents which are selected from C1-C6 alkyl groups, C1-C4 alkoxy groups, halogen atoms or phenyl groups; or
4 to 10-membered saturated heterocyclic group that may have 1 to 3 substituents which are selected from C1-C6 alkyl groups, C1-C4 alkoxy groups, halogen atoms or phenyl groups.

[4] The compound or a pharmaceutically acceptable salt thereof according to [1], wherein X represents a bromine atom or an iodine atom.
[5] The compound or a pharmaceutically acceptable salt thereof according to [4], wherein X represents an iodine atom.
[6] The compound or a pharmaceutically acceptable salt thereof according to [5], wherein Y represents an oxygen atom.
[7] The compound or a pharmaceutically acceptable salt thereof according to [6], wherein

R represents a linear C1-C3 alkyl group that may have 1 to 3 substituents which are selected from C1-C2 alkyl groups, C1-C2 alkoxy groups, C1-C2 haloalkyl groups, fluorine atoms or chlorine atoms;
a C3-C7 cycloalkyl group that may have 1 to 2 substituents which are selected from C1-C2 alkyl groups, or C1-C2 alkoxy groups, fluorine atoms or chlorine atoms;
a C4-C6 cycloalkenyl group that may have 1 to 2 substituents which are selected from C1-C2 alkyl groups, or C1-C2 alkoxy groups, fluorine atoms or chlorine atoms; or
a 4 to 6-membered saturated heterocyclic group that may have 1 to 3 substituents which are selected from C1-C2 alkyl groups, C1-C2 alkoxy groups, fluorine atoms or chlorine atoms.

[8] The compound or a pharmaceutically acceptable salt thereof according to any one of [1] to [7], wherein the compound is selected from the group consisting of the following compounds:

O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-cyclopentyl carbonothioate;
O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-isopropyl carbonothioate;
O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl pentan-3-yl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopent-3-en-1-yl carbonate; and
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl (bicyclo[2.2.1]heptan-2-yl) carbonate.

[9] The compound or a pharmaceutically acceptable salt thereof according to any one of [1] to [8], wherein the compound is selected from the group consisting of the following compounds:

(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate; and
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate.

[10] An antitumor agent comprising the compound or a pharmaceutically acceptable salt thereof according to any one of [1] to [9], as an active ingredient.
[11] A pharmaceutical composition comprising the compound or a pharmaceutically acceptable salt thereof according to any one of [1] to [9] and a pharmaceutically acceptable carrier.
[12] The antitumor agent according to [10] or the pharmaceutical composition according to [11], formulated as an oral preparation or an injection.
[13] A method for preventing and/or treating a tumor, comprising administering the compound or a pharmaceutically acceptable salt thereof according to any one of [1] to [9] to a subject in need thereof.
[14] A method for preventing and/or treating a tumor, comprising administering the compound or a pharmaceutically acceptable salt thereof according to any one of [1] to [9], formulated as an oral preparation or an injection, to a subject in need thereof.
[15] The compound or a pharmaceutically acceptable salt thereof according to any one of [1] to [9] for use as a pharmaceutical composition.
[16] The compound or a pharmaceutically acceptable salt thereof according to any one of [1] to [9] for use in prevention and/or treatment of a tumor.
[17] The compound or a pharmaceutically acceptable salt thereof according to any one of [1] to [9], formulated as an oral preparation or an injection, for use in prevention and/or treatment of a tumor.
[18] Use of the compound or a pharmaceutically acceptable salt thereof according to any one of [1] to [9], for producing an antitumor agent.
[19] Use of the compound or a pharmaceutically acceptable salt thereof according to any one of [1] to [9], for producing a pharmaceutical composition.
[20] Use of the compound or a pharmaceutically acceptable salt thereof according to any one of [1] to [9], for producing an antitumor agent, formulated as an oral preparation or an injection.
[21] The antitumor agent according to [10], the method according to [13] or [14], the compound or a pharmaceutically acceptable salt thereof according to any one of [15] to [17], or the use according to any one of [18] to [20], wherein the tumor is selected from the group consisting of head and neck cancer (e.g. oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.
[22] The antitumor agent, method, use, compound or a pharmaceutically acceptable salt thereof according to [21], wherein the tumor is a brain tumor.
[23] A compound represented by the following general formula (2)

[Formula 2]

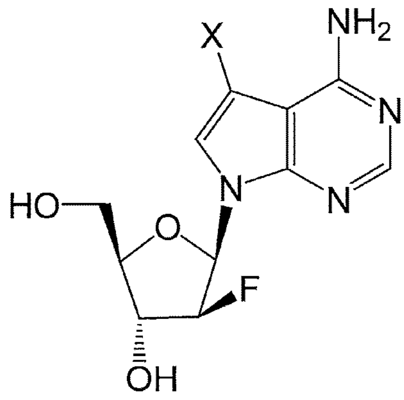

(2)

wherein

X represents a chlorine atom, a bromine atom or an iodine atom,

or a pharmaceutically acceptable salt thereof.

[24] An antitumor agent or pharmaceutical composition comprising the compound or a pharmaceutically acceptable salt thereof according to [23], as active ingredients, and a pharmaceutically acceptable carrier.

[25] The antitumor agent or pharmaceutical composition according to [24], formulated as an oral preparation or an injection.

[26] A method for preventing and/or treating a tumor, comprising administering the compound or a pharmaceutically acceptable salt thereof according to [23].

[27] The compound or a pharmaceutically acceptable salt thereof according to [23], for preventing and/or treating a tumor.

[28] Use of the compound or a pharmaceutically acceptable salt thereof according to [23], for producing a pharmaceutical composition or an antitumor agent.

[29] The antitumor agent or pharmaceutical composition according to [24] or [25], the method for preventing and/or treating a tumor according to [26], the compound or a pharmaceutically acceptable salt thereof according to [27] or use according to [28], wherein the tumor is selected from the group consisting of head and neck cancer (e.g., oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

[30] The antitumor agent or pharmaceutical composition, method for preventing and/or treating a tumor, compound or a pharmaceutically acceptable salt thereof or use according to [29], wherein the tumor is a brain tumor.

[31] The antitumor agent or pharmaceutical composition consisting of the compound or a pharmaceutically acceptable salt thereof according to any one of [1] to [9] and [23], which is used in combination with an alkylating agent.

[32] An enhancer of an antitumor effect comprising the compound or a pharmaceutically acceptable salt thereof according to any one of [1] to [9] and [23], for enhancing an antitumor effect of an alkylating agent.

[33] An antitumor agent comprising the compound or a pharmaceutically acceptable salt thereof according to any one of [1] to [9] and [23], for treating a cancer patient having received administration of an alkylating agent.

[34] A method for preventing and/or treating a tumor, comprising administering the compound or a pharmaceutically acceptable salt thereof according to any one of [1] to [9] and [23], which is used in combination with an alkylating agent.

[35] A method for enhancing an antitumor effect of an alkylating agent, comprising administering the compound or a pharmaceutically acceptable salt thereof according to any one of [1] to [9] and [23] to a patient.

[36] A method for preventing and/or treating a tumor, comprising administering an antitumor agent consisting of the compound or a pharmaceutically acceptable salt thereof according to any one of [1] to [9] and [23], and an alkylating agent.

[37] Use of the compound or a pharmaceutically acceptable salt thereof according to any one of [1] to [9] and [23], for treating a tumor, which is administered in combination with an alkylating agent.

[38] Use of the compound or a pharmaceutically acceptable salt thereof according to any one of [1] to [9] and [23], for enhancing an antitumor effect of an alkylating agent, which is administered in combination with an alkylating agent.

[39] Use of the compound or a pharmaceutically acceptable salt thereof according to any one of [1] to [9] and [23], for treating a tumor, characterized by treating a cancer patient having received administration of an alkylating agent.
[40] Use of the compound or a pharmaceutically acceptable salt thereof according to any one of [1] to [9] and [23], for enhancing an antitumor effect of an alkylating agent, which treats a cancer patient having received administration of an alkylating agent.
[41] Use of the compound or a pharmaceutically acceptable salt thereof according to any one of [1] to [9] and [23], for producing an antitumor agent, wherein the antitumor agent is administered in combination with an alkylating agent.
[42] Use of the compound or a pharmaceutically acceptable salt thereof according to any one of [1] to [9] and [23], for producing an enhancer of an antitumor effect of an alkylating agent, wherein the enhancer of an antitumor effect is administered in combination with the alkylating agent.
[43] Use of the compound or a pharmaceutically acceptable salt thereof according to any one of [1] to [9] and [23], for producing an antitumor agent, wherein the antitumor agent is administered to a cancer patient having received administration of an alkylating agent or a cancer patient to receive administration of an alkylating agent.
[44] Use of the compound or a pharmaceutically acceptable salt thereof according to any one of [1] to [9] and [23], for producing an enhancer of an antitumor effect of an alkylating agent, wherein the enhancer of an antitumor effect is administered to a cancer patient having received administration of an alkylating agent or a cancer patient to receive administration of an alkylating agent.
[45] The antitumor agent according to [31] or [33], the enhancer of an antitumor effect according to [32], the method according to any one of [34] to [36], or the use according to any one of [37] to [44], wherein the alkylating agent is temozolomide.
[46] The antitumor agent according to [31] or [33], the enhancer of an antitumor effect according to [32], the method according to any one of [34] to [36], or the use according to any one of [37] to [44], which is used in combination with a radiation therapy in addition to the alkylating agent.
[47] An antitumor agent or a pharmaceutical composition consisting of the compound or a pharmaceutically acceptable salt thereof according to any one of [1] to [9] and [23], which is used in combination with a radiation therapy.
[48] An enhancer of an antitumor effect comprising the compound or a pharmaceutically acceptable salt thereof according to any one of [1] to [9] and [23], for enhancing an antitumor effect of a radiation therapy.
[49] An antitumor agent comprising the compound or a pharmaceutically acceptable salt thereof according to any one of [1] to [9] and [23], for treating a cancer patient having received a radiation therapy.
[50] A method for preventing and/or treating a tumor, comprising the compound or a pharmaceutically acceptable salt thereof according to any one of [1] to [9] and [23], which is used in combination with a radiation therapy.
[51] A method for enhancing an antitumor effect of a radiation therapy, comprising administering the compound or a pharmaceutically acceptable salt thereof according to any one of [1] to [9] and [23] to a patient.
[52] A method for preventing and/or treating a tumor, comprising administering an antitumor agent consisting of the compound or a pharmaceutically acceptable salt thereof according to any one of [1] to [9] and [23] to a cancer patient having received a radiation therapy.
[53] Use of the compound or a pharmaceutically acceptable salt thereof according to any one of [1] to [9] and [23], for treating a tumor, which is used in combination with a radiation therapy.
[54] Use of the compound or a pharmaceutically acceptable salt thereof according to any one of [1] to [9] and [23], for enhancing an antitumor effect, which is used in combination with a radiation therapy.
[55] Use of the compound or a pharmaceutically acceptable salt thereof according to any one of [1] to [9] and [23], for treating a tumor, which treats a cancer patient having received a radiation therapy.
[56] Use of the compound or a pharmaceutically acceptable salt thereof according to any one of [1] to [9] and [23], for enhancing an antitumor effect, which treats a cancer patient having received a radiation therapy.
[57] Use of the compound or a pharmaceutically acceptable salt thereof according to any one of [1] to [9] and [23], for producing an antitumor agent, wherein the antitumor agent is used in combination with a radiation therapy.
[58] Use of the compound or a pharmaceutically acceptable salt thereof according to any one of [1] to [9] and [23], for producing an enhancer of an antitumor effect, wherein the enhancer of an antitumor effect is used in combination with a radiation therapy.
[59] Use of the compound or a pharmaceutically acceptable salt thereof according to any one of [1] to [9] and [23], for producing an antitumor agent, wherein the antitumor agent is administered to a cancer patient having received a radiation therapy.
[60] Use of the compound or a pharmaceutically acceptable salt thereof according to any one of [1] to [9] and [23], for producing an enhancer of an antitumor effect, wherein the enhancer of an antitumor effect is administered to a cancer patient having received a radiation therapy.
[61] The antitumor agent according to [47] or [49], the enhancer of an antitumor effect according to [48], the method according to any one of [50] to [52] or the use according to any one of [53] to [60], which is used in combination with an alkylating agent in addition to the radiation therapy.

[62] The antitumor agent, enhancer of an antitumor effect, method or use according to [61], wherein the alkylating agent is temozolomide.

[63] A combination comprising: a pharmaceutical composition comprising the compound or a pharmaceutically acceptable salt thereof according to any one of [1] to [9] and [23]; and an alkylating agent.

[64] A combination drug comprising the compound or a pharmaceutically acceptable salt thereof according to any one of [1] to [9] and [23] and an alkylating agent.

[65] The combination according to [63] or the combination drug according to [64], which is used in combination with a radiation therapy.

[66] The combination according to [63] or the combination drug according to [64], formulated as an oral preparation or an injection.

Advantageous Effects of Invention

The novel carbonate compound of a nucleoside having a pyrrolopyrimidine skeleton of the present invention or a pharmaceutically acceptable salt thereof is useful as an antitumor agent having excellent safety and exerting a high antitumor effect. Also, the novel carbonate compound of the present invention or a pharmaceutically acceptable salt thereof, if it is used in combination with an alkylating agent and/or a radiation therapy, shows an excellent combinational effect.

Brief Description of Drawings

[Figure 1] Figure 1 shows IR (vertical axis) on the final day of evaluation when the Comparative Example 2-compound and Example 1-compound were administered to BALB/cA Jcl-nu/nu mice transplanted with a human brain tumor cell line (U-87MG).

[Figure 2] Figure 2 shows BWC (vertical axis) on individual evaluation days (horizontal axis) when the Comparative Example 2-compound and Example 1-compound were administered to BALB/cA Jcl-nu/nu mice transplanted with a human brain tumor cell line (U-87MG). O represents a control group of mice (not treated); ■ represents a group receiving the Comparative Example 2-compound; and ▲ represents a group receiving the Example 1-compound.

[Figure 3] Figure 3 shows effect indexes on Day 6 after administration of the Comparative Example 2-compound and the Example 5-compound to BALB/cA Jcl-nu/nu mice transplanted with a human brain tumor cell line (U-87MG).

[Figure 4] Figure 4 shows RTV (vertical axis) on individual evaluation days (horizontal axis) when the Comparative Example 2-compound was administered to BALB/cA Jcl-nu/nu mice transplanted with a human hematopoietic organ tumor cell line (MV-4-11). ○ represents a control group (not treated); and ■ represents a group receiving the Comparative Example 2-compound.

[Figure 5] Figure 5 shows BWC (vertical axis) on individual evaluation days (horizontal axis) when the Comparative Example 2-compound was administered to BALB/cA Jcl-nu/nu mice transplanted with a human hematopoietic organ tumor cell line (MV-4-11). ○ represents a control group (not treated); and ■ represents a group receiving the Comparative Example 2-compound.

[Figure 6] Figure 6 shows RTV (vertical axis) on individual evaluation days (horizontal axis) when the Example 1-compound was administered to BALB/cA Jcl-nu/nu mice transplanted with a human hematopoietic organ tumor cell line (MV-4-11). ○ represents a control group (not treated); and ▲ represents a group receiving the Example 1-compound.

[Figure 7] Figure 7 shows BWC (vertical axis) on individual evaluation days (horizontal axis) when the Example 1-compound was administered to BALB/cA Jcl-nu/nu mice transplanted with a human hematopoietic organ tumor cell line (MV-4-11). ○ represents a control group (not treated); and ▲ represents a group receiving the Example 1-compound.

[Figure 8] Figure 8 shows changes of lymphocytes (LYMPH) on the final evaluation day of groups of BALB/cA Jcl-nu/nu mice transplanted with a human hematopoietic organ tumor cell line (MV-4-11) receiving the Example 1-compound and the Comparative Example 2 compound, respectively, relative to a control group (not treated).

[Figure 9] Figure 9 shows cell viability of a human brain tumor cell line (U-87MG) treated with the Comparative Example 2-compound and a radiation therapy singly or in combination.

[Figure 10] Figure 10 shows cell viability of a human brain tumor cell line (U-87MG) treated with the Comparative Example 2-compound and temozolomide (TMZ) singly or in combination.

Description of Embodiments

Now, the present invention will be more specifically described. It should not be construed that the scope of the present invention is limited to the embodiments described below.

<Definitions of terms>

The terms used in the specification have meanings commonly used in the technical field, unless otherwise specified.

In the specification, the expression "CA-CB" in connection with the description of a chemical group means that the number of carbon atoms is from A to B. For example, "C1-C6 alkyl group" means an alkyl group having 1 to 6 carbon atoms; "C6-C14 aromatic hydrocarbon oxy group" means an oxy group to which an aromatic hydrocarbon group having 6 to 14 carbon atoms is bound. The expression "A to B-membered" means that the number of atoms (members) constituting a ring is from A to B. More specifically, "4 to 10-membered saturated heterocyclic group" means a saturated heterocyclic group having a ring constituted of 4 to 10 members.

In the specification, the "substituent", if not explicitly stated, is, e.g., a hydrogen atom, a halogen atom, a hydroxy group, a carboxyl group, an alkyl group, an alkenyl group, an alkynyl group, an alkoxy group, a cycloalkyl group, a cycloalkenyl group, a cycloalkyl-alkyl group, a haloalkyl group, an alkoxyalkyl group, an aromatic hydrocarbon group, an aralkyl group, a saturated heterocyclic group, an unsaturated heterocyclic group, or the like but examples of the substituent are not limited to these. When a substituent as mentioned above is present, if not explicitly stated, the number of the substituents is typically 1, 2 or 3 and preferably 1 or 2.

In the specification, the "halogen atom" is specifically, a chlorine atom, a bromine atom, a fluorine atom or an iodine atom, preferably a chlorine atom, a fluorine atom or a bromine atom and more preferably a chlorine atom and a fluorine atom.

In the specification, the "alkyl group" refers to a linear or branched saturated hydrocarbon group. Examples thereof include, but are not limited to, C1-C6 alkyl groups such as a methyl group, an ethyl group, a n-propyl group, an isopropyl group, a n-butyl group, a 1-methylpropyl group, an isobutyl group, a tert-butyl group, a 2,2-dimethylpropyl group, a n-pentyl group, an isopentyl group, a sec-pentyl group, a 1-ethylpropyl group, a 1,1-dimethylpropyl group, a 1,2-dimethylpropyl group, a 2,2-dimethylpropyl group, a n-hexyl group, an isohexyl group, a 3-methylpentyl group, a 2,3-dimethylbutyl group, a 2,2-dimethylbutyl group and a 3,3-dimethylbutyl group. In an embodiment of the present invention, the alkyl group is preferably a methyl group, an ethyl group, a n-propyl group, an isopropyl group, a n-butyl group, a 1-methylpropyl group, an isobutyl group, a tert-butyl group, a 2,2-dimethylpropyl group or a 1-ethyl propyl group and more preferably a methyl group, an ethyl group, a n-propyl group, an isopropyl group or a 1-ethylpropyl group.

In the specification, the "alkenyl group" refers to a linear or branched unsaturated hydrocarbon group having at least one (for example, 1 to 2, preferably 1) double bond. Examples thereof include, but are not limited to, C2-C6 alkenyl groups such as a vinyl group, an allyl group, a 1-propenyl group, a 2-methyl-2-propenyl group, an isopropenyl group, a 1-, 2- or 3-butenyl group, a 2-, 3- or 4-pentenyl group, a 2-methyl-2-butenyl group, a 3-methyl-2-butenyl group and a 5-hexenyl group. In an embodiment of the present invention, the alkenyl group is preferably a vinyl group, an allyl group, a 1-propenyl group or a 2-methyl-2-propenyl group.

In the specification, the "alkynyl group" refers to a linear or branched unsaturated hydrocarbon group having at least one (for example, 1 to 2, preferably 1) triple bond. Examples thereof include, but are not limited to, C2-C6 alkynyl groups such as an ethynyl group, a 1- or 2-propynyl group, a 1-, 2- or 3-butynyl group and a 1-methyl-2-propynyl group. In an embodiment of the present invention, the alkynyl group is preferably an ethynyl group or a 2-propynyl group.

In the specification, the "alkoxy group" refers to an oxy group having an alkyl group as mentioned above. Examples thereof include, but are not limited to, C1-C6 alkoxy groups such as a methoxy group, an ethoxy group, a n-propoxy group, an isopropoxy group, a n-butoxy group, an isobutoxy group, a sec-butoxy group, a tert-butoxy group, a pentyloxy group, an isopentyloxy group and an hexyloxy group. In an embodiment of the present invention, the alkoxy group is preferably a methoxy group or an ethoxy group and more preferably a methoxy group.

In the specification, the "cycloalkyl group" refers to a monocyclic or polycyclic saturated hydrocarbon group. Examples thereof include, but are not limited to, C3-C10 cycloalkyl groups such as a cyclopropyl group, a cyclobutyl group, a cyclopentyl group, a cyclohexyl group, a cycloheptyl group, a cyclodecyl group, a [2.2.0]bicyclohexyl group, a [2.2.1]bicycloheptanyl group and a [3.1.1]bicycloheptanyl group. In an embodiment of the present invention, the cycloalkyl group is preferably a cyclopropyl group, a cyclobutyl group, a cyclopentyl group, a cyclohexyl group, a cycloheptyl group, a [2.2.1]bicycloheptanyl group or a [3.1.1]bicycloheptanyl group and particularly preferably a cyclobutyl group or a cyclopentyl group.

In the specification, the "cycloalkenyl group" refers to a monocyclic or polycyclic unsaturated hydrocarbon group having at least one (for example, 1 to 2, preferably 1) carbon-carbon double bond. Examples thereof include, but are not limited to, C4-C10 cycloalkenyl groups such as a cyclobutenyl group, a cyclopentenyl group, a cyclohexenyl group,

a cycloheptenyl group, a cyclodecenyl group and a 1H, 2H, 3H-indene group. In an embodiment of the present invention, the cycloalkenyl group is preferably a cyclobutenyl group, a cyclopentenyl group or a 1H, 2H, 3H-indene group and particularly preferably a cyclopentenyl group.

In the specification, the "cycloalkyl-alkyl group" refers to an alkyl group as mentioned above having at least one cycloalkyl group as mentioned above. Examples thereof include, but are not limited to, C3-C10 cycloalkyl-C1-C4 alkyl groups such as a cyclopropylmethyl group, a cyclobutylmethyl group, a cyclopentyl methyl group, a cyclohexylmethyl group and a cycloheptylmethyl group.

In the specification, the "haloalkyl group" refers to an alkyl group as mentioned above having at least one (preferably 1 to 10, more preferably 1 to 3) halogen atom. Examples thereof include, but are not limited to, C1-C6 haloalkyl groups such as a fluoromethyl group, a difluoromethyl group, a trifluoromethyl group, a trichloromethyl group, a 1-fluoroethyl group, a 2-fluoroethyl group, a 1,1-difluoroethyl group, a 1,1,1-trifluoroethyl group, a 2,2,2-trichloroethyl group, a 1-(fluoromethyl)-2-fluoroethyl group, a monofluoro-n-propyl group, a 1,1,1-trifluoro-n-propyl group, a perfluoro-n-propyl group and a perfluoroisopropyl group. In an embodiment of the present invention, the haloalkyl group is preferably a C1-C4 alkyl group having 1 to 3 chlorine or fluorine atoms, more specifically, a trifluoromethyl group, a 1-fluoroethyl group, a 1,1-difluoroethyl group, a 1,1,1-trifluoroethyl group, a 2,2,2-trichloroethyl group or a 1-(fluoromethyl)-2-fluoroethyl group and more preferably a 2,2,2-trichloroethyl group or a 1-(fluoromethyl)-2-fluoroethyl group.

In the specification, the "alkoxyalkyl group" refers to an alkyl group as mentioned above having at least one alkoxy group as mentioned above. Examples thereof include, but are not limited to, C1-C6 alkoxy-C1-C6 alkyl groups such as a methoxymethyl group, an ethoxyethyl group, a methoxyethyl group and a methoxypropyl group.
In an embodiment of the present invention, the alkoxyalkyl group is preferably a C1-C3 alkoxy-C1-C4 alkyl group, more specifically a methoxymethyl group or an ethoxymethyl group and more preferably a methoxymethyl group.

In the specification, the "aromatic hydrocarbon group" refers to a cyclic substituent composed of carbon atoms and hydrogen atoms and having an unsaturated bond, which is a monocyclic or polycyclic aromatic hydrocarbon group having 4e + 2 electrons (e represents an integer of 1 or more) in the cyclic $\pi$ electron system. Examples thereof include, but are not limited to, a phenyl group, a naphthyl group, a tetrahydronaphthyl group and an anthracenyl group. In an embodiment of the present invention, the aromatic hydrocarbon group is preferably a phenyl group.

In the specification, the "aralkyl group" refers to an alkyl group as mentioned above substituted with an aromatic hydrocarbon group. Examples thereof include, but are not limited to, C7-C16 aralkyl groups such as a benzyl group, a 1-phenylethyl group, a phenylpropyl group, a naphthylmethyl group and a naphthylethyl group. In an embodiment of the present invention, the aralkyl group is a C7-C10 aralkyl group and preferably a benzyl group or a 1-phenylethyl group.

In the specification, the "saturated heterocyclic group" refers to a monocyclic or polycyclic saturated heterocyclic group having at least one (preferably 1 to 5, more preferably 1 to 3) hetero atom selected from a nitrogen atom, an oxygen atom and a sulfur atom. Examples thereof include, but are not limited to, an aziridinyl group, an azetidinyl group, an imidazolidinyl group, a morpholino group, a pyrrolidinyl group, a piperidinyl group, a piperazinyl group, a tetrahydrofuranyl group, a tetrahydropyranyl group, a tetrahydrothiophenyl group, a thiazolidinyl group and an oxazolidinyl group. In an embodiment of the present invention, the saturated heterocyclic group is a 4 to 10-membered saturated heterocyclic group having at least one nitrogen atom or oxygen atom, preferably an azetidinyl group, a pyrrolidinyl group, a piperidinyl group or a tetrahydrofuranyl group, more preferably an azetidinyl group, a pyrrolidinyl group or a tetrahydrofuranyl group, and most preferably a tetrahydrofuranyl group.

In the specification, the "unsaturated heterocyclic group" refers to a monocyclic or polycyclic completely unsaturated or partially unsaturated heterocyclic group having at least one (preferably 1 to 5, more preferably 1 to 3) heteroatom selected from a nitrogen atom, an oxygen atom and a sulfur atom. Examples thereof include, but are not limited to, an imidazolyl group, a thienyl group, a pyrrolyl group, an oxazolyl group, an isoxazolyl group, a thiazolyl group, an isothiazolyl group, a thiadiazolyl group, an oxadiazolyl group, a pyrazolyl group, a triazolyl group, a tetrazolyl group, a pyridyl group, a pyrazyl group, a pyrimidinyl group, a pyridadinyl group, an indolyl groupe, an isoindolyl group, an indazolyl group, a triazolopyridyl group, a benzoimidazolyl group, a benzoxazolyl group, a benzothiazolyl group, a benzothienyl group, a furanyl group, a benzofuranyl group, a purinyl group, a quinolyl group, a isoquinolyl group, a quinazolinyl group, a quinoxalyl group, a methylenedioxyphenyl group, an ethylenedioxyphenyl group and a dihydrobenzofuranyl group. In an embodiment of the present invention, the unsaturated heterocyclic group is a 5 to 10-membered unsaturated heterocyclic group, more preferably an imidazolyl group, a pyrazolyl group, a thiazolyl group, an isoxazolyl group or a furanyl group, further preferably an imidazolyl group, a pyrazolyl group or a thiazolyl group, and most preferably an imidazolyl group.

In the specification, the "unsaturated hydrocarbon group" refers to a linear or branched unsaturated hydrocarbon group containing at least one carbon-carbon double bond or triple bond. Examples thereof include, but are not limited to, C2-C6 unsaturated hydrocarbon groups such as a vinyl group, an allyl group, a methylvinyl group, a 1-propenyl group, a butenyl group, a pentenyl group, a hexenyl group, an ethynyl group and a 2-propynyl group. In an embodiment of the present invention, the unsaturated hydrocarbon group is preferably a linear or branched hydrocarbon group having 2 to 4 carbon atoms and at least one carbon-carbon double bond or triple bond and more preferably a vinyl group, an allyl

group or a 1-propenyl group.

<Compound of formula (1)>

In one embodiment of the present invention, there is provided a compound represented by the following formula (1) :

[Formula 3]

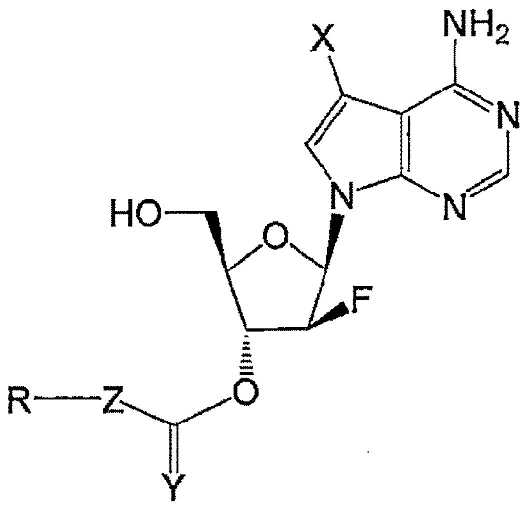

(1)

wherein

X represents a chlorine atom, a bromine atom or an iodine atom,
Y represents an oxygen atom or a sulfur atom,
Z represents an oxygen atom or a sulfur atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent, or a 5 to 10-membered unsaturated heterocyclic group that may have a substituent. The compound of the present invention is a novel compound having pyrrolo[2,3-d]pyrimidine as a basic skeleton.

In a compound represented by general formula (I) of the present invention, X represents a chlorine atom, a bromine atom or an iodine atom. In one embodiment of the present invention, X may be a bromine atom. In another embodiment of the present invention, X may be an iodine atom. In a preferred embodiment of the present invention, X is a bromine atom or an iodine atom. In a further preferred embodiment, X can be an iodine atom.

In one embodiment of the present invention, there is provided a compound represented by the following formula (1) :

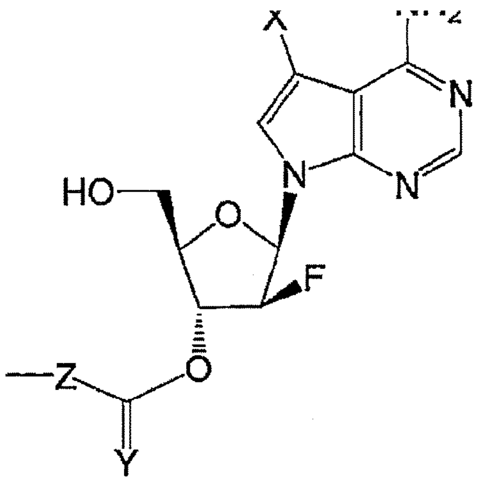

(1)

wherein

X represents a bromine atom or an iodine atom,
Y represents an oxygen atom or a sulfur atom,
Z represents an oxygen atom or a sulfur atom,
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent. The compound of the present invention is a novel compound having pyrrolo[2,3-d]pyrimidine as a basic skeleton.

In a compound represented by general formula (I) of the present invention, Y represents an oxygen atom or a sulfur atom. In one embodiment of the present invention, Y may be an oxygen atom. In another embodiment of the present invention, Y may be a sulfur atom. In a preferred embodiment of the present invention, Y can be an oxygen atom.
In a compound represented by general formula (I) of the present invention, Z represents an oxygen atom or a sulfur atom. In one embodiment of the present invention, Z may be an oxygen atom. In another embodiment of the present invention, Z may be a sulfur atom. In a preferred embodiment of the present invention, Z can be an oxygen atom.
In a compound represented by general formula (I) of the present invention, R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent.
Examples of the "linear C1-C6 alkyl group" represented by R include C1-C6 alkyl groups as mentioned above such as a methyl group, an ethyl group, a n-propyl group, a n-butyl group, a n-pentyl group and a n-hexyl group. The "linear C1-C6 alkyl group" is preferably a methyl group, an ethyl group, a n-propyl group, a n-butyl group and more preferably a methyl group, an ethyl group or a n-propyl group.
As the "substituent" in the "linear C1-C6 alkyl group that may have a substituent" represented by R, the substituents as mentioned above are mentioned. The substituent is preferably a C1-C6 alkyl group, a C1-C4 alkoxy group, a C1-C4 haloalkyl group, a halogen atom or an aromatic hydrocarbon group, more preferably a C1-C3 alkyl group, a C1-C3 alkoxy group, a fluorine atom, a chlorine atom or a phenyl group, more preferably a methyl group, an ethyl group, a methoxy group, a chlorine atom, an alkyl fluoride group or a phenyl group, and further preferably a methyl group or an ethyl group.
The "linear C1-C6 alkyl group that may have a substituent" represented by R is preferably a linear C1-C6 alkyl group that may have 1 to 3 substituents which are selected from C1-C6 alkyl groups, C1-C4 alkoxy groups, C1-C4 haloalkyl groups, halogen atoms or aromatic hydrocarbon groups,

more preferably a linear C1-C6 alkyl group that may have 1 to 3 substituents which are selected from C1-C6 alkyl groups, C1-C4 alkoxy groups, C1-C4 haloalkyl groups, halogen atoms or phenyl groups,

further preferably a linear C1-C4 alkyl group that may have 1 to 3 substituents which are selected from C1-C3 alkyl groups, C1-C3 alkoxy groups, C1-C3 haloalkyl groups, fluorine atoms, chlorine atoms or phenyl groups,
further preferably a linear C1-C3 alkyl group that may have 1 to 3 substituents which are selected from C1-C2 alkyl groups, C1-C2 alkoxy groups, C1-C2 haloalkyl groups, fluorine atoms or chlorine atoms,
still further preferably a methyl group, an ethyl group, a n-propyl group, an isopropyl group, a 1-ethyl-propyl group, a 1-(fluoromethyl)-2-fluoroethyl group, a 2-methoxy-1-methylethyl group, a tert-butyl group, a 2,2-dimethylpropyl group, a benzyl group, a 1-phenylethyl group, a 1-methylbenzyl group, a 2,2,2-trichloroethyl group or a trichloromethyl group, and
most preferably an ethyl group, an isopropyl group or a 1-ethyl-propyl group.

Examples of the "C2-C6 alkenyl group" represented by R include the C2-C6 alkenyl groups as mentioned above. The "C2-C6 alkenyl group" is preferably a 1-propenyl group or a 2-propenyl group.
As the "substituent" in the "C2-C6 alkenyl group that may have a substituent" represented by R, the substituents as mentioned above are mentioned. The substituent is preferably a C1-C4 alkyl group and more preferably a methyl group.
The "C2-C6 alkenyl group that may have a substituent" represented by R is preferably a 1-methyl-2-propenyl group.
As the substituent in the "C2-C6 alkynyl group" represented by R, C2-C6 alkynyl groups as mentioned above are mentioned. The C2-C6 alkynyl group is preferably a 2-propynyl group.
As the "substituent" in the "C2-C6 alkynyl group that may have a substituent" represented by R, the substituents as mentioned above are mentioned. The substituent is preferably a methyl group.
The "C2-C6 alkynyl group that may have a substituent" represented by R is preferably a 1-methyl-2-propynyl group.
Examples of the "C3-C10 cycloalkyl group" represented by R include C3-C10 cycloalkyl groups as mentioned above. The "C3-C10 cycloalkyl group" is preferably a C3-C8 cycloalkyl group, more preferably a cyclopropyl group, a cyclobutyl group, a cyclopentyl group, a cyclohexyl group, a cycloheptyl group, a [2.2.1]bicycloheptanyl group or a [3.1.1]bicycloheptanyl group, and further preferably a cyclopentyl group.
The "C3-C10 cycloalkyl group that may have a substituent" represented by R is preferably a C3-C8 cycloalkyl group, more preferably a cyclopropyl group, a cyclobutyl group, a cyclopentyl group, a cyclohexyl group, a cycloheptyl group, a [2.2.1]bicycloheptanyl group or a [3.1.1]bicycloheptanyl group and further preferably a cyclopentyl group.
Examples of the "C4-C10 cycloalkenyl group" represented by R include C4-C10 cycloalkenyl groups as mentioned above. The "C4-C10 cycloalkenyl group" is preferably a 1H, 2H, 3H-indene group or a cyclopentenyl group and more preferably a cyclopentenyl group.
The "C4-C10 cycloalkenyl group that may have a substituent" represented by R is preferably a cyclopentenyl group or a 1H, 2H, 3H-indene group and more preferably a cyclopentenyl group.
Examples of the "C6-C10 aromatic hydrocarbon group" represented by R include C6-C10 aromatic hydrocarbon groups as mentioned above. The "C6-C10 aromatic hydrocarbon group" is preferably a phenyl group or a naphthyl group and more preferably a phenyl group.
The "C6-C10 aromatic hydrocarbon group that may have a substituent" represented by R is preferably a phenyl group.
Examples of the "4 to 10-membered saturated heterocyclic group" represented by R include 4 to 10-membered saturated heterocyclic groups as mentioned above. The "4 to 10-membered saturated heterocyclic group" is preferably a 4 to 8-membered saturated heterocyclic group, more preferably a 4 to 6-membered saturated heterocyclic group, further preferably 4 to 6-membered saturated heterocyclic group having at least one oxygen atom and most preferably a tetrahydrofuranyl group.
The "4 to 10-membered saturated heterocyclic group that may have a substituent" represented by R is preferably a 4 to 8-membered saturated heterocyclic group, more preferably a 4 to 6-membered saturated heterocyclic group, further preferably a 4 to 6-membered saturated heterocyclic group having at least one oxygen atom, and most preferably a tetrahydrofuranyl group.
Examples of the "5 to 10-membered unsaturated heterocyclic group" represented by R include 5 to 10-membered unsaturated heterocyclic groups as mentioned above. The "5 to 10-membered unsaturated heterocyclic group" is preferably an imidazolyl group, a pyrazolyl group or a thiazolyl group and more preferably an imidazolyl group.
The "5 to 10-membered unsaturated heterocyclic group that may have a substituent" represented by R is an imidazolyl group, a pyrazolyl group or a thiazolyl group and more preferably an imidazolyl group.
In a compound represented by general formula (I) of the present invention, R preferably represents a linear C1-C6 alkyl group that may have 1 to 3 substituents which are selected from C1-C6 alkyl groups, C1-C4 alkoxy groups, C1-C4 haloalkyl groups, halogen atoms or aromatic hydrocarbon groups;

a C3-C10 cycloalkyl group that may have 1 to 3 substituents which are selected from C1-C6 alkyl groups, C1-C4

alkoxy groups, halogen atoms or aromatic hydrocarbon groups;

a C4-C10 cycloalkenyl group that may have 1 to 3 substituents which are selected from C1-C6 alkyl groups, C1-C4 alkoxy groups, halogen atoms or aromatic hydrocarbon groups;

a 4 to 10-membered saturated heterocyclic group that may have 1 to 3 substituents which are selected from C1-C6 alkyl groups, C1-C4 alkoxy groups, halogen atoms or aromatic hydrocarbon groups; or

a 5 to 10-membered unsaturated heterocyclic group that may have 1 to 3 substituents which are selected from C1-C6 alkyl groups, C1-C6 alkenyl groups, C1-C6 alkynyl groups, C1-C4 alkoxy groups, hydroxy groups, or halogen atom or aromatic hydrocarbon groups.

In a compound represented by general formula (I) of the present invention, R more preferably represents a linear C1-C6 alkyl group that may have 1 to 3 substituents which are selected from C1-C6 alkyl groups, C1-C4 alkoxy groups, C1-C4 haloalkyl groups, halogen atoms or phenyl groups;

a C3-C10 cycloalkyl group that may have 1 to 3 substituents which are selected from C1-C6 alkyl groups, C1-C4 alkoxy groups, halogen atoms or phenyl groups;

a C4-C10 cycloalkenyl group that may have 1 to 3 substituents which are selected from C1-C6 alkyl groups, C1-C4 alkoxy groups, halogen atoms or phenyl groups; or

a 4 to 10-membered saturated heterocyclic group that may have 1 to 3 substituents which are selected from C1-C6 alkyl groups, C1-C4 alkoxy groups, halogen atoms or phenyl groups.

In a compound represented by general formula (I) of the present invention, R more preferably represents a linear C1-C4 alkyl group that may have 1 to 3 substituents which are selected from C1-C3 alkyl groups, C1-C3 alkoxy groups, C1-C3 haloalkyl groups, fluorine atoms, chlorine atoms or phenyl groups;

a C3-C8 cycloalkyl group that may have 1 to 2 substituents which are selected from C1-C3 alkyl groups, C1-C3 alkoxy groups or halogen atoms;

a C4-C8 cycloalkenyl group that may have 1 to 2 substituents which are selected from C1-C3 alkyl groups, C1-C3 alkoxy groups or halogen atoms; or

a 4 to 8-membered saturated heterocyclic group that may have 1 to 3 substituents which are selected from C1-C3 alkyl groups, C1-C3 alkoxy groups or halogen atoms.

In a compound represented by general formula (I) of the present invention, R further preferably represents a linear C1-C3 alkyl group that may have 1 to 3 substituents which are selected from C1-C2 alkyl groups, C1-C2 alkoxy groups, C1-C2 haloalkyl groups, fluorine atoms or chlorine atoms;

a C3-C7 cycloalkyl group that may have 1 to 2 substituents which are selected from C1-C2 alkyl groups, C1-C2 alkoxy groups, fluorine atoms or chlorine atoms;

a C4-C6 cycloalkenyl group that may have 1 to 2 substituents which are selected from C1-C2 alkyl groups, or C1-C2 alkoxy groups, fluorine atoms or chlorine atoms; or

a 4 to 6-membered saturated heterocyclic group that may have 1 to 3 substituents which are selected from C1-C2 alkyl groups, C1-C2 alkoxy groups, fluorine atoms or chlorine atoms.

In a compound represented by general formula (I) of the present invention, R further preferably represents a methyl group, an ethyl group, a n-propyl group, an isopropyl group, a 1-ethyl-propyl group, a 2-ethyl-propyl group, a 2,2-dimethylpropyl group, a 1-(fluoromethyl)-2-fluoroethyl group, a 2-methoxy-1-methylethyl group, a tert-butyl group, a 2,2,2-trichloromethyl group, a benzyl group, a 1-phenylethyl group, a cyclopropyl group, a cyclobutyl group, a cyclopentyl group, a cyclohexyl group, a cycloheptyl group, a [2.2.1]bicycloheptanyl group, a [3.1.1]bicycloheptanyl group, a cyclopentenyl group, a cyclopentyl group, a 1H,2H,3H-indene group or a tetrahydrofuranyl group.

In a compound represented by general formula (I) of the present invention, R most preferably represents an ethyl group, an isopropyl group, a 1-ethyl-propyl group, a cyclopentyl group, a [2.2.1]bicycloheptanyl group, a [3.1.1]bicycloheptanyl group or a cyclopentenyl group.

In one embodiment of the present invention, there is provided a compound represented by formula (I), wherein

X represents a chlorine atom, a bromine atom or an iodine atom,
Y represents an oxygen atom or a sulfur atom,
Z represents an oxygen atom or a sulfur atom, and
R represents a linear C1-C6 alkyl group that may have 1 to 3 substituents which are selected from C1-C6 alkyl groups, C1-C4 alkoxy groups, C1-C4 haloalkyl groups, halogen atoms or phenyl groups;

a C3-C10 cycloalkyl group that may have 1 to 3 substituents which are selected from C1-C6 alkyl groups, C1-C4 alkoxy groups, halogen atoms or phenyl groups;

a C4-C10 cycloalkenyl group that may have 1 to 3 substituents which are selected from C1-C6 alkyl groups, C1-C4 alkoxy groups, halogen atoms or phenyl groups; or

a 4 to 10-membered saturated heterocyclic group that may have 1 to 3 substituents which are selected from C1-C6 alkyl groups, C1-C4 alkoxy groups, halogen atoms or phenyl groups,

or a pharmaceutically acceptable salt thereof.

In one embodiment of the present invention, there is provided a compound represented by formula (I), wherein

X represents a chlorine atom, a bromine atom or an iodine atom,

Y represents an oxygen atom or a sulfur atom,

Z represents an oxygen atom or a sulfur atom, and

R represents a linear C1-C4 alkyl group that may have 1 to 3 substituents which are selected from C1-C3 alkyl groups, C1-C3 alkoxy groups, C1-C3 haloalkyl groups, fluorine atoms, chlorine atoms or phenyl groups;

a C3-C8 cycloalkyl group that may have 1 to 2 substituents which are selected from C1-C3 alkyl groups, C1-C3 alkoxy groups or halogen atoms;

a C4-C8 cycloalkenyl group that may have 1 to 2 substituents which are selected from C1-C3 alkyl groups, C1-C3 alkoxy groups or halogen atoms; or

a 4 to 8-membered saturated heterocyclic group that may have 1 to 3 substituents which are selected from C1-C3 alkyl groups, C1-C3 alkoxy groups or halogen atoms; or a pharmaceutically acceptable salt thereof.

In one embodiment of the present invention, there is provided a compound represented by formula (I), wherein,

X represents a bromine atom or an iodine atom,

Y represents an oxygen atom or a sulfur atom,

Z represents an oxygen atom or a sulfur atom, and

R represents a linear C1-C3 alkyl group that may have 1 to 3 substituents which are selected from C1-C2 alkyl groups, C1-C2 alkoxy groups, C1-C2 haloalkyl groups, fluorine atoms or chlorine atoms;

a C3-C7 cycloalkyl group that may have 1 to 2 substituents which are selected from C1-C2 alkyl groups, C1-C2 alkoxy groups, fluorine atoms or chlorine atoms;

a C4-C6 cycloalkenyl group that may have 1 to 2 substituents which are selected from C1-C2 alkyl groups, C1-C2 alkoxy groups, fluorine atoms or chlorine atoms; or

a 4 to 6-membered saturated heterocyclic group that may have 1 to 3 substituents which are selected from C1-C2 alkyl groups, C1-C2 alkoxy groups, fluorine atoms or chlorine atoms,

or a pharmaceutically acceptable salt thereof.

In one embodiment of the present invention, there is provided a compound represented by formula (I), wherein

X represents an iodine atom,

Y represents an oxygen atom or a sulfur atom,

Z represents an oxygen atom or a sulfur atom, and

R represents a linear C1-C6 alkyl group that may have 1 to 3 substituents which are selected from C1-C2 alkyl groups or C1-C2 haloalkyl groups; a C3-C6 cycloalkyl group that may have 1 to 3 substituents which are selected from C1-C2 alkyl groups; or

a C4-C6 cycloalkenyl group that may have 1 to 3 substituents which are selected from C1-C2 alkyl groups, or a pharmaceutically acceptable salt thereof.

In one embodiment of the present invention, there is provided a compound represented by formula (I), wherein,

X represents an iodine atom,

Y represents an oxygen atom or a sulfur atom,

Z represents an oxygen atom or a sulfur atom, and

R represents a methyl group, an ethyl group, a n-propyl group, an isopropyl group, a 1-ethyl-propyl group, a 2-ethyl-propyl group, a 2,2-dimethylpropyl group, a 1-(fluoromethyl)-2-fluoroethyl group, a 2-methoxy-1-methylethyl group, a tert-butyl group, a 2,2,2-trichloromethyl group, a benzyl group, a 1-phenylethyl group, a cyclopropyl group, a cyclobutyl group, a cyclopentyl group, a cyclohexyl group, a cycloheptyl group, a [2.2.1]bicycloheptanyl group, a cyclopentenyl group, a cyclopentyl group, a 1H,2H,3H-indene group or a tetrahydrofuranyl group,

or a pharmaceutically acceptable salt thereof.

In one embodiment of the present invention, there is provided a compound represented by formula (I), wherein,

X represents an iodine atom,
Y represents an oxygen atom,
Z represents an oxygen atom or a sulfur atom, and
R represents an ethyl group, an isopropyl group, a 1-ethyl-propyl group, a cyclopentyl group, a [2.2.1]bicycloheptanyl group or a cyclopentenyl group,

or a pharmaceutically acceptable salt thereof.

In one embodiment of the present invention, there is provided a compound represented by formula (I), wherein

X represents an iodine atom,
Y represents an oxygen atom or a sulfur atom,
Z represents an oxygen atom or a sulfur atom, and
R represents a linear C1-6 alkyl group that may have a substituent; a C3-10 cycloalkyl group that may have a substituent; or a C4-10 cycloalkenyl group that may have a substituent, and
in R, the carbon to be bound to Z is a secondary carbon atom,

or a pharmaceutically acceptable salt thereof. In an embodiment of the present invention, a compound represented by formula (I) can be obtained by introducing R by using R-OH or R-SH. In the case where the carbon to be bound to Z in R is a secondary carbon atom, if Z represents an oxygen atom, a secondary alcohol is selected as R-OH, whereas, if Z is a sulfur atom, a secondary thiol is selected as R-SH. If R-OH is a secondary alcohol, examples of R-OH to be used include, but are not limited to, isopropanol, cyclopentanol, 1-ethyl propan-1-ol, cyclopentenol, 1-phenylethan-1-ol, [2.2.1]bicycloheptan-1-ol and 1-(fluoromethyl)-2-fluoroethan-1-ol. If R-SH is a secondary thiol, examples of R-SH to be used include, but are not limited to, isopropyl thiol.

In a preferred embodiment of the present invention, there is provided a compound represented by formula (I), wherein

X represents an iodine atom,
Y represents an oxygen atom,
Z represents an oxygen atom, and
R represents a linear C1-6 alkyl group that may have a linear C1-6 alkyl group, a C1-C4 haloalkyl group, a halogen atom or a C6-10 aromatic hydrocarbon group, as a substituent;
a C3-10 cycloalkyl group that may have a linear C1-6 alkyl group, a halogen atom or a C6-10 aromatic hydrocarbon group, as a substituent; or
a C4-10 cycloalkenyl group that may have a linear C1-6 alkyl group, a halogen atom or a C6-10 aromatic hydrocarbon group, as a substituent, and
in R, the carbon to be bound to Z is a secondary carbon atom,

or a pharmaceutically acceptable salt thereof.

In a more preferred embodiment of the present invention, there is provided a compound represented by formula (I), wherein,

X represents an iodine atom,
Y represents an oxygen atom,
Z represents an oxygen atom, and
R represents a linear C1-6 alkyl group that may have a single linear C1-6 alkyl group, a single C1-C4 haloalkyl group, 1 to 2 fluorine atoms or a single C6-10 aromatic hydrocarbon group, as a substituent(s);
a C3-10 cycloalkyl group that may have a single linear C1-6 alkyl group, 1 to 2 fluorine atoms or a single C6-10 aromatic hydrocarbon group, as a substituent(s); or a C4-10 cycloalkenyl group that may have a single linear C1-6 alkyl group, 1 to 2 fluorine atoms or a single C6-10 aromatic hydrocarbon groups, as a substituent(s); and
in R, the carbon to be bound to Z is a secondary carbon atom,

or a pharmaceutically acceptable salt thereof.

In a further more preferred embodiment of the present invention, there is provided a compound represented by formula (I), wherein

X represents an iodine atom,
Y represents an oxygen atom,
Z represents an oxygen atom, and
R represents a linear C1-3 alkyl group that may have a single linear C1-3 alkyl group, a single C1-C3 haloalkyl group, 1 to 2 fluorine atoms or a single phenyl group, as a substituent(s);
a C4-8 cycloalkyl group that may have a single linear C1-3 alkyl group, 1 to 2 fluorine atoms or a single phenyl group, as a substituent(s); or
a C4-6 cycloalkenyl group that may have a single linear C1-3 alkyl group, 1 to 2 fluorine atoms or a single phenyl group, as a substituent(s); and
in R, the carbon to be bound to Z is a secondary carbon atom,

or a pharmaceutically acceptable salt thereof.

In another embodiment of the present invention, there is provided a compound represented by formula (I), wherein,

X represents a chlorine atom, a bromine atom or an iodine atom,
Y represents an oxygen atom,
Z represents an oxygen atom or a sulfur atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent,

or a pharmaceutically acceptable salt thereof.

In a preferred embodiment of the present invention, there is provided a compound represented by formula (I), wherein

X represents a bromine atom or an iodine atom,
Y represents an oxygen atom,
Z represents an oxygen atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent, or a pharmaceutically acceptable salt thereof.

In another embodiment of the present invention, there is provided a compound represented by formula (I), wherein

X represents a bromine atom or an iodine atom,
Y represents an oxygen atom or a sulfur atom,
Z represents an oxygen atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent,

or a pharmaceutically acceptable salt thereof.

In a preferred embodiment of the present invention, there is provided a compound represented by formula (I), wherein,

X represents a bromine atom,
Y represents an oxygen atom,
Z represents an oxygen atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent,

or a pharmaceutically acceptable salt thereof.

In another embodiment of the present invention, there is provided a compound represented by formula (I), wherein

X represents an iodine atom,
Y represents an oxygen atom or a sulfur atom,
Z represents an oxygen atom or a sulfur atom,

If Y and Z are each an oxygen atom, R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon groups that may have a substituent, a 4 to 10-membered saturated heterocyclic group that may have a substituent or a 5 to 10-membered unsaturated heterocyclic group that may have a substituent, if Y represents a sulfur atom and Z represents an oxygen atom, R represents a linear C1-C6 alkyl substituted with a linear C1-C6 alkyl,
if Y represents an oxygen atom and Z represents a sulfur atom, R represents a linear C1-C6 alkyl that may be substituted with a linear C1-C6 alkyl,
or a pharmaceutically acceptable salt thereof.

In one embodiment of the present invention, specific examples of the compound of the present invention can include, but are not limited to, the following compounds:

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;
(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate;
(3) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-cyclopentyl carbonothioate;
(4) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-isopropyl carbonothioate;
(5) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate;
(6) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl 1-methylpropyl carbonate;
(7) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopent-3-en-1-yl carbonate;
(8) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl (bicyclo[2.2.1]heptan-2-yl) carbonate,

or pharmaceutically acceptable salts thereof.

In a preferred embodiment of the present invention, the compound of the present invention may be selected from the group consisting of the compounds (1) to (2) or a pharmaceutically acceptable salt thereof and preferably a compound selected from the group consisting of the followings:

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;
(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate,
or a pharmaceutically acceptable salt thereof.

In one embodiment of the present invention, there is provided a compound represented by the following formula (2) :

[Formula 5]

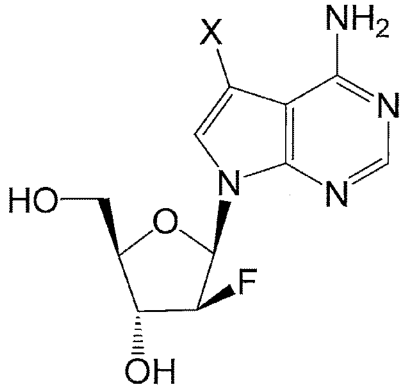

(2)

wherein
X represents a chlorine atom, a bromine atom or an iodine atom,
or a pharmaceutically acceptable salt thereof.

In one embodiment of the present invention, there is provided a compound selected from the group consisting of the followings:

(1) (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol,
(2) (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol, and
(3) (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol,

or a pharmaceutically acceptable salt thereof, as a compound of the present invention.

<Process for producing compound represented by formula (1)>

Now, a process for producing a compound according to the present invention will be described.

A compound represented by formula (1) of the present invention can be produced, for example, by the following production process or a process shown in Examples. However, the process for producing a compound represented by formula (1) of the present invention is not limited to these shown in these reaction examples. The products obtained in individual steps can be subjected to the following steps with or without isolation/purification by a isolation/purification methodknown in the technical field such as concentration, concentration under reduced pressure, crystallization, solvent extraction, reprecipitation and chromatography. In the following production process, a protecting group may be introduced or removed, if necessarily, regardless of whether a description is made or not, and the order of individual steps may be appropriately changed.

To starting materials and products obtained in individual steps, a protecting group that is easily converted into a functional group may be introduced as needed. This is sometimes effective for individual steps or enables to change the order of individual steps. As the protecting group to be used herein, a protecting group described, for example, in literatures ["Protective Groups in Organic Synthesis" written by Greene and Wuts, the fifth edition, John Wiley & Sons Inc., 2014] may be used. The protecting group may be appropriately selected depending on the reaction condition employed in each step. After a reaction is carried out by introducing a protecting group, the protecting group is removed, as needed. In this manner, a desired compound can be obtained.

[Production process 1] Process for producing compound represented by general formula (1a)(in general formula (1), Z represents an oxygen atom)

[Formula 6]

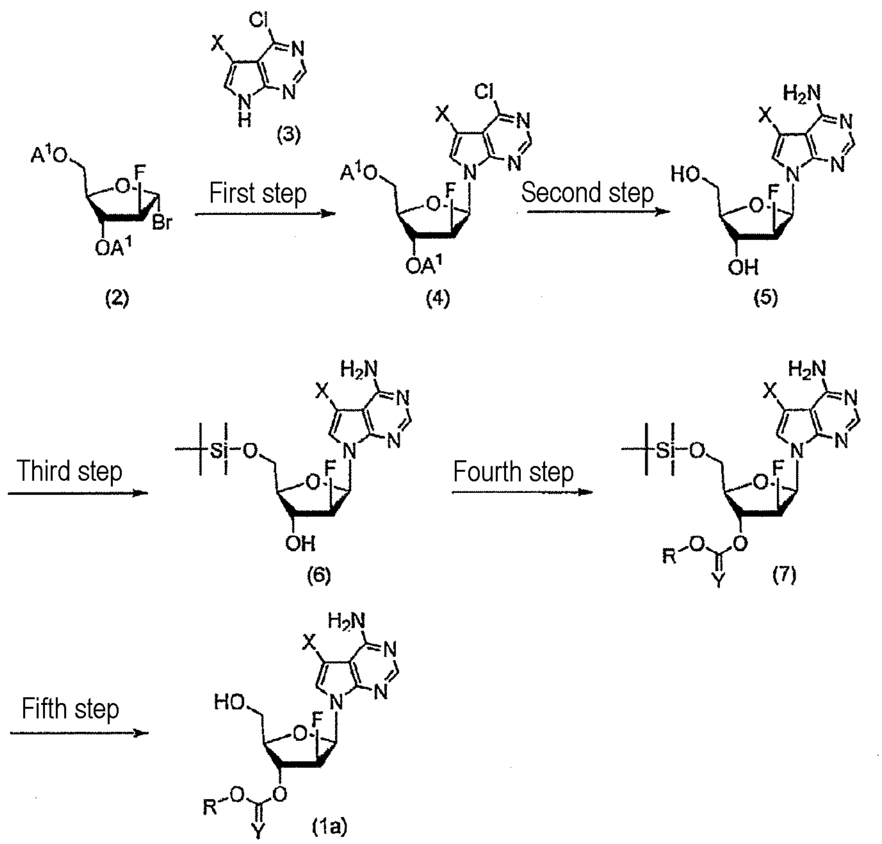


wherein X, Y and R are the same as defined above and $A^1$ represents an acyl group

(First step)

In this step, a compound represented by general formula (2) and a compound represented by general formula (3) are reacted in the presence of a base to obtain a compound represented by general formula (4).

The $A^1$ of a compound represented by general formula (2) is not particularly limited as long as it can be deprotected by ammonia. Examples thereof include an acyl group such as a benzoyl group or an acetyl group.

Examples of the base to be used in the reaction include inorganic bases such as sodium hydroxide, sodium hydride, lithium hydroxide, potassium hydride and potassium hydroxide. The solvent to be used in the reaction is not particularly limited as long as it does not affect the reaction. Examples of the solvent include acetonitrile, dioxane, tetrahydrofuran, N,N-dimethylacetamide, N,N-dimethylformamide and dimethyl sulfoxide. These solvents can be used alone or as a mixture. In the reaction, if necessary, an organic tertiary amine such as tris(2-(2-methoxyethoxy)ethyl)amine may be used. In the reaction, a compound represented by general formula (3) is used in an amount of about 0.5 to 20 moles and preferably about 0.7 to 5 moles; a base is used in an amount of 1.0 to 40 moles, preferably about 1.4 to 10 moles; and an organic tertiary amine is used in an amount of 0.01 to 10 moles and preferably about 0.02 to 5 moles, relative to one mole of a compound represented by general formula (2). The reaction temperature is -30 to 100°C and preferably - 20 to 60°C. The reaction time is 0.1 to 48 hours and preferably 1 to 24 hours.

(Second step)

In this step, a compound represented by general formula (4) and ammonia are reacted to successfully produce a compound represented by general formula (5).

The solvent to be used in the reaction is not particularly limited as long as it does not affect the reaction. Examples of the solvent include dioxane, dimethoxyethane, tetrahydrofuran, N,N-dimethylacetamide, dimethyl sulfoxide and water. These solvents can be used alone or as a mixture. In the reaction, ammonia is used in an amount of about

3 to 1000 moles and preferably about 5 to 500 moles relative to one mole of a compound represented by general formula (4). The reaction temperature is 0 to 200°C and preferably 20 to 150°C. The reaction time is 0.1 to 48 hours and preferably 1 to 24 hours.

(Third step)

In this step, a compound represented by general formula (5) and a tert-butyldimethylsilylation reagent are reacted to successfully produce a compound represented by general formula (6) having the 5'-position alone protected.
Examples of the tert-butyldimethylsilylation reagent to be used in this reaction include tert-butyldimethylchlorosilane. The solvent to be used in this reaction is not particularly limited as long as it does not affect the reaction. Examples of the solvent include dichloromethane, chloroform, ethyl acetate, tetrahydrofuran, dioxane, toluene, N,N-dimethylformamide, N,N-dimethylacetamide and dimethyl sulfoxide. These solvents can be used alone or as a mixture. In the reaction, if necessary, a base may be used. Examples of the base include organic amines such as imidazole, 1-methylimidazole, triethylamine, tripropylamine, diisopropylethylamine, N-methylmorpholine, pyridine, 4-(N,N-dimethylamino)pyridine, lutidine and collidine; and inorganic bases such as sodium hydrogen carbonate, sodium carbonate and potassium carbonate. Base alone may be used as a solvent. In the reaction, a tert-butyldimethylsilylation reagent may be used in an amount of about 1 to 20 moles and preferably about 1 to 10 moles; and a base may be used in an amount of about 1 to 5000 moles and preferably about 1 to 1000 moles, relative to one mole of a compound represented by general formula (5). The reaction temperature is -30 to 100°C and preferably -10 to 60°C. The reaction time is 0.1 to 100 hours and preferably 1 to 48 hours.

(Fourth step)

In this step, a compound represented by general formula (6) and a carbonation reagent represented by ROC(=Y)-Wa (R and Y are the same as defined above, Wa represents a halogen, a p-nitrophenoxy group or a 1H-imidazol-1-yl group) or an alkoxythiocarbonylation reagent are reacted to successfully produce a compound represented by general formula (7).
The compound represented by RO-C(=Y)-Wa to be used in this reaction may be prepared in accordance with a method commonly known in the technical field. The compound can be obtained, for example, by reacting triphosgene and the corresponding alcohol represented by R-OH (R represents the same as defined above). The compound represented by RO-C(=Y)-Wa can be isolated and purified as needed but may be used in this step without purification. This reaction may be carried out in accordance with a method commonly known in the technical field. The solvent to be used is not particularly limited as long as it does not affect the reaction. Examples of the solvent include dichloromethane, dichloroethane, chloroform, ethyl acetate, tetrahydrofuran, dioxane, toluene, N,N-dimethylformamide, N,N-dimethylacetamide and dimethyl sulfoxide. These solvents can be used alone or as a mixture. In the reaction, a base may be used, if necessary. Examples of the base include organic amines such as imidazole, 1-methylimidazole, triethylamine, tripropylamine, diisopropylethylamine, N-methylmorpholine, pyridine, 4-(N,N-dimethylamino) pyridine, lutidine, collidine and 1,8-diazabicyclo[5.4.0]undec-7-en, and inorganic bases such as sodium hydrogen carbonate, sodium carbonate and potassium carbonate. These bases can be used alone or as a mixture. The base alone may be used as a solvent. In the reaction, a compound represented by RO-C(=Y)-Wa is used in an amount of about 1 to 20 moles and preferably about 1 to 10 moles, and a base in an amount of about 1 to 5000 moles and preferably about 1 to 1000 moles, relative to one mole of a compound represented by general formula (6). The reaction temperature is -30 to 130°C and preferably -10 to 100°C. The reaction time is 0.1 to 100 hours and preferably 1 to 48 hours.

(Fifth step)

In this step, a reagent for removing a protecting group is reacted with a compound represented by general formula (7) to remove a protecting group only from the 5'-position, with the result that a compound represented by general formula (1a) can be produced.
The solvent to be used is not particularly limited as long as it does not affect the reaction. Examples of the solvent include dichloromethane, toluene, ethyl acetate, tetrahydrofuran, dioxane, isopropanol, ethanol, methanol and water. These solvents can be used alone or as a mixture. The reagent for removing a protecting group is not particularly limited as long as it is commonly used in removing a protecting group of a silyl group. Examples of the reagent include fluoride-ion reagents such as tetrabutyl ammonium fluoride, hydrogen fluoride and potassium fluoride; mineral acids such as hydrochloric acid, hydrogen bromide, sulfuric acid, and phosphoric acid; and organic acids such as trifluoroacetic acid, acetic acid, propionic acid, formic acid, methanesulfonic acid and p-toluenesulfonic acid.
In the reaction, a reagent for removing a protecting group is used in an amount of about 0.5 to 1000 moles and preferably about 1 to 100 moles relative to one mole of a compound represented by general formula (7). The reaction temperature

is -30 to 130°C and preferably -10 to 80°C. The reaction time is 0.1 to 100 hours and preferably 0.5 to 24 hours.

[Production process 2] Process for producing compound represented by general formula (1b)(in general formula (1), Z represents a sulfur atom)

[Formula 7]

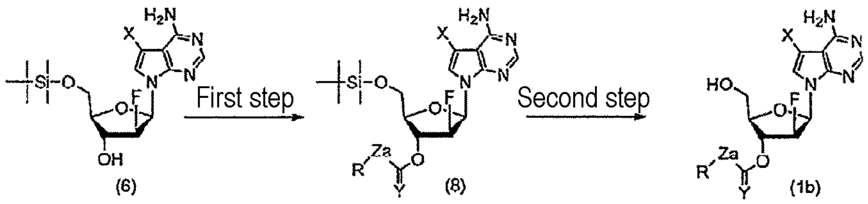


wherein X, Y and R are the same as defined above, and Za represents a sulfur atom

(First step)

In this step, a reagent represented by Wb-C(=Y)-Wb (Y is the same as defined above, Wb represents a p-nitrophenoxy group or a 1H-imidazol-1-yl group) is reacted with a compound represented by general formula (6), and then, further reacted with a thiol represented by R-SH (R represents the same as defined above) to produce a compound represented by general formula (8).

The solvent to be used in the reaction is not particularly limited as long as it does not affect the reaction. Examples of the solvent include dichloromethane, dichloroethane, chloroform, ethyl acetate, tetrahydrofuran, dioxane, toluene, N,N-dimethylformamide, N,N-dimethylacetamide and dimethyl sulfoxide. These solvents can be used alone or as a mixture. In the reaction, a base may be used, if necessary. Examples of the base include organic amines such as imidazole, 1-methylimidazole, triethylamine, tripropylamine, diisopropylethylamine, N-methylmorpholine, pyridine, 4-(N,N-dimethylamino) pyridine, lutidine, collidine and 1,8-diazabicyclo[5.4.0]undec-7-en; and inorganic bases such as sodium hydrogen carbonate, sodium carbonate and potassium carbonate. These bases can be used alone or as a mixture. Also, a base alone may be used as a solvent. Examples of the compound represented by Wb-C(=Y)-Wb to be used in this reaction include bis(p-nitrophenyl)carbonate, carbonyldiimidazole and thiocarbonyldiimidazole. In the reaction, a compound represented by Wb-CY-Wb is used in an amount of about 1 to 20 moles and preferably about 1 to 10 moles; a thiol represented by R-SH is used in an amount of about 1 to 1000 moles and preferably about 1 to 100 moles; the base is used in an amount of about 1 to 5000 moles and preferably about 1 to 1000 moles, relative to one mole of a compound represented by general formula (6). The reaction temperature is -30 to 130°C and preferably -10 to 100°C. The reaction time is 0.1 to 100 hours and preferably 1 to 48 hours.

(Second step)

In this step, a reagent for removing a protecting group is reacted with a compound represented by general formula (8) to remove the protecting group only from the 5'-position. In this manner, a compound represented by general formula (1b) can be produced.

The solvent to be used is not particularly limited as long as it does not affect the reaction. Examples of the solvent include dichloromethane, toluene, ethyl acetate, tetrahydrofuran, dioxane, isopropanol, ethanol, methanol and water. These solvents can be used alone or as a mixture. The reagent for removing a protecting group to be used is not particularly limited as long as it is commonly used for removing a protection group of a silyl group. Examples of the reagent include fluoride-ion reagents such as tetrabutyl ammonium fluoride, hydrogen fluoride and potassium fluoride; mineral acids such as hydrochloric acid, hydrogen bromide, sulfuric acid and phosphoric acid; and organic acids such as trifluoroacetic acid, acetic acid, propionic acid, formic acid, methanesulfonic acid and p-toluenesulfonic acid.
In the reaction, a reagent for removing a protecting group is used in an amount of about 0.5 to 1000 moles and preferably about 1 to 100 moles relative to one mole of a compound represented by general formula (8). The reaction temperature is -30 to 130°C and preferably -10 to 80°C. The reaction time is 0.1 to 100 hours and preferably 0.5 to 24 hours.

As described above, a compound represented by general formula (1) or a pharmaceutically acceptable salt thereof, in particular, a compound represented by general formula (1a) having a carbonate at the 3'-position or a compound

represented by general formula (1b) having a thiocarbonate at the 3'-position can be produced.

The compound represented by general formula (1) or a pharmaceutically acceptable salt thereof thus produced can be purified by a method commonly used in the technical field. Specific examples of the purification method include, but are not limited to, fractionation by silica gel chromatography or reversed phase chromatography and extraction with an organic layer and a water layer.

The compound of the present invention is a novel carbonate compound having a pyrrolopyrimidine skeleton or a pharmaceutically acceptable salt thereof, in this sense, a novel nucleoside derivative. The compound of the present invention can be used as a prodrug, since the compound is converted into an active nucleoside derivative having an antitumor effect by removing a carbonate *in vivo*. Since pharmacokinetics that the active nucleoside derivative originally has can be further improved by the mechanism, and additionally, toxicity that the active nucleoside derivative has can be reduced. Because of this, the compound of the present invention can be used as an extremely excellent antitumor agent.

Reduction of toxicity due to a compound of the present invention is realized in any animal species to which the compound of the present invention is administered. Whether toxicity is reduced or not can be confirmed by checking suppressions of, e.g., weight loss and reduction of blood components in the animal species receiving the compound.

If a compound of the present invention has isomers such as an optical isomer, a stereoisomer, a rotational isomer and a tautomer, individual isomers and a mixture of isomers are included in the compound of the present invention, unless otherwise specified.

A salt of a compound of the present invention refers to a pharmaceutically acceptable salt such as a base addition salt or an acid addition salt.

A compound of the present invention or a pharmaceutically acceptable salt thereof may be present in amorphous form or crystal form. A compound or a pharmaceutically acceptable salt thereof, even if it has a single crystalline form or a polymorphic mixture, is included in the compound of the present invention or a pharmaceutically acceptable salt thereof. A compound of the present invention or a pharmaceutically acceptable salt thereof may be a solvate (for example, hydrate) or a non-solvate. Both a solvate and a non-solvate thereof are included in the compound of the present invention or a pharmaceutically acceptable salt thereof. A compound labeled with, e.g., isotopes (for example, $^{3}H$, $^{14}C$, $^{35}S$, $^{125}I$) are included in the compound of the present invention or a pharmaceutically acceptable salt thereof.

The compound of the present invention or a pharmaceutically acceptable salt thereof to be used as a medical drug can be produced into various dosage forms depending on prevention or therapeutic purpose by adding, if necessary, a pharmaceutically acceptable carrier. Examples of dosage form of the medical drug include an oral preparation, an injection, a suppository, an ointment and a patch. Preferably an oral preparation or an injection is employed and more preferably an oral preparation is employed.

Examples of the dosage form of the compound of the present invention include, but are not limited to, an oral preparation or an injection containing a compound according to any one of (1) to (8) or a pharmaceutically acceptable salt thereof.

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;
(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate;
(3) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-cyclopentyl carbonothioate;
(4) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-isopropyl carbonothioate;
(5) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate;
(6) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl pentan-3-yl carbonate;
(7) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopent-3-en-1-yl carbonate; and
(8) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl (bicyclo[2.2.1]heptan-2-yl) carbonate.

The various dosage forms of the compound of the present invention are more preferably an oral preparation or an injection containing the compound according to (1) or (2) or a pharmaceutically acceptable salt thereof.

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate; or

(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate.

The various dosage forms of the compound of the present invention are preferably an oral preparation of the compound according to (1) or a pharmaceutically acceptable salt thereof, an injection of the compound according to (1) or a pharmaceutically acceptable salt thereof, an oral preparation of the compound according to (2) or a pharmaceutically acceptable salt thereof, or an injection of the compound according to (2) or a pharmaceutically acceptable salt thereof.
The dosage form of the compound of the present invention is more preferably the oral preparation (1) or the oral preparation (2) mentioned above.
These dosage forms can be each prepared by a method known to those skilled in the art.
The term of "treating" or "treatment" used in the specification includes applying a treatment for the purpose of curing cancer or ameliorating a symptom of cancer, suppressing progression, occurrence or recurrence of cancer, or mitigating a symptom.
The term "effective amount" used in the specification refers to the amount of a pharmaceutically active medical agent sufficient to induce a biological or medical response in a tissue, a system, or in an animal or a human. The effective amount is determined by a researcher, a veterinarian, a doctor or other clinicians. In one embodiment of the present invention, the "effective amount" refers to the amount of an medical-drug active ingredient sufficient to mitigate at least one clinical symptom in a human patient. In one embodiment of the present invention, "effective amount" may be a "prophylactically effective amount", i.e., an amount sufficient to prevent cancer. In one embodiment of the present invention, the "effective amount" of the medical agent which is administered in combination with an alkylating agent can be appropriately reduced from the effective amount of the medical agent singly administered in consideration of not only medicinal effects of both a compound of the present invention or a pharmaceutically acceptable salt thereof and the alkylating agent, but also side effects of them. In one embodiment of the present invention, the "effective amount" of the medical agent which is used in combination with a radiation therapy can be appropriately reduced from the effective amount of the medical agent singly administered in consideration of not only effects of both a compound of the present invention or a pharmaceutically acceptable salt thereof and the radiation therapy, but also side effects of them.
The term "subject" used in the specification includes not only a mammal but also a non-mammal, preferably a human. In one embodiment of the present invention, the subject is a human patient, more specifically, can be a human diagnosed to need a treatment for clinical symptoms or medical conditions associated with cancer disclosed in the specification. The subject may sometimes need an existing treatment for a cancer or a prophylactic treatment for preventing or reducing a risk of developing cancer. The "necessity" of the subject for an existing treatment or a prophylactic treatment for a cancer used in the specification, includes not only necessity determined by a medical professional but also a desire by the patient for the treatment.
In one embodiment of the present invention, there is provided an antitumor agent comprising a compound of the present invention or a pharmaceutically acceptable salt thereof as an active ingredient. In another embodiment of the present invention, there is provided a method for preventing and/or treating a tumor, comprising administering a compound of the present invention or a pharmaceutically acceptable salt thereof to a subject in need thereof. In further another embodiment of the present invention, there is provided use of a compound of the present invention or a pharmaceutically acceptable salt thereof for producing an antitumor agent. In further still another embodiment of the present invention, there is provided a compound of the present invention or a pharmaceutically acceptable salt thereof for use in prevention and/or treatment of a tumor.
In one embodiment of the present invention, there is provided a pharmaceutical composition comprising a compound of the present invention or a pharmaceutically acceptable salt thereof. A pharmaceutical composition according to one embodiment of the present invention comprises a compound of the present invention or a pharmaceutically acceptable salt thereof and a pharmaceutically acceptable carrier. In another embodiment of the present invention, there is provided use of a compound of the present invention or a pharmaceutically acceptable salt thereof for producing a pharmaceutical composition. In another one embodiment of the present invention, there is provided a compound of the present invention or a pharmaceutically acceptable salt thereof for use as a medical drug.
In one embodiment of the present invention, a compound of the present invention or a pharmaceutically acceptable salt thereof can be used in combination with an alkylating agent and/or a radiation therapy.
In one embodiment of the present invention, there is provided a compound of the present invention or a pharmaceutically acceptable salt thereof, which is administered in combination with an alkylating agent. In another embodiment of the present invention, there is provided a compound of the present invention or a pharmaceutically acceptable salt thereof, which is administered in combination with an alkylating agent, for producing an antitumor agent. In another one embodiment of the present invention, there is provided a compound of the present invention or a pharmaceutically acceptable salt thereof, which is administered in combination with an alkylating agent, for treating a tumor.
In the present invention, the "alkylating agent" is active *in vivo* against cancer and an optional medical-drug active ingredient different from a compound of the present invention (or a pharmaceutically acceptable salt thereof).

Examples of the alkylating agent to be used in combination include a prodrug of the alkylating agent to be used in combination, a free acid, a free base and a pharmaceutically acceptable salt. Generally, an optional and appropriate additional anti-cancer drug can be used in any combination with a compound of the present invention or a pharmaceutically acceptable salt thereof in a single-dose preparation (for example, a combination of fixed-dosage drugs) or separately in one or more dosage forms. The single-dose preparation enables simultaneously administration of medical-drug active ingredients (simultaneous administration of different medical-drug active ingredients), sequential administrations or separate administrations thereof to a subject. In a specific embodiment, a compound of the present invention and an alkylating agent are administered in combination at intervals of several minutes, several hours or several days. In one embodiment, a single or a plurality of additional anti-cancer drugs are included in a pharmaceutical product, as mentioned above.

In the present invention, examples of the "alkylating agent" include, but are not limited to, chlorambucil, chlornaphazine, chlorophosphamide, cytophosphane, estramustine, ifosfamide, mannomustine, mechlorethamine, mechlorethamine hydrochloride, melphalan, novembichin, phenesterine, prednimustine, trimustine chloroethylamine, trofosfamide, and uracil mustard; alkyl sulfonates such as busulfan, improsulfan and piposulfan; nitrosoureas such as carmustine, chlorozotocin, fotemustine, lomustine, nimustine, ranimustine, streptozotocin and TA-07; ethylene imines such as altretamine, thiotepa, triethylenemelamine, triethylenethiophosphoramide, triethylenephosphoramide and trimethylolomelamine; methylmelamine; ambamustine; bendamustine; dacarbazine; etoglucid; irofulven; mafosfamide; mitobronitol; mitolactol; pipobroman; procarbazine; temozolomide; treosulfan; triaziquone and dianhydrogalactitol.

In a preferred embodiment of the present invention, the "alkylating agent" is chlorambucil, estramustine, ifosfamide, melphalan, carmustine, fotemustine, lomustine, nimustine, ranimustine, altretamine, bendamustine, dacarbazine, procarbazine, temozolomide or dianhydrogalactitol. In a further preferred embodiment of the present invention, the "alkylating agent" is estramustine, carmustine, lomustine, nimustine, ranimustine, bendamustine, procarbazine, temozolomide or dianhydrogalactitol. In the most preferred embodiment of the present invention, the "alkylating agent" is temozolomide.

In one embodiment of the present invention, these "alkylating agents" may be used singly or in combination.

In one embodiment of the present invention, an alkylating agent can be administered in combination and a radiation therapy is further used in combination.

In one embodiment of the present invention, there is provided a compound of the present invention or a pharmaceutically acceptable salt thereof, which is used in combination with a radiation therapy. In another embodiment of the present invention, there is provided use of a compound of the present invention or a pharmaceutically acceptable salt thereof, which is used in combination with a radiation therapy, for producing an antitumor agent. In another one embodiment of the present invention, there is provided a compound of the present invention or a pharmaceutically acceptable salt thereof, which is used in combination with a radiation therapy, for treating a tumor.

Techniques for applying a radiation therapy are widely used in the technical field and described, for example, in the "Radiation Therapy Planning Guidelines 2016". These techniques can be used in the invention described in the specification.

Radiation therapies are roughly divided into an external radiation therapy and an internal radiation therapy. The external radiation therapy refers to a therapy for treating cancer by applying radiation from outside of the body, whereas, the internal radiation therapy is a therapy for treating cancer by applying radiation from the interior of the body. In the external radiation therapy, a therapy or irradiation method commonly applied such as a high-energy radiotherapy, a three-dimensional conformal radiotherapy, intensity-modulated radiotherapy (IMRT), image-guided radiotherapy (IGRT), stereotactic radiotherapy (SRT) and stereotactic radiosurgery (SRS), is selected. In the internal radiation therapy, brachytherapy, and a therapy by an unsealed radioisotope are selected. In these radiation therapies, the type of radial ray is selected from an electron ray, X-ray, $\alpha$ (alpha) ray, $\beta$ (beta) ray, $\gamma$ (gamma) ray, proton ray, heavy particle ray, depending on the carcinoma. In the present invention, the radiation therapies and radial rays mentioned above can be used in combination and are not limited to these.

In one embodiment of the present invention, a radiation therapy can be used in combination and an alkylating agent can be administered further in combination.

In one embodiment of the present invention, a compound of the present invention or a pharmaceutically acceptable salt thereof can be used alone for treating cancer, and further used in combination with a radiation therapy. When a compound of the present invention or a pharmaceutically acceptable salt thereof is used alone for treating cancer or when the compound or a salt thereof is used in combination with a radiation therapy, an alkylating agent can be administered in combination.

In one embodiment of the present invention, the compound represented by formula (I) to be used in combination with an alkylating agent and/or a radiation therapy is a compound selected from the group consisting of the following compounds:

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;

(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate;

(3) O-((2R, 3R, 4S, 5R)-5-(4-amino-5-iodo-7H-pyrrolo[2,3-d]pyrimidin-7-yl)-4-fluoro-2-(hydroxymethyl)tetrahydrofuran-3-yl) S-cyclopentyl carbonothioate;

(4) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-isopropyl carbonothioate;

(5) O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate;

(6) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl pentan-3-yl carbonate;

(7) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopent-3-en-1-yl carbonate; and

(8) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl (bicyclo[2.2.1]heptan-2-yl) carbonate,

or a pharmaceutically acceptable salt thereof

In one embodiment of the present invention, the compound of the present invention may be more preferably a compound selected from the group consisting of the following compounds:

(1) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate; and

(2) (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate, or a pharmaceutically acceptable salt thereof.

In one embodiment of the present invention, the compound represented by formula (2) to be used in combination with an alkylating agent and/or a radiation therapy is a compound selected from the group consisting of the following compounds:

(1) (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol,

(2) (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol, and

(3) (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol,

or a pharmaceutically acceptable salt thereof.

In a specific embodiment of the present invention, a compound of the present invention is administered in an effective amount.

In one embodiment, the alkylating agent is administered in an effective amount.

In a specific embodiment, the alkylating agent to be used in combination with a compound of the present invention or a pharmaceutically acceptable salt thereof is administered in a therapeutically effective amount.

In one embodiment, a compound of the present invention and an alkylating agent are simultaneously administered.

In one embodiment, a compound of the present invention and an alkylating agent are separately administered.

In one embodiment, a compound of the present invention and an alkylating agent are sequentially administered. In one embodiment, a compound of the present invention is administered before administration of an alkylating agent. In one embodiment, a compound of the present invention is administered after an alkylating agent is administered.

In one embodiment, administration of a compound of the present invention and application of a radiation therapy are simultaneously carried out.

In one embodiment, administration of a compound of the present invention and application of a radiation therapy are sequentially carried out. In one embodiment, a compound of the present invention is administered before a radiation therapy is applied. In one embodiment, a compound of the present invention is administered after a radiation therapy is applied.

In one embodiment, a compound disclosed in the present invention or a pharmaceutically acceptable salt thereof and an alkylating agent are administered in the form of a single pharmaceutical product containing these and at least one pharmaceutically acceptable carrier.

Examples of the pharmaceutically acceptable carrier include various organics or inorganic carriers commonly used as components for pharmaceutical products. Examples of the pharmaceutically acceptable carrier to be blended

in a solid formulation include an excipient, a binder, a disintegrant, a lubricant, a coating agent and a colorant. Examples of the pharmaceutically acceptable carrier to be blended in a liquid formulation include a solvent, a dissolution aid, a suspending agent, a tonicity agent, a buffer and a soothing agent. If necessary, pharmaceutical additives such as a preservative, an antioxidant, a sweetener and a stabilizer can also be used.

If an oral solid preparation is prepared, a compound of the present invention and an excipient, if necessary, e.g., a binder, a disintegrant, a lubricant, a colorant and a flavoring agent, are added. Thereafter, the mixture can be formed into tablets, coated tablets, granules, powders and capsules in accordance with a customary method.

If an injection is prepared, a compound of the present invention, a pH regulator, a buffer, a stabilizer, a tonicity agent, a local anesthetic and etc. are added. The mixture can be produced into subcutaneous, intramuscular and intravenous injections in accordance with a customary method.

The effective amount of the compound of the present invention to be blended in each of the above unit dosage form varies depending on, e.g., the symptom of the subject to which the compound is to be applied and the dosage form, is commonly 0.05 to 10000 mg for an oral preparation, 0.01 to 5000 mg for an injection, and 1 to 10000 mg for a suppository, per unit dose form as a prodrug. In one embodiment of the present invention, the effective amount of the compound of the present invention to be blended in the unit dosage form is preferably 0.05 to 1000 mg for an oral preparation, 0.01 to 500 mg for an injection, and 1 to 1000 mg for a suppository, per unit dosage form as a prodrug.

The dosage amount of a medical agent having a dosage form as mentioned above per day varies depending on the symptom, body weight, age and sex of a subject and cannot be simply determined. The dosage amount of a compound of the present invention can be usually 0.05 to 50000 mg per adult (body weight: 50 kg) per day and preferably 0.1 to 10000 mg as a prodrug. The dosage amount is preferably 0.05 to 1000 mg for an oral preparation, 0.01 to 500 mg for an injection and 1 to 1000 mg for a suppository, as a prodrug.

Examples of the tumor to which a compound of the present invention is to be applied include, but are not particularly limited to, head and neck cancer (e.g. oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

Examples of the brain tumor to be treated by a compound of the present invention include a metastatic brain tumor and a primary brain tumor.

Examples of the brain tumor include, but are not particularly limited to, metastatic brain tumors (for example, brain metastasis of, e.g., lung cancer, breast cancer, stomach cancer, colorectal cancer, bladder cancer, biliary tract cancer and uterine cancer), pilocytic astrocytoma, diffuse astrocytoma, oligodendroglioma/oligoastrocytoma, anaplastic astrocytoma/anaplastic oligodendroglioma, anaplastic oligoastrocytoma, glioblastoma, ependymoma, anaplastic ependymoma, ganglioglioma, central neurocytoma, medulloblastoma, germinoma, central nervous system malignant lymphoma, meningioma, schwannoma, GH producing pituitary adenoma, PRL producing pituitary adenoma, ACTH producing pituitary adenoma, non-functional pituitary adenoma, craniopharyngioma, chordoma, hemangioblastoma and epidermoid tumor.

Since a compound of the present invention is used as a prodrug, the compounds represented by the following formulas, respectively which are the active compounds represented by:

(2R,3R,4S,5R)-5-(4-amino-5-iodo-7H-pyrrolo[2,3-d]pyrimidin-7-yl)-4-fluoro-2-(hydroxymethyl) tetrahydrofuran-3-ol;
(2R,3R,4S,5R)-5-(4-amino-5-bromo-7H-pyrrolo[2,3-d]pyrimidin-7-yl)-4-fluoro-2-(hydroxymethyl) tetrahydrofuran-3-ol; and
(2R,3R,4S,5R)-5-(4-amino-5-chloro-7H-pyrrolo[2,3-d]pyrimidin-7-yl)-4-fluoro-2-(hydroxymethyl) tetrahydrofuran-3-ol

or pharmaceutically acceptable salts thereof, are applied to the same carcinomas as mentioned above.

[Formula 8]

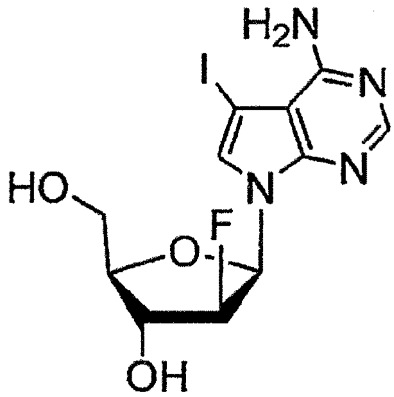

[Formula 9]

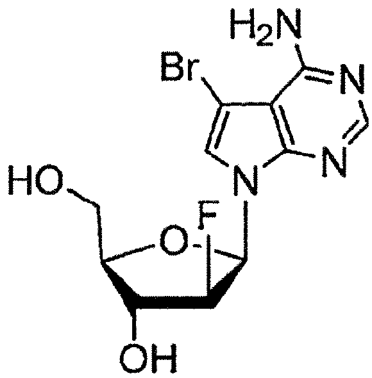

[Formula 10]

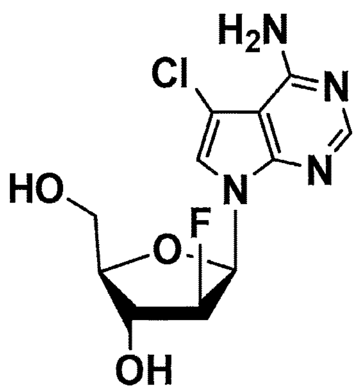

Examples

The present invention will be more specifically described by way of Examples and Test Examples shown below, but the present invention is not limited to these Examples.

As the reagents used in Examples, commercially available reagents were used unless otherwise specified. Silica gel column chromatography and basic silica gel column chromatography were carried out by using prepacked

columns manufactured by Shoko Science Co., Ltd. or Biotage Ltd.

Reverse phase preparative HPLC column chromatography was carried out in the following conditions. Injection amount and gradient were appropriately set.

Column: CAPCELL PAK C18 MGIII, 30 × 50 mm, 5 μm, manufactured by OSAKA SODA
UV detection: 254 nm
Column flow rate: 40 mL/minute
Mobile phase: water/acetonitrile (0.1% formic acid)
Injection amount: 0.1-1.0 mL
Gradient water/acetonitrile 10% → 90% (7 minutes)

NMR spectra were measured by use of AL400 (400 MHz; manufactured by JEOL Ltd.(JEOL)), Mercury 400 (400 MHz; manufactured by Agilent Technologies), AVANCE NEO (400 MHz; Bruker), AVANCE III HD (500 MHz; Bruker)-type spectrometer. When tetramethylsilane is contained in a deuterated solvent, tetramethylsilane was used as the internal standard. In the other cases, an NMR solvent was used as the internal standard. All δ values were indicated by ppm.

LC-MS spectra were measured by use of SQD manufactured by Waters in the following two conditions. [M+H]+ values were shown.
MS detection: ESI positive
UV detection: 254 and 210 nm,
Column flow rate: 0.5 mL/minute
Mobile phase: water/acetonitrile (0.1% formic acid) injection amount: 1 μL
Column: Acquity BEH, 2.1 × 50 mm, 1.7 μm Gradient:

| Measurement time (minutes) | Water/Acetonitrile (0.1% formic acid) | |
|---|---|---|
| 0 | 95 | 5 |
| 0.1 | 95 | 5 |
| 2.1 | 5 | 95 |
| 3.0 | STOP | |

What are meant by the following abbreviations:

| | |
|---|---|
| s: | singlet |
| d: | doublet |
| t: | triplet |
| q: | quartet |
| dd: | double doublet |
| m: | multiplet |
| br: | broad |
| brs: | broad singlet |
| d6-DMSO: | deuterated dimethyl sulfoxide |
| D2O: | heavy water |
| HPMC: | hydroxypropyl methylcellulose |

[Comparative Example 1]

Synthesis of (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol

[Formula 11]

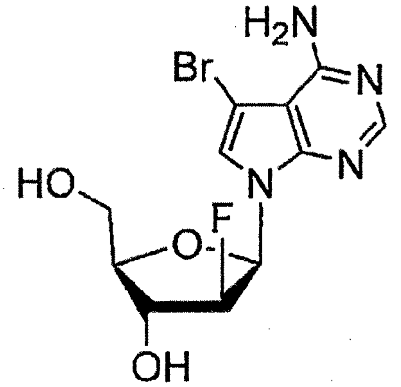

(Step 1)

5-Bromo-4-chloro-7H-pyrrolo[2,3-d]pyrimidine (2.2 g) was suspended in acetonitrile (80 mL). To the mixture, tris(2-(2-methoxyethoxy)ethyl)amine (0.17 mL) and powdery potassium hydroxide (1.1 g) were added. The mixture was stirred while heating at 50°C for 5 hours. To this mixed solution, an acetonitrile (20 mL) solution of ((2R,3R,4S,5R)-3-(benzoyloxy)-5-bromo-4-fluorotetrahydrofuran-2-yl) methylbenzoate (4.5 g) obtained by a method described in the literature (Bioorg. Med. Chem, 20, 2012,5202-5214) was added at room temperature. The resultant mixed solution was stirred at room temperature for 1.5 hours. A part of the solution was taken and subjected to LC-MS analysis. Based on the LC-MS spectrum, the presence of ((2R,3R,4S,5R)-3-(benzoyloxy)-5-(5-bromo-4-chloro-7H-pyrrolo[2,3-d]pyrimidin-7-yl)-4-fluorotetrahydrofuran-2-yl) methyl benzoate was confirmed. Thereafter, purification was carried out in accordance with a method commonly used to obtain the benzoate (5.1 g).

(Step 2)

The benzoate (3.0 g) obtained in step 1 of Comparative Example 1 was suspended in a mixed solution of a 25% aqueous solution of ammonia (16 mL) and 1,4-dioxane (16 mL). The suspension was stirred at 120°C for 5 hours. A part of the solution was taken and subjected to LC-MS analysis. Based on the LC-MS spectrum, the presence of the title compound was confirmed.
Thereafter, purification was carried out in accordance with a method commonly used to obtain the title compound (Br-form) (1.3 g).
$^1$H-NMR (d6-DMSO) $\delta$ (ppm): 3.55-3.67 (2H, m), 3.74-3.78 (1H, m), 4.29-4.37 (1H, m), 5.02-5.17 (1H, m), 5.06 (1H, t, J = 5.6 Hz), 5.87 (1H, d, J = 4.8 Hz), 6.52 (1H, dd, J = 4.4, 14 Hz), 6.83 (2H, brs), 7.48 (1H, d, J = 2.0 Hz), 8.09 (1H, s)
ESI-MS: m/z 347, 349 (MH+)

[Comparative Example 2]

Synthesis of (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol

[Formula 12]

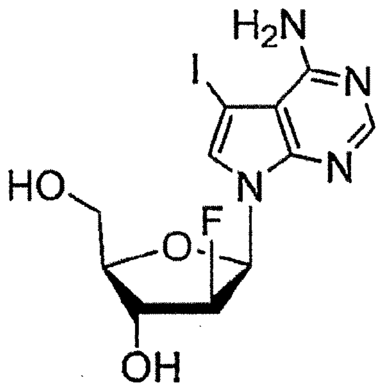

The title compound was synthesized in accordance with a method described in the literature (Bioorg. Med. Chem, 20, 2012, 5202-5214).

[Comparative Example 6]

Synthesis of (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol

[Formula 13]

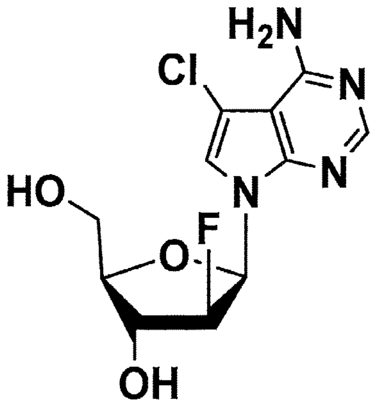

The title compound was synthesized from 5-chloro-4-chloro-7H-pyrrolo[2,3-d]pyrimidine in accordance with the method described in Comparative Example 1.

$^{1}$H-NMR (d6-DMSO) δ (ppm): 3.63-3.79 (3H, m), 4.34-4.39 (1H, m), 5.09-5.20 (2H, m), 5.90 (1H, s), 6.56 (1H, d, J = 12 Hz), 6.93 (2H, brs), 7.46 (1H, s), 8.12 (1H, s) ESI-MS: m/z 303, 305 (MH+)

[Comparative Example 7]

Synthesis of (2R,3R,4S,5R)-5-(4-amino-5-iodo-7H-pyrrolo[2,3-d]pyrimidin-7-yl)-4,4-difluoro-2-(hydroxymethyl) tetrahydrofuran-3-ol

[Formula 14]

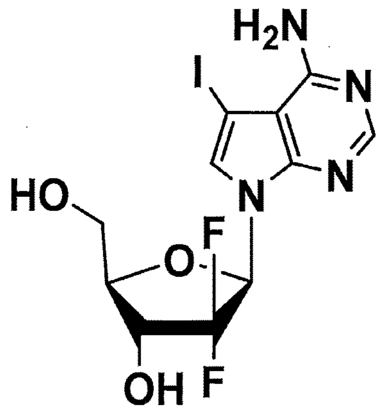

The title compound was synthesized in accordance with a method described in the literature (ChemMedChem, 8, 2013, 832-846).

[Example 1]

Synthesis of (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate

(Step 1)

(2R,3R,4S,5R)-5-(4-Amino-5-iodo-7H-pyrrolo[2,3-d]pyrimidin-7-yl)-4-fluoro-2-(hydroxymethyl) tetrahydrofuran-3-ol (500 mg) obtained in Comparative Example 2 was dissolved in pyridine (3 mL). To this solution, tert-butyldimethylchlorosilane (249 mg) was added. The reaction solution was stirred at room temperature for 12 hours. Methanol (1 mL) was added to the reaction solution, which was stirred for 20 minutes and then concentrated under reduced pressure. The resultant residue was suspended with ethyl acetate (5 mL) and 1 N hydrochloric acid (5 mL). The solid matter generated was filtered to obtain (2R,3R,4S,5R)-5-(4-amino-5-iodo-7H-pyrrolo[2,3-d]pyrimidin-7-yl)-2-(((tert-butyldimethylsilyl)oxy)methyl)-4-fluorotetrahydrofuran-3-ol (460 mg) .

(Step 2)

Cyclopentanol (1 g) was dissolved in pyridine (10 mL). To this solution, 1,1'-carbonyldiimidazole (2.0 g) was added. The reaction solution was stirred under ice cooling for 3 hours and concentrated under reduced pressure. To the concentrated reaction solution, ethyl acetate (10 mL) and a saturated aqueous solution of ammonium chloride (10 mL) were added. After layers were allowed to separate, the organic layer was washed sequentially with a saturated aqueous solution of ammonium chloride (10 mL), water (10 mL), and a saturated saline solution (10 mL), in this order and dried over sodium sulfate. After sodium sulfate was removed by filtration, the resultant solution was concentrated under reduced pressure and subjected twice to azeotropic distillation with toluene (10 mL) to obtain cyclopentyl 1H-imidazole-1-carboxylate (1.5 g).

(Step 3)

(2R,3R,4S,5R)-5-(4-Amino-5-iodo-7H-pyrrolo[2,3-d]pyrimidin-7-yl)-2-(((tert-butyldimethylsilyl)oxy)methyl)-4-fluorotetrahydrofuran-3-ol (460 mg) obtained in step 1 of this Example was dissolved in pyridine (3 mL). To this solution, cyclopentyl 1H-imidazole-1-carboxylate (163 mg) obtained in step 2 of this Example, and subsequently, 1,8-diazabicyclo[5.4.0]undec-7-en (145 mg) were added. The reaction solution was stirred at 70°C for 5 hours. To the reaction solution, ethyl acetate (10 mL) and water (10 mL) were added. After layers were allowed to separate, the organic layer was washed twice with 1 N hydrochloric acid (5 mL), subsequently with a saturated saline solution (10 mL) and dried over sodium sulfate. After the sodium sulfate was removed by filtration, the solution was concentrated under reduced pressure. The resultant residue was purified by silica gel column chromatography (hexane/ethyl acetate 60%) to obtain (2R,3R,4S,5R)-5-(4-amino-5-iodo-7H-pyrrolo[2,3-d]pyrimidin-7-yl)-2-(((tert-butyldimethylsilyl)oxy)methyl)-4-fluorotetrahydrofuran-3-yl cyclopentyl carbonate (470 mg).

(Step 4)

(2R,3R,4S,5R)-5-(4-Amino-5-iodo-7H-pyrrolo[2,3-d]pyrimidin-7-yl)-2-(((tert-butyldimethylsilyl)oxy)methyl)-4-fluorotetrahydrofuran-3-yl cyclopentyl carbonate (440 mg) obtained in step 3 of this Example was dissolved in ethanol (5 mL). To the solution, 1 N hydrochloric acid (1 mL) was added. The reaction solution was stirred at 60°C for 2 hours and directly purified by silica gel column chromatography (hexane/ethyl acetate 90%) to obtain (2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate (I form) (230 mg).

The compounds of Examples 2 to 21 were synthesized in accordance with the production process of Example 1. "Comparative Example 1" and "Comparative Example 2" described in the column of "starting substance" of Table 1 represent the compound (Br form) of Comparative Example 1 and the compound (I form) of Comparative Example 2, respectively, which were used (in place of the compound of Comparative Example 2 to be used) in step 1 of Examples. Alcohols listed in the column of "Alcohol (R-OH)" in Table 1 represent alcohols used in place of cyclopentanol of step 2 of Example 1. These alcohols are all commercially available. The chemical names, chemical structures and physical property values of the compounds of Examples 1 to 21 are shown in Table 1-1 to Table 1-6.

[Table 1-1]

[Table 1]

| Example | Starting substance | Alcohol (R-0H) | Skeleton | Compound name | 1H-NMR δ (ppm) | ESI-MS |
|---|---|---|---|---|---|---|
| 1 | Comparative Example 2 | 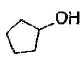 | 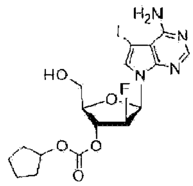 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl cyclopentyl carbonate | (d6-DMS0) 1.52-1.90(8H, m), 3.58-3.71 (2H, m), 3.99-4.03(1H, m), 5.02-5.05 (1H, m). 5.16 (1H, t, J = 5.2 Hz), 5.24-5.50 (2H, m). 6.50 (1H, dd, J = 18, 4.0 Hz), 6.72 (2H, brs), 7.53 (1H, d, J = 2.4 Hz), 8.10(1H, s) | m/z 507 (MH+) |

(continued)

| Example | Starting substance | Alcohol (R-0H) | Skeleton | Compound name | 1H-NMR δ (ppm) | ESI-MS |
|---|---|---|---|---|---|---|
| 2 | Comparative Example 2 | 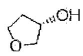 | 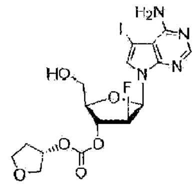 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl (tetrahydrofuran-3-yl) carbonate | (d6-DMS0) 1.95-2.20(2H, m), 3.59-3.84 (6H, m), 4.03 (1H, q, J = 4.4 Hz), 5.15-5.21 (2H, m), 5.25-5.31(1H, m), 5.34-5.53 (1H, m). 6.50 (1H, dd, J = 4.0, 18 Hz), 6.72(2H, brs), 7.52(1H, d, J = 2.8 Hz), 8.10 (1H, s) | m/z 509 (MH+) |
| 3 | Comparative Example 2 | 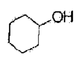 | 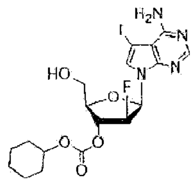 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl cyclohexyl carbonate | (d6-DMS0) 1.21-1.83 (10H, m), 3.58-3.71 (2H, m), 4.00-4.04 (1H, m), 4.54-4.61(1H, m), 5.16(1H, t, J = 5.6 Hz), 5.26-5.51 (2H, m), 6.51(1H, dd, J = 18, 4.4 Hz), 6.71(2H, brs), 7.53(1H, d, J = 2.8 Hz), 8.10(1H. s) | m/z 521 (MH+) |
| 4 | Comparative Example 2 | 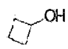 | 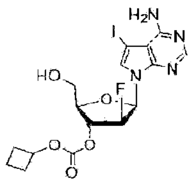 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl cyclobutyl carbonate | (d6-DMS0) 1.50-2.31(6H, m), 3.58-3.71 (2H, m), 4.00-4.03(1H, m), 4.85-4.92 (1H, m), 5.16 (1H, t, J = 6.4 Hz), 5.25-5.50 (2H, m), 6.50 (1H, dd, J = 4.4, 18 Hz), 6.72(2H, brs), 7.53(1H, d, J = 2.4 Hz), 8.10 (1H, s) | m/z 493 (MH+) |

[Table 1-2]

| | | | | | | |
|---|---|---|---|---|---|---|
| 5 | Comparative Example 2 | 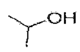 | 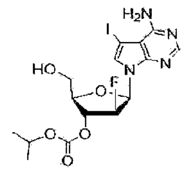 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl isopropyl carbonate | (d6-DMS0) 1.24(6H, d, J = 6.0 Hz), 3. 58-3.71 (2H, m), 4.00-4.03(1H, m), 4.77-4. 83(1H, m), 5.17(1H, t, J = 6.0 Hz), 5.25-5.50 (2H, m), 6.50 (1H, dd, J = 4.0, 18 Hz), 6.71 (2H, brs), 7.54(1H, d, J = 2.4 Hz), 8.10(1H, s) | m/z 481 (MH+) |
| 6 | Comparative Example 2 | 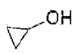 | 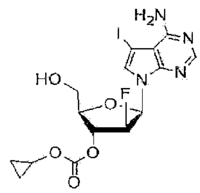 | (2R,3R,4S,5R)-5-(4-Amino-5-iodo-7H-pyrrolo [2,3-d]pyrimidin-7-yl)-4-fl uoro-2-(hydroxymethyl) tetrahydrofuran-3-yl cyclopropyl carbonate | (d6-DMS0) 0.65-0.75 (4H, m), 3.57-3.69(2H, m), 3.98-4.02(1H, m), 4.09-4.14(1H, m), 5.15 (1H, t, J = 6.0 Hz), 5.25-5.49(2H, m), 6.48 (1H, dd, J = 18, 4.0 Hz), 6.70(2H, brs), 7.52(1H, d, J = 2.4 Hz), 8.08(1H, s) | m/z 479 (MH+) |
| 7 | Comparative Example 2 | 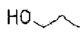 | 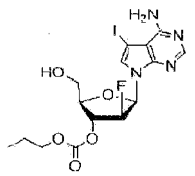 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl propyl carbonate | (d6-DMS0)0.88(3H,t,J = 7.6 Hz), 1.58-1.66 (2H, m), 3.59-3.71(2H, m), 4.01-4. 04(1H, m), 6.02-5.05(1H, m), 4.08 (2H, t, J = 6.8 Hz), 5.17 (1H, t, J = 5.6 Hz), 5.27-5.51(2H, m), 6.51 (1H, dd, J = 18, 4.4 Hz), 6.72(2H, brs), 7.54(1H, d, J = 2.0 Hz), 8.10(1H, s) | m/z 481 (MH+) |
| 8 | Comparative Example 2 | 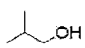 | 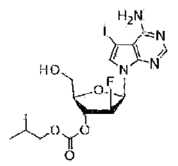 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl 2-methylpropyl carbonate | (d6-DMS0) 0.89(6H, d, J = 6.8 Hz), 1.86-1.96 (1H, m), 3.69-3.70 (2H, m), 3.93 (2H, d, J = 6.8 Hz), 4.02-4.05(1H, m), 5.17(1H, t, J = 5.6 Hz), 5.27-5.52(2H, m), 6.52 (1H, dd, J = 18, 4.0 Hz), 6.72(2H, brs), 7.54(1H, d, J = 2.4 Hz), 8.10(1H, s) | m/z 495 (MH+) |

[Table 1-3]

| | | | | | | |
|---|---|---|---|---|---|---|
| 9 | Comparative Example 2 | 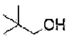 | 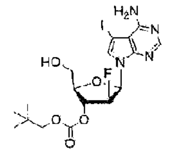 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl 2,2-dimethylpropyl carbonate | (d6-DMS0) 0.90(9H, s), 3.60-3.70(2H, m), 3.85(2H, d, J = 1.6 Hz), 4. 02-4.06 (1H, m), 5.17(1H, t, J = 5.6 Hz), 5.28-5.53 (2H, m), 6. 52(1H, dd, J = 17, 4.0 Hz), 6.72(2H, brs), 7.54(1H, d, J = 2.4 Hz), 8.10(1H, s) | m/z 509 (MH+) |
| 10 | Comparative Example 2 | 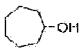 | 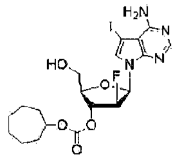 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl cycloheptyl carbonate | (d6-DMS0) 1.27-2.01(12H, m), 3.64-3.77(2H, m), 4.06-4.09(1H, m), 4.77-4.84(1H, m), 1 5.22(1H, t, J = 6.4 Hz), 5.31-5.56(2H, m), 6.57(1H, dd, J = 18, 4.4 Hz), 6.77(2H, brs), 7.60(1H, d, J = 2.4 Hz), 8.17(1H, s) | m/z 535 (MH+) |
| 11 | Comparative Example 2 | 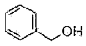 | 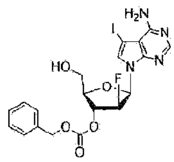 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl benzyl carbonate | (d6-DMS0) 3.58-3.69(2H, m), 4.01-4.04(1H, m), 5.13-5.21(3H, m), 5.30-5.53(2H, m), 6.51(1H, dd, J = 18, 4.4 Hz), 6.71(2H, brs), 7.32-7.40(5H, m), 7.53(1H, d, J = 2.4 Hz), 8.09(1H, s) | m/z 529 (MH+) |
| 12 | Comparative Example 2 | 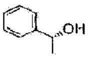 | 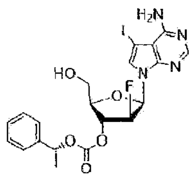 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl (1-phenylethyl) carbonate | (d6-DMS0) 1.53(3H, d, J = 6.4 Hz), 3.55-3.68(2H, m), 4.03-4.05(1H, m), 5.27-5.57 (2H, m), 5.68-5.73(1H, m), 6.56(1H, dd, J = 17, 4.4 Hz), 7.30-7.38 (5H, m), 7.76(1H, s), 8.33 (1H, s) | m/z 543 (MH+) |

[Table 1-4]

| | | | | | | |
|---|---|---|---|---|---|---|
| 13 | Comparative Example 2 | 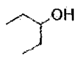 | 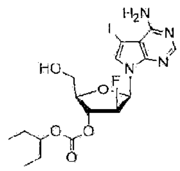 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl pentan-3-yl carbonate | (d6-DMS0) 0.85-0.90 (6H, m), 1.53-1.67 (4H, m), 3.63-3.74 (2H, m), 4.04-4.06 (1H, m). 4.55-4.60 (1H, m), 5.21(1H, t, J = 6.5 Hz), 5.32-5.53 (2H, m), 6.56(1H, dd, J = 17, 4.5 Hz), 6.75 (2H, brs), 7.58(1H, d, J = 2.5 Hz), 8.14(1H, s) | m/z 509 (MH+) |
| 14 | Comparative Example 2 | 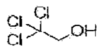 | 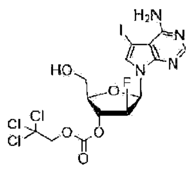 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl 2,2,2-trichloroethyl carbonate | (d6-DMS0) 3.66-3.77 (2H, m), 4.13-4.16 (1H, m), 4.98-5.04 (2H, m), 5.23-5.25 (1H, m), 5.43-5.63 (2H, m), 6.60(1H, dd, J = 17, 4.5 Hz), 6. 76 (2H, brs), 7.60(1H, d, J = 2.5 Hz), 8.14(1H, s) | m/z 569, 571, 573 (MH+) |
| 15 | Comparative Example 2 | 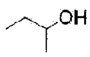 | 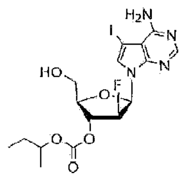 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl 1-methyl propyl carbonate | (d6-DMS0) 0.87-0.91 (3H, m), 1.24-1.26 (3H, m), 1.55-1.66 (2H, m), 3.62-3.75 (2H, m), 4.04-4.07 (1H, m), 4.64-4.72 (1H, m), 5.20(1H, t, J = 6.0 Hz), 5.30-5.55 (2H, m), 6.55(1H, dd, J = 18, 4.0 Hz), 6.74 (2H. brs), 7.57(1H, d, J = 2.4 Hz), 8.14(1H, s) | m/z 495 (MH+) |
| 16 | Comparative Example 2 | 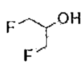 | 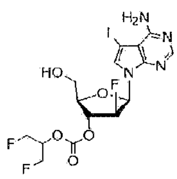 | (2R,3R,4S,5R)-5-(4-Amino-5-iodo-7H-pyrrolo[2,3-d] pyrimidin-7-yl)-4-fluoro-2-(hydroxymethyl) tetrahydrofuran-3-yl 1-(fluoromethyl)-2-fluoroethyl carbonate | (d6-DMS0) 3.64-3.76 (2H, m), 4.09-4.12 (1H, m), 4.59-4.84 (4H, m), 5.11-5.26 (1H, m), 5.22 (1 H, t, J = 6.0 Hz), 5.35-5.60 (2H, m), 6.57(1H, dd, J = 18, 4.4 Hz), 6.75 (2H, brs), 7.58(1H, d, J = 2.4 Hz), 8.14(1H, s) | m/z 517 (MH+) |

[Table 1-5]

| | | | | | | |
|---|---|---|---|---|---|---|
| 17 | Comparative Example 2 | 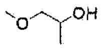 | 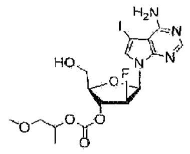 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl (2-methoxy-1-methylethyl) carbonate | (d6-DMS0) 1.24(3H, d, J = 6.4 Hz), 3.28 (3H, s), 3.43-3.45(2H, m), 3.66-3.74 (2H, m), 4.04-4.08(1H, m), 4.84-4.93 (1H, m), 5.19-1 5.23 (1H, m), 5.30-5.55(2H, m), 6.52-6.58 (1H, m), 6.75 (2H, brs), 7.57-7.68(1H, m), 8.14(1H, s) | m/z 511 (MH+) |
| 18 | Comparative Example 2 | 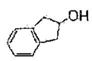 | 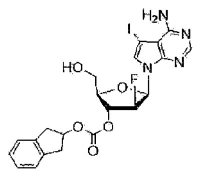 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl (1H,2H,3H-inden-2-yl) carbonate | (d6-DMS0) 3.03-3.09(2H, m), 3.30-3.36 (2H, m), 3.62-3.74 (2H, m), 4.02-4.05(1H, m), 5.19(1H, t, J = 6.0 Hz), 5.29-5.54(3H, m), 6.51(1H, dd, J = 18, 4.4 Hz), 6.74(2H, brs), 7.17-7.30(4H, m), 7.56(1H, d, J = 2.4 Hz), 8.12(1H, s) | m/z 555 (MH+) |

(continued)

| | | | | | | |
|---|---|---|---|---|---|---|
| 19 | Comparative Example 2 | 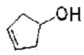 | 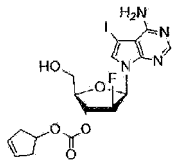 | (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl cyclopent-3-en-1-yl carbonate | (d6-DMS0) 1.74-1.79 (2H, m), 2.41-2.48 (1H, m), 2.66-2.78 (2H, m), 3.58-3.76 (3H, m), 4.06-4.10 (2H, m), 5.23-5.37 (2H, m), 5.43-5.59 (1H, m), 5.74-5.78 (2H, m), 6.56 (1H, dd, J = 17.3, 4.2 Hz), 7.68-7.74 (1H, m), 8.26 (1H, s) | m/z 555 (MH+) |
| 20 | Comparative Example 2 | 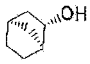 Racemic mixture relative configuration display | 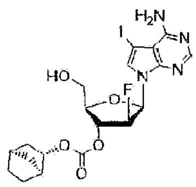 | Mixture of diastereomers of (2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl ((1S,2S, 4R)-bicyclo[2.2.1] heptan-2-yl carbonate | (d6-DMS0) 1.02-1.22 (3H, m), 1.36-1.57 (4H, m), 1.70-1.79 (1H, m), 2.26-2.39 (3H, m), 3.62-3.77 (3H, m), 4.04-4.13 (2H, m), 4.49-4.57 (1H, m), 5.28-5.37 (1H, m), 5.42-5.60 (1H, m), 6.57 (1H, dd, J = 16.9, 4.2 Hz), 7.77 (1H, s), 8.32 (1H, s) | m/z 533 (MH+) |

[Table 1-6]

| | | | | | | |
|---|---|---|---|---|---|---|
| 21 | Comparative zxample 1 | 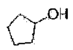 | 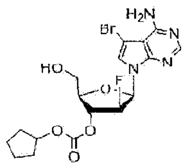 | (2R,3R,4S,5R)-5-(4-Amino-5-bromo-7H-pyrrolo[2,3-d]pyrimidin-7-yl)-4-fluoro-2-(hydroxymethyl)tetrahydrofuran-3-yl cyclopentyl carbonate | (d6-DMS0) 1.52-1.87 (8H, m), 3.59-3.71 (2H, m), 4.00-4.04 (1H, m), 5.02-5.05 (1H, m), 5.16(1H, t, J = 6.4 Hz), 5.25-5.52 (2H, m), 6.50(1H, dd, J = 18, 4.4 Hz), 6.87 (2H, brs), 7.51(1H, d, J = 2.4 Hz), 8.11(1H, s) | m/z 459, 461 (MH+) |

[Example 22]

Synthesis of O-(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate

(Step 1)

(2R,3R,4S,5R)-5-(4-Amino-5-iodo-7H-pyrrolo[2,3-d]pyrimidin-7-yl)-2-(((tertbutyldimethylsilyl)oxy)methyl)-4-fluorotetrahydrofuran-3-ol (80 mg) obtained in step 1 of Example 1 and N,N-diisopropylethylamine (0.15 mL) were dissolved in N,N-dimethylformamide (1.5 mL). To the solution, bis(4-nitrophenyl)carbonate (72 mg) was added and the resultant solution was stirred at room temperature for one hour. After ethanethiol (0.15 mL) was added, the solution was stirred for further one hour. Ethyl acetate (5 mL) and a 0.5 N aqueous solution of sodium hydroxide (5 mL) were added. The layers were allowed to separate and the organic layer was washed sequentially with a 0.5 N aqueous solution of sodium hydroxide (5 mL) and a saturated saline solution (5 mL) in this order, and concentrated under reduced pressure. The residue obtained was purified by silica gel column chromatography (methanol/chloroform 5%) to obtain O-((2R,3R,4S,5R)-5-(4-amino-5-iodo-7H-pyrrolo[2,3-d]pyrimidin-7-yl)-2-(((tert-butyldimethylsilyl)oxy)methyl)-4-fluorotetrahydrofuran-3-yl) S-ethyl carbonothioate (57 mg).

(Step 2)

O-((2R,3R,4S,5R)-5-(4-Amino-5-iodo-7H-pyrrolo[2,3-d]pyrimidin-7-yl)-2-(((tert-butyldimethylsilyl)oxy)methyl)-4-fluorotetrahydrofuran-3-yl) S-ethyl carbonothioate (57 mg) obtained in step 1 of this Example was dissolved in ethanol (3 mL). To the solution, 6 N hydrochloric acid (0.5 mL) was added. The reaction solution was stirred at room temperature for 2 hours and directly purified by silica gel column chromatography (methanol/chloroform 10%) to obtain O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate (23 mg).

Compounds of Examples 23 to 25 were synthesized in accordance with the production process of Example 22. "Comparative Example 1" and "Comparative Example 2" described in the column of "starting substance" of Table 2 represent the compound (Br form) of Comparative Example 1 and the compound (I form) of Comparative Example 2, respectively, which were used (in place of the compound of Comparative Example 2 to be used) in step 1 of Examples. In the column of "Thiol (R-SH)" in Table 2, thiols used in place of ethanethiol of step 1 of Example 22 are listed. These thiols are all commercially available. The chemical names, chemical structures and physical property values of the compounds of Examples 22 to 25 are listed in Table 2.

[Table 2]

[Table 2]

| Example | Starting substance | Thiol (R-SH) | Skeleton | Compound name | 1H-NMR δ (ppm) | ESI-MS |
|---|---|---|---|---|---|---|
| 22 | Comparative Example 2 | 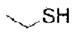 | 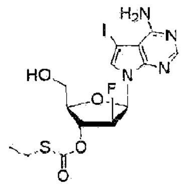 | O-((2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl) S-ethyl carbonothioate | (d6-DMS0) 1.32 (3H, t, J = 7.5 Hz), 2.97(2H, q, J = 7.5 Hz), 3. 65-3. 77 (2H, m), 4.08-4.10 (1H, m), 5.26(1H, t, J = 6.0 Hz), 5.46-5.66(2H, m), 6.59(1H, dd, J = 17, 4.0 Hz), 6.77 (2H, brs), 7.62 (1H, d, J = 2.5 Hz), 8.18(1H, s) | m/z 483 (MH+) |
| 23 | Comparative Example 2 | 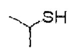 | 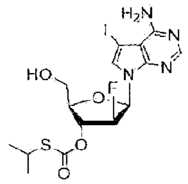 | O-((2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl) S-isopropyl carbonothioate | (d6-DMS0) 1. 35 (6H, d, J = 7.0 Hz), 3. 50-3. 58(1H, m), 3.61-3.72(2H, m), 4.04-4.06 (1H, m), 5.22(1H, t, J = 5.5 Hz), 5.43-5. 62 (2H, m), 6.55(1H, dd, J = 4.5, 17 Hz), 6.76(2H, brs), 7.58 (1H, d, J = 2.5 Hz), 8.14(1H, s) | m/z 497 (MH+) |
| 24 | Comparative Example 2 | 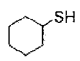 | 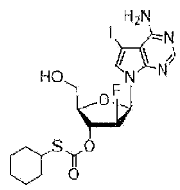 | O-((2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl) S-cyclohexyl carbonothioate | (d6-DMS0) 1.28-2.04(10H, m), 3.39-3.47(1H, m), 3.64-3.77(2H, m), 4.06-4.10(1H, m), 4.54-4.61(1H, m), 5. 25(1H, t, J = 6.4 Hz), 5.44-5.65 (2H, m), 6.58(1H, dd, J = 17, 4.4 Hz), 6.78(2H, brs), 7.61(1H, d, J = 2.8 Hz), 8.17(1H, s) | m/z 537 (MH+) |
| 25 | Comparative Example 2 | 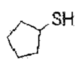 | 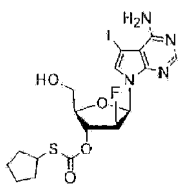 | O-((2R,3R,4S,5R)-5-(4-Amino-5-iodo-7H-pyrrolo [2,3-d]pyrimidin-7-yl)-4-fluoro-2-(hydroxymethyl) tetrahydrofuran-3-yl) S-cyclopentyl carbonothioate | (d6-DMS0) 1.56-2.16(8H, m), 3.64-3.77(3H, m), 4.07-4.10(1H, m), 5.25(1H, t, J = 6.0 Hz), 5.45-5.66 (2H, m), 6.58(1H, dd, J = 17, 4.4 Hz), 6. 78(2H, brs), 7.61(1H, d, J = 2.4 Hz), 8.18(1H, s) | m/z 523 (MH+) |

[Example 26]

Synthesis of O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) O-isopropyl

carbonothioate

[Formula 15]

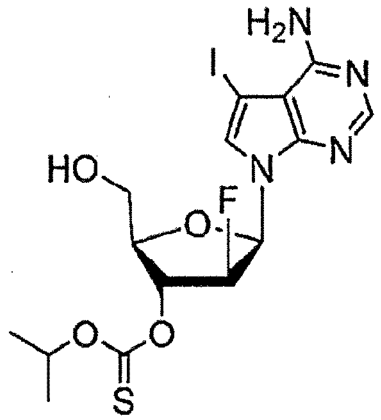

The title compound was synthesized in accordance with the synthesis process of Example 1 except that 1,1'-thiocarbonyldiimidazole was used in place of 1,1'-carbonyldiimidazole and isopropanol was used in place of cyclopentanol in step 2.

$^1$H-NMR (d6-DMSO) δ (ppm): 1.41 (6H, dd, J = 6.4, 2.0 Hz), 3.69-3.81 (2H, m), 4.17-4.20 (1H, m), 5.27 (1H, t, J = 6.0 Hz), 5.35-5.93 (3H, m), 6.58 (1H, dd, J = 3.6, 20Hz), 6.79 (2H, brs), 7.61 (1H, d, J = 2.8 Hz), 8.18 (1H, s) ESI-MS: m/z 497 (MH+)

[Example 27]

Synthesis of (2R,3R,4S,5R)-5-(4-amino-5-chloro-7H-pyrrolo[2,3-d]pyrimidin-7-yl)-4-fluoro-2-(hydroxymethyl)tetrahydrofuran-3-yl cyclopentyl carbonate

[Formula 16]

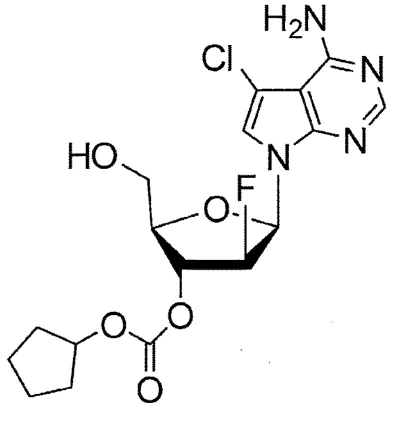

The title compound was synthesized from (2R,3R,4S,5R)-5-(4-Amino-5-chloro-7H-pyrrolo[2,3-d]pyrimidin-7-yl)-4-fluoro-2-(hydroxymethyl) tetrahydrofuran-3-ol (500 mg) obtained in Comparative Example 6 and cyclopentanol in accordance with the synthesis process of Example 1.

$^1$H-NMR (d6-DMSO): 1.56-1.89 (8H, m), 3.62-3.73 (2H, m), 4.05-4.06 (1H, m), 5.06-5.09 (1H, m), 5.20 (1H, t, J = 5.6 Hz), 5.29-5.55 (2H, m), 6.56 (1H, dd, J = 18, 4.0 Hz), 6.96 (2H, brs), 7.49 (1H, d,J = 2.0 Hz), 8.14 (1H, s)

ESI-MS: m/z 415, 417 (MH +)

[Comparative Example 3]

Synthesis of ((2R,3R,4S,5R)-5-(4-amino-5-iodo-7H-pyrrolo[2,3-d]pyrimidin-7-yl)-4-fluoro-3-hydroxy tetrahydrofuran-2-yl) methyl cyclopentyl carbonate

[Formula 17]

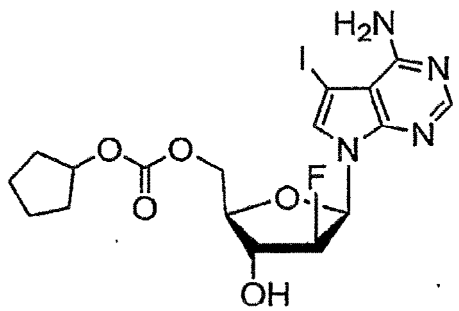

The title compound was synthesized from cyclopentyl 1H-imidazole-1-carboxylate obtained in step 2 of Example 1 in accordance with the process of step 3 of Example 1 except that (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol obtained in Comparative Example 2 was used in place of (2R,3R,4S,5R)-5-(4-amino-5-iodo-7H-pyrrolo[2,3-d]pyrimidin-7-yl)-2-(((tert-butyldimethylsilyl)oxy)methyl)-4-fluorotetrahydrofuran-3-ol.

$^1$H-NMR (d6-DMSO) δ (ppm): 1.49-1.86 (8H, m), 3.96-4.00 (1H, m), 4.27-4.39 (3H, m), 4.97-5.19 (2H, m), 6.07 (1H, d, J = 4.8 Hz), 6.56 (1H, dd, J = 16, 4.4 Hz), 6.70 (2H, brs), 7.40 (1H, d, J = 2.0 Hz), 8.10 (1H, s)

ESI-MS: m/z 507 (MH+)

[Comparative Example 4]

Synthesis of ((2R,3R,4S,5R)-5-(4-amino-5-iodo-7H-pyrrolo[2,3-d]pyrimidin-7-yl)-3-((ethoxycarbonyl)oxy)-4-fluorotetrahydrofuran-2-yl) methyl ethyl carbonate

[Formula 18]

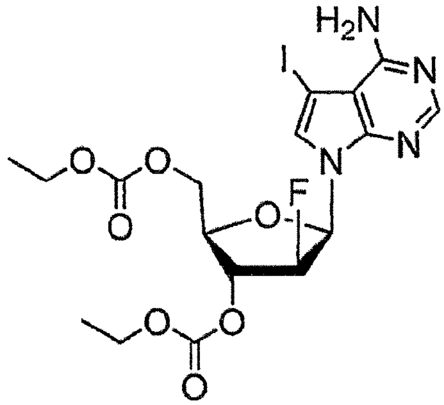

Ethyl 1H-imidazole-1-carboxylate was obtained in accordance with the process of step 2 of Example 1 except that ethanol was used in place to cyclopentanol. The title compound was synthesized in accordance with the process of step 3 of Example 1 except that (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol obtained in Comparative Example 2 was used in place of (2R,3R,4S,5R)-5-(4-amino-5-iodo-7H-pyrrolo[2,3-d]pyrimidin-7-yl)-2-(((tert-butyldimethylsilyl)oxy)methyl)-4-fluorotetrahydrofuran-3-ol; and ethyl 1H-imidazole-1-carboxylate obtained in the above step was used in place of cyclopentyl 1H-imidazole-1-carboxylate.

$^1$H-NMR (d6-DMSO) $\delta$ (ppm): 1.18-1.23 (6H, m), 4.09-4.19 (4H, m), 4.25-4.47 (3H, m), 5.31-5.56 (2H, m), 6.55 (1H, dd, J = 4.4, 18 Hz), 6.72 (2H, brs), 7.42 (1H, d, J = 2. 8 Hz), 8.10 (1H, s)

ESI-MS: m/z 539 (MH+)

[Comparative Example 5]

Synthesis of isopropyl (7-((2R,3S,4R,5R)-3-fluoro-4-hydroxy-5-(hydroxymethyl) tetrahydrofuran-2-yl)-5-iodo-7H-pyrrolo[2,3-d] pyrimidin-4-yl)carbamate

[Formula 19]

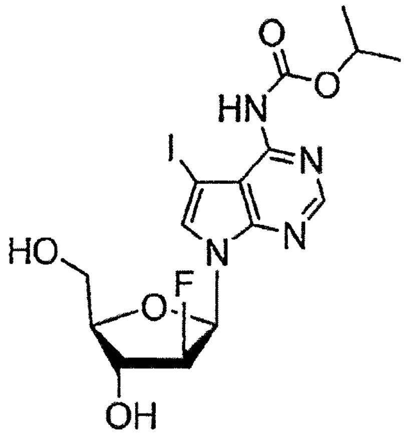

(Step 1)

(2R,3R,4S,5R)-5-(4-Amino-5-iodo-7H-pyrrolo[2,3-d]pyrimidin-7-yl)-2-(((tert-butyldimethylsilyl)oxy)methyl)-4-fluorotetrahydrofuran-3-ol (200 mg) obtained in step 1 of Example 1 was dissolved in pyridine (2 mL) and isopropyl chloroformate (0.064 mL) was added thereto. The reaction solution was stirred at room temperature for 4 hours. Subsequently isopropyl chloroformate (0.050 mL) was further added and the reaction solution was stirred at room temperature for one hour. Methanol (1 mL) was added to the reaction solution, which was stirred for 10 minutes and concentrated under reduced pressure. Ethyl acetate (5 mL) and 0.5 N hydrochloric acid were added to the solution, which was allowed to separate into layers. The organic layer was concentrated under reduced pressure and the resultant residue was purified by silica gel column chromatography (hexane/ethyl acetate 90%) to obtain isopropyl (7-((2R,3S,4R,5R)-5-(((tertbutyldimethylsilyl)oxy)methyl)-3-fluoro-4-hydroxy tetrahydrofuran-2-yl)-5-iodo-7H-pyrrolo[2,3-d]pyrimidin-4-yl)carbamate (190 mg).

(Step 2)

Isopropyl (7-((2R,3S,4R,5R)-5-(((tertbutyldimethylsilyl)oxy)methyl)-3-fluoro-4-hydroxy tetrahydrofuran-2-yl)-5-iodo-7H-pyrrolo[2,3-d]pyrimidin-4-yl)carbamate (190 mg) obtained in step 2 of this Example was dissolved in ethanol (3 mL) and 1 N hydrochloric acid (0.6 mL) was added thereto. The reaction solution was stirred at 50°C for one hour and directly purified by silica gel column chromatography (methanol/chloroform 10%) to obtain isopropyl (7-((2R,3S,4R,5R)-3-fluoro-4-hydroxy-5-(hydroxymethyl) tetrahydrofuran-2-yl)-5-iodo-7H-pyrrolo[2,3-d]pyrimidin-4-yl)carbamate (63 mg).

$^1$H-NMR (d6-DMSO) δ (ppm): 1.25-1.27 (6H, m), 3.59-3.70 (2H, m), 3.80-3.84 (1H, m), 4.35-4.43 (1H, m), 4.86-5.26 (3H, m), 5.92 (1H, d, J = 5.2 Hz), 6.65 (1H, dd, J = 4.4, 14 Hz), 7.86 (1H, d, J = 1.6 Hz), 8.60 (1H, s), 9.75 (1H, s)

ESI-MS: m/z 481 (MH+)

The compounds obtained in Examples and Comparative Examples were evaluated by the following tests.

[Test Example 1] Evaluation 1 of presence of prodrug in mouse and absorbency thereof

The dosage amounts of the compounds of Comparative Examples 1 to 5 and Examples 1 to 26 are those (mole/10 mL/kg) shown in Tables. Solutions for administration were prepared with an 0.5% aqueous solutions of HPMC so as to satisfy the dosage amounts.

Note that, the "amount of mole/10 mL/kg" means as follows: for example "10 mmol/10 mL/kg" means that 10 mmol of a test compound per body-weight (1 kg) of a mouse was dispersed or dissolved in a solution (10 mL) for administration. The same applies hereinafter.

The solutions for administration prepared above were orally administered to mice (Balb/cA, male) not fasted each by use of an oral administration sonde.

The mice were anesthetized with isoflurane 0.5, 1, 2, 4 and 6 hours after administration and blood was taken from the facial vein by use of a specific needle (animal lancet) and a heparin-coated hematocrit tube.

The blood sampled was immediately centrifuged (13000 rpm, 2 minutes, 4°C) to prepare the plasma samples. After proteins were removed from the plasma samples, the concentrations of the test compound in the plasma samples were measured by LC/MS/MS.

The value of "area under the curve" (AUC) of drug concentration in blood versus time was calculated using analysis software such as Phoenix (registered trademark) WinNonlin (registered trademark).

The AUC (AUC 0-6 hr) values obtained in the time period of 0 to 6 hours after administration are shown in Table 3 and Table 4. In Table 3, "Comparative Example 1" and "Comparative Example 2" represent the "compound (Br form) of Comparative Example 1" and the "compound (I form) of Comparative Example 2", respectively. In Table 4, "Comparative Example 6" represents the "compound (Cl form) of Comparative Example 6".

[Table 3]

| Compound | Dosage | AUC0-6hr (μM-hr) |
|---|---|---|
| Comparative Example 1 | 17 μmol/kg | 2.07 |
| Comparative Example 2 | 7.6 μmol/kg | 0.21 |
| Example 1 | 7.6 μmol/kg | 0.68 in terms of active compound of Comparative Example 2 |
| Example 2 | 7.6 μmol/kg | 0.33 in terms of active compound of Comparative Example 2 |
| Example 3 | 7.6 μmol/kg | 0.20 in terms of active compound of Comparative Example 2 |
| Example 4 | 7.6 μmol/kg | 0.48 in terms of active compound of Comparative Example 2 |
| Example 5 | 7.6 μmol/kg | 1.0 in terms of active compound of Comparative Example 2 |
| Example 6 | 7.6 μmol/kg | 0.10 in terms of active compound of Comparative Example 2 |
| Example 7 | 7.6 μmol/kg | 0.07 in terms of active compound of Comparative Example 2 |

(continued)

| Compound | Dosage | AUC0-6hr (μM-hr) |
|---|---|---|
| Example 8 | 7.6 μmol/kg | 0.15 in terms of active compound of Comparative Example 2 |
| Example 9 | 7.6 μmol/kg | 0.26 in terms of active compound of Comparative Example 2 |
| Example 10 | 7.6 μmol/kg | 0.15 in terms of active compound of Comparative Example 2 |
| Example 11 | 7.6 μmol/kg | 0.21 in terms of active compound of Comparative Example 2 |
| Example 12 | 7.6 μmol/kg | 0.31 in terms of active compound of Comparative Example 2 |
| Example 13 | 7.6 μmol/kg | 0.72 in terms of active compound of Comparative Example 2 |
| Example 14 | 7.6 μmol/kg | 0.17 in terms of active compound of Comparative Example 2 |
| Example 15 | 7.6 μmol/kg | 0.12 in terms of active compound of Comparative Example 2 |
| Example 16 | 7.6 μmol/kg | 0.49 in terms of active compound of Comparative Example 2 |
| Example 17 | 7.6 μmol/kg | 0.30 in terms of active compound of Comparative Example 2 |
| Example 18 | 7.6 μmol/kg | 0.38 in terms of active compound of Comparative Example 2 |
| Example 19 | 7.6 μmol/kg | 0.93 in terms of active compound of Comparative Example 2 |
| Example 20 | 7.6 μmol/kg | 0.88 in terms of active compound of Comparative Example 2 |
| Example 21 | 17 μmol/kg | 3.7 in terms of active compound of Comparative Example 1 |
| Example 22 | 7.6 μmol/kg | 0.81 in terms of active compound of Comparative Example 2 |
| Example 23 | 7.6 μmol/kg | 1.0 in terms of active compound of Comparative Example 2 |
| Example 24 | 7.6 μmol/kg | 0.54 in terms of active compound of Comparative Example 2 |
| Example 25 | 7.6 μmol/kg | 1.50 in terms of active compound of Comparative Example 2 |
| Example 26 | 7.6 μmol/kg | 0.37 in terms of active compound of Comparative Example 2 |
| Comparative Example 3 | 7.6 μmol/kg | 0.039 in terms of active compound of Comparative Example 2 |
| Comparative Example 4 | 7.6 μmol/kg | 0.006 in terms of active compound of Comparative Example 2 |
| Comparative Example 5 | 7.6 μmol/kg | 0.003 in terms of active compound of Comparative Example 2 |

[Table 4]

| Compound | Dosage | AUC0-6hr (μM-hr) |
|---|---|---|
| Comparative Example 6 | 9.9 μmol/kg | 2.07 |
| Example 27 | 9.9 μmol/kg | 1.7 in terms of active compound of Comparative Example 6 |

From the results shown above, it was found that the compound of Example 21 is a prodrug of the compound (Br form) of Comparative Example 1 serving as an active substance. It was also found that the compounds of Examples 1 to 20 and 22 to 26 are prodrugs of the compound (I form) of Comparative Example 2 serving as an active substance. It was further found that the compound of Example 27 is a prodrug of the compound (Cl form) of Comparative Example 6 serving as an active substance. It was further found that some of the compounds of Examples 1 to 27 show higher AUC values than the cases where the corresponding active substances shown in Comparative Examples 1 to 2 and 6 were orally administered.

[Test Example 2] <u>Evaluation 1 of growth suppression activity in human tumor cell line</u>

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown

below and the concentration (IC50 (μM)) of the compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results of the compound (Br form) of Comparative Example 1 are shown in Table 5; whereas, the results of the compound (I form) of Comparative Example 2 are shown in Table 6. "Comparative Example 1" in Table 5 and "Comparative Example 2" in Table 6 represent the "compound (Br form) of Comparative Example 1" and the "compound (I form) of Comparative Example 2", respectively.

[Table 5]

| Type | Cell line | Comparative Example 1 | Type | Cell line | Comparative Example 1 |
|---|---|---|---|---|---|
| | | IC50 (μM) | | | IC50 (μM) |
| Human bone tumor | A673 | 0.13 | Human lung cancer | H2126 | 0.11 |
| Human breast cancer | HCC1806 | 0.13 | Human lung cancer | H2170 | 0.26 |
| Human breast cancer | MCF7 | 0.08 | Human lung cancer | H226 | 0.13 |
| Human brain tumor | A172 | 0.14 | Human lung cancer | H23 | 0.05 |
| Human brain tumor | LN229 | 0.63 | Human lung cancer | H441 | 0.14 |
| Human head and neck cancer | CA9-22 | 0.08 | Human lung cancer | H460 | 0.08 |
| Human head and neck cancer | DOK | 0.11 | Human lung cancer | H526 | 0.17 |
| Human hematopoietic organ tumor | HL60 | 0.30 | Human lung cancer | H69 | 0.11 |
| Human hematopoietic organ tumor | K562 | 0.11 | Human lung cancer | Mero82 | 0.46 |
| Human hematopoietic organ tumor | MOLT4 | 0.06 | Human lung cancer | Mero83 | 0.35 |
| Human hematopoietic organ tumor | MV-4-11 | 0.03 | Human lung cancer | MES01 | 0.41 |
| Human hematopoietic organ tumor | RPMI8226 | 0.34 | Human lung cancer | SDM103T2 | 0.58 |
| Human hematopoietic organ tumor | CCRFCEM | 0.05 | Human lung cancer | Mero48a | 0.16 |
| Human hematopoietic organ tumor | BHL-89 | 0.23 | Human lung cancer | SPC111 | 0.36 |
| Human hematopoietic organ tumor | BC3 | 0.02 | Human ovary cancer | A2780 | 0.05 |
| Human hematopoietic organ tumor | BCP1 | 0.11 | Human pancreatic cancer | BXPC3 | 0.27 |
| Human renal cancer | 786-0 | 0.22 | Human pancreatic cancer | CAPAN2 | 0.05 |
| Human colorectal cancer | DLD1 | 0.05 | Human pancreatic cancer | CFPAC1 | 0.11 |

(continued)

| Type | Cell line | Comparative Example 1 | Type | Cell line | Comparative Example 1 |
|---|---|---|---|---|---|
| | | IC50 (μM) | | | IC50 (μM) |
| Human colorectal cancer | HCT116 | 0.12 | Human pancreatic cancer | MIAPACA2 | 0.07 |
| Human colorectal cancer | HT29 | 0.11 | Human prostate cancer | DUI45 | 0.09 |
| Human colorectal cancer | SW48 | 0.13 | Human skin cancer | COL0792 | 0.89 |
| Human colorectal cancer | SW620 | 0.23 | Human stomach cancer | HS746T | 0.25 |
| Human lung cancer | DMS273 | 0.40 | Human stomach cancer | MKN45 | 0.21 |
| Human lung cancer | EBC1 | 0.06 | Human stomach cancer | N87 | 0.03 |
| Human lung cancer | H1703 | 0.02 | Human stomach cancer | NUGC3 | 0.07 |
| Human lung cancer | H1975 | 0.07 | human bladder cancer | HT1197 | 0.13 |
| Human lung cancer | H2081 | 0.09 | human bladder cancer | HT1376 | 0.06 |

[Table 6]

| Type | Cell line | Comparative Example 2 |
|---|---|---|
| | | IC50 (μM) |
| Human brain tumor | A172 | 0.03 |
| Human brain tumor | LN229 | 0.02 |
| Human hematopoietic organ tumor | MOLT4 | 0.01 |
| Human hematopoietic organ tumor | MV-4-11 | 0.004 |
| Human hematopoietic organ tumor | CCRFCEM | 0.04 |
| Human colorectal cancer | DLD1 | 0.04 |
| Human colorectal cancer | HCT116 | 0.09 |
| Human colorectal cancer | HT29 | 0.13 |
| Human lung cancer | H1703 | 0.04 |
| Human lung cancer | H69 | 0.06 |
| Human pancreatic cancer | CFPAC1 | 0.05 |
| Human pancreatic cancer | MIAPACA2 | 0.04 |
| Human stomach cancer | MKN45 | 0.13 |

From the results shown above, it was found that the compounds of Comparative Example 1 and Comparative Example 2 have antitumor effects on a wide variety of tumors.

[Test Example 3] Evaluation 2 of growth suppression activity to human tumor cell line

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown below and the concentration (IC50 (nM)) of the compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results are shown in Table 7. In Table 7, "Comparative Example 1" and "Comparative Example 2" represent the "compound (Br form) of Comparative Example 1" and the "compound (I form) of Comparative Example 2", respectively. In Table 8, "Comparative Example 6" represent the "compound (Cl form) of Comparative Example 6" .

[Table 7]

| Type | Cell line | Comparative Example 1 | Type | Cell line | Comparative Example 2 |
|---|---|---|---|---|---|
| | | IC50 (nM) | | | IC50 (nM) |
| Human lung cancer | A549 | 24 | Human lung cancer | A549 | 14 |
| Human lung cancer | H1650 | 11 | Human lung cancer | H1650 | 8 |
| Human lung cancer | H2122 | 17 | Human lung cancer | H2122 | 9 |
| Human lung cancer | H358 | 69 | Human lung cancer | H358 | 52 |
| Human lung cancer | HCC827 | 228 | Human lung cancer | HCC827 | 176 |
| Human brain tumor | A431 | 32 | Human brain tumor | A431 | 18 |
| Human colorectal cancer | RKO | 31 | Human breast cancer | SK-BR-3 | 23 |
| Human breast cancer | SK-BR-3 | 26 | | | |

[Table 8]

| Type | Cell line | Comparative Example 6 IC50 (nM) |
|---|---|---|
| Human lung cancer | A549 | 54 |
| Human lung cancer | H1650 | 31 |
| Human lung cancer | H2122 | 58 |
| Human lung cancer | H358 | 273 |
| Human lung cancer | HCC827 | 795 |
| Human brain tumor | A431 | 124 |
| Human colorectal cancer | RKO | 135 |

(continued)

| Type | Cell line | Comparative Example 6 IC50 (nM) |
|---|---|---|
| Human breast cancer | SK-BR-3 | 88 |

From the results shown above, it was found that the compounds of Comparative Examples 1, 2 and 6 have antitumor effects on a wide variety of tumors.

[Test Example 4] Evaluation 3 of growth suppression activity to human tumor cell line

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown below and the concentration (IC50 (nM)) of a compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results are shown in Table 9. In Table 9, "Comparative Example 2" and "Comparative Example 7" represent the "compound (I form) of Comparative Example 2" and the "compound (difluoro form) of Comparative Example 7", respectively.

[Table 9]

| Type | Cell line | Comparative Example 2 IC50 (nM) | Comparative Example 7 IC50 (nM) |
|---|---|---|---|
| Human lung cancer | A427 | 98 | 1136 |
| Human lung cancer | H520 | 29 | 465 |

From the results shown above, it was found that IC50 of Comparative Example 2-compound is more than 10 times lower than that of Comparative Example 7-compound and has an excellent antitumor effect.

[Test Example 5] Evaluation 1 of antitumor effect and toxicity in mouse

A human brain tumor cell line (U-87 MG) was subcutaneously transplanted in the right chest of each of BALB/cA Jcl-nu/nu mice. After tumor transplantation, the major axes (mm) and minor axes (mm) of the tumors were measured and tumor volumes (TV) were calculated. The mice were assigned to individual groups such that the average TVs of the groups became equal. The day of grouping mice was determined Day 0.

Comparative Example 2-compound (I form) was orally administered in a dose of 12 mg/kg/day every day from Day 1, whereas, Example 1-compound was orally administered in a dose of 15.4 mg/kg/day, every day. Thereafter, administration was continued as long as a weight loss was acceptable and then evaluated. Referring to the following literature, administration followed by evaluation/observation was continued until a weight loss became 20% or more.

LABIO 21, No. 30, OCT. 2007, P27

The tumor volumes (TV) and body weights (BW) of individual groups were evaluated. As the indexes of antitumor effect and toxicity, a tumor-growth inhibition rate (IR) based on relative tumor volume (RTV) to that of Day 0 and a body weight change (BWC) relative to that of Day 0 were obtained, respectively, in accordance with the following equations. IRs and BWCs of a control group (no treatment), Example 1-compound administration group and Comparative Example 2-compound administration group were compared.

$$\text{TV (mm}^3) = (\text{major axis} \times \text{minor axis}^2)/2$$

$$RTV = (TV \text{ on evaluation date})/(TV \text{ on Day } 0)$$

$$IR (\%) = (1 - (RTV \text{ of administration group on evaluation date})/(RTV \text{ of control group on evaluation date})) \times 100$$

$$BWC (\%) = (BW \text{ on evaluation date} - BW \text{ on Day } 0)/(BW \text{ on Day } 0) \times 100$$

IRs of Comparative Example 2-compound administration group and Example 1-compound administration group on the final day of evaluation (Day 6 in Comparative Example 2 and Day 13 in Example 1) are shown in Figure 1 and BWC versus elapsed days is shown in Figure 2.

As a result of the above evaluations, it was observed that administration is continued longer in Example 1-compound administration group compared to Comparative Example 2-compound administration group, in other words, a high antitumor effect was observed while the effect on body weight was mild. From the observation, it was found that the compound of Example 1 has a high antitumor effect and is excellent in view of safety.

A compound of the present invention, typically the compound of Example 1, has a high antitumor effect and is excellent in view of safety.

[Test Example 6] Evaluation 2 of antitumor effect and toxicity in mouse

A human brain tumor cell line (U-87 MG) was subcutaneously transplanted in the right chest of each of BALB/cA Jcl-nu/nu mice. After tumor transplantation, the major axes (mm) and minor axes (mm) of the tumors were measured and tumor volumes (TV) were calculated. The mice were assigned to individual groups such that the average TVs of the groups became equal. The day of grouping mice was determined Day 0.

Comparative Example 2-compound (I form) was orally administered in a dose of 12 mg/kg/day every day from Day 1, whereas, Example 5-compound was orally administered in a dose of 14.6 mg/kg/day, every day.

The tumor volumes (TV) and body weights (BW) of individual groups were evaluated. As the indexes of antitumor effect and toxicity, a tumor-growth inhibition rate (IR) based on relative tumor volume (RTV) to that of Day 0 and a body weight change (BWC) relative to that of Day 0 were obtained, respectively, in accordance with the following equations. In order to evaluate "effect versus toxicity", a ratio based on IR and BWC (effect index) was obtained, and Example 5-compound administration group was compared to Comparative Example 2-compound administration group.

$$TV (mm^3) = (\text{major axis} \times \text{minor axis}^2)/2$$

$$RTV = (TV \text{ on evaluation date})/(TV \text{ on Day } 0)$$

$$IR (\%) = (1 - (RTV \text{ of administration group on evaluation date})/(RTV \text{ of Control group on evaluation date})) \times 100$$

$$BWC (\%) = (BW \text{ on evaluation date} - BW \text{ on Day } 0)/(BW \text{ on Day } 0) \times 100$$

$$\text{Effect index} = IR(\%)/(-BWC(\%))$$

Effect indexes of Comparative Example 2-Compound (I form) and Example 5-compound administration groups on Day 6 are shown in Figure 3.

As a result of the above evaluations, it was observed that "effect versus toxicity" of Example 5-compound administration group is high compared to that of Comparative Example 2-compound administration group.

From the observation, it was found that the compound of Example 5 has an antitumor effect and safety in a well-balanced way.

A compound of the present invention, typically the compound of Example 5, has a high antitumor effect and safety in a well-balanced way.

[Test Example 7] Evaluation 3 of antitumor effect and toxicity in mouse

A human hematopoietic organ tumor cell line (MV-4-11) was subcutaneously transplanted in the right chest of each of BALB/cA Jcl-nu/nu mice. After tumor transplantation, the major axes (mm) and minor axes (mm) of the tumors were measured and tumor volumes (TV) were calculated. The mice were assigned to individual groups such that the average TVs of the groups became equal.
The day of grouping mice was determined Day 0.

Comparative Example 2-compound (I form) was orally administered in a dose of 12 mg/kg/day every day from Day 1, whereas, Example 1-compound was orally administered in a dose of 10.8 mg/kg/day, every day. Thereafter, administration was continued as long as a weight loss was acceptable and then evaluated. Referring to the following literature, administration followed by evaluation/observation was continued until a weight loss became 20% or more.
LABIO 21, No. 30, OCT. 2007, P27

The tumor volumes (TV) and body weights (BW) of individual groups were evaluated. As the indexes of antitumor effect and toxicity, the relative tumor volume (RTV) to that of Day 0 and a body weight change (BWC) were obtained, respectively, in accordance with the following equations and plotted on a chart. RTV and BWC versus elapsed days were compared among a control group (no treatment), Example 1-compound administration group and Comparative Example 2-compound administration group.

$$\text{TV (mm}^3\text{)} = (\text{major axis} \times \text{minor axis}^2)/2$$

$$\text{RTV} = (\text{TV on evaluation date})/(\text{TV on Day 0})$$

$$\text{BWC (\%)} = (\text{BW on evaluation date} - \text{BW on Day 0})/(\text{BW on Day 0}) \times 100$$

Figure 4 and Figure 5 show RTV and BWC versus elapsed days, respectively, when Comparative Example 2-compound (I form) was administered. Figure 6 and Figure 7 shows RTV and BWC versus elapsed days when Example 1-compound was administered.

On the final day of evaluation, the blood cell components (lymphocytes: LYMPH (Lymphocytes)) of individual groups were analyzed. As another index of toxicity, the rate (T/C %) of blood cell component each of an Example 1-compound administration group and a Comparative Example 2-compound administration group relative to that of the control group was obtained in accordance with the following equation. The results are shown in Figure 8.

$$\text{T/C (\%)} = (\text{Number of blood cell component in administration group}/(\text{Number of blood cell component in control group}) \times 100$$

As a result of the above evaluations, it was observed that administration can be continued longer in the Example 1-compound administration group than the Comparative Example 2-compound administration group, in other words, a remarkable antitumor effect (tumor regression was confirmed) was observed while an effect on body weight and hematological toxicity were low.

From the above results, it was demonstrated that the compound of Example 1 has a high antitumor effect and

is excellent in view of safety.

A compound of the present invention, typically the compound of Example 1, has a high antitumor effect and excellent in view of safety.

[Test Example 8] Evaluation of combination use of compound of the invention and radiation

A human brain tumor cell line (U-87 MG) was suspended in a medium, seeded in individual wells of a multi-well plate and cultured. In the case where radiation exposure and addition of a compound are simultaneously carried out, both treatments were carried out on two days after initiation of culture. In the case where addition of a compound preceded radiation exposure, a serially diluted solution of a compound was added on the day after initiation of culture and radiation exposure was carried out on two days after initiation of culture. In the case where radiation exposure preceded addition of a compound, radiation exposure was carried out on the day after initiation of culture and a serially diluted solution of a compound was added on the two days after initiation of culture. Day 5 after seeding, cells were fixed with a 25% aqueous solution of glutaraldehyde and stained with a staining fluid (0.05 w/v% crystal violet in 20% methanol). After an extract (0.1 N $NaH_2PO_4$: 100% ethanol = 1:1) was added and stirred, an absorbance at 540 nm was measured to obtain a cell count. The cell viability was calculated in accordance with the following equation and statistically processed in accordance with a Student's t-test.

$$\text{Cell viability (\%)} = (T/C) \times 100$$

T: Absorbance of a well exposed to radiation or containing a compound
C: Absorbance of a well neither exposed to radiation nor containing a compound

The results are shown in Figure 9.

As a result of the above evaluations, it was observed that even if treatments were carried out in any order (the same-day treatment, compound first and radiation first), cell viability significantly decreased in a combination treatment with the Comparative Example 2-compound and radiation exposure compared to the single treatments.

From the above results, it was demonstrated that the Comparative Example 2-compound produces a combinational antitumor effect with radiation exposure.

[Test Example 9] Evaluation of combinational effect of the compound and temozolomide (TMZ)

A human brain tumor cell line (U-87 MG) was suspended in a medium, seeded in individual wells of a multi-well plate and cultured. In the case where two compounds are simultaneously added, both compounds were added on two days after initiation of culture. In the case where addition of the compound of the present invention preceded, a serially diluted solution of the compound was added on the day after initiation of culture and a serially diluted solution of TMZ was added two days after initiation of culture. In the case where addition of TMZ preceded, a serially diluted solution of TMZ was added on the day after initiation of culture and a serially diluted solution of the compound was added on two days after initiation of culture. Day 5 after seeding, cells were fixed with a 25% aqueous solution of glutaraldehyde and stained with a staining fluid (0.05 w/v% crystal violet in 20% methanol). After an extract (0.1 N $NaH_2PO_4$: 100% ethanol = 1:1) was added and stirred, an absorbance at 540 nm was measured to obtain a cell count. The cell viability was calculated in accordance with the following equation and statistically processed in accordance with a Student's t-test.

$$\text{Cell viability (\%)} = (T/C) \times 100$$

T: Absorbance of a well containing TMZ or a compound
C: Absorbance of a well containing neither TMZ nor a compound

The results are shown in Figure 10.

As a result of the above evaluations, even if treatments were carried out in any order (the same-day treatment, compound first and TMZ first), a significant decrease in cell viability was observed in a combination treatment with the Comparative Example 2-compound and TMZ compared to the single treatments.

From the above, it was demonstrated that combination use of the Comparative Example 2-compound and TMZ exerts a combinational antitumor effect.

As described in the foregoing, the compound of the present invention is useful as an antitumor agent showing excellent safety and high antitumor effect. The compound of the present invention exerts an excellent combinational

effect when it is used in combination with an alkylating agent and/or radiation therapy.

## Claims

1. A compound represented by the following general formula (1):

[Formula 1]

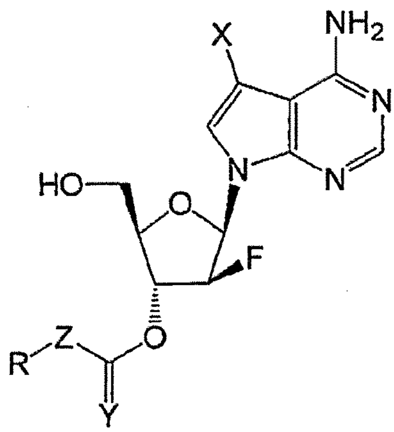

(1)

wherein

X represents a chlorine atom, a bromine atom or an iodine atom,
Y represents an oxygen atom or a sulfur atom,
Z represents an oxygen atom or a sulfur atom, and
R represents a linear C1-C6 alkyl group that may have a substituent, a C2-C6 alkenyl group that may have a substituent, a C2-C6 alkynyl group that may have a substituent, a C3-C10 cycloalkyl group that may have a substituent, a C4-C10 cycloalkenyl group that may have a substituent, a C6-C10 aromatic hydrocarbon group that may have a substituent, a 4 to 10-membered saturated heterocyclic group that may have a substituent or a 5 to 10-membered unsaturated heterocyclic group that may have a substituent,

or a pharmaceutically acceptable salt thereof.

2. The compound or a pharmaceutically acceptable salt thereof according to claim 1, wherein

R represents a linear C1-C6 alkyl group that may have 1 to 3 substituents which are selected from C1-C6 alkyl groups, C1-C6 alkenyl groups, C1-C6 alkynyl groups, C1-C4 alkoxy groups, C1-C4 haloalkyl groups, hydroxy groups, halogen atoms or aromatic hydrocarbon groups;
a C3-C10 cycloalkyl group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic hydrocarbon groups;
a C4-C10 cycloalkenyl group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic hydrocarbon groups; or
a 4 to 10-membered saturated heterocyclic group that may have 1 to 3 substituents which are selected from C1-C6 alkyl groups, C1-C6 alkenyl groups, C1-C6 alkynyl groups, C1-C4 alkoxy groups, hydroxy groups, halogen atoms or aromatic hydrocarbon groups.

3. The compound or a pharmaceutically acceptable salt thereof according to claim 2, wherein

R represents a linear C1-C6 alkyl group that may have 1 to 3 substituents which are selected from C1-C6 alkyl groups, C1-C4 alkoxy groups, C1-C4 haloalkyl groups, halogen atoms or phenyl groups, as substituents; a C3-C10 cycloalkyl group that may have 1 to 3 C1-C6 alkyl groups, C1-C4 alkoxy groups, halogen atoms or phenyl

groups;
a C4-C10 cycloalkenyl group that may have 1 to 3 substituents which are selected from C1-C6 alkyl groups, C1-C4 alkoxy groups, halogen atoms or phenyl groups; or
4 to 10-membered saturated heterocyclic group that may have 1 to 3 substituents which are selected from C1-C6 alkyl groups, C1-C4 alkoxy groups, halogen atoms or phenyl groups.

**4.** The compound or a pharmaceutically acceptable salt thereof according to claim 3, wherein X represents a bromine atom or an iodine atom.

**5.** The compound or a pharmaceutically acceptable salt thereof according to claim 4, wherein X represents an iodine atom.

**6.** The compound or a pharmaceutically acceptable salt thereof according to claim 5, wherein Y represents an oxygen atom.

**7.** The compound or a pharmaceutically acceptable salt thereof according to claim 6, wherein

R represents a linear C1-C3 alkyl group that may have 1 to 3 substituents which are selected from C1-C2 alkyl groups, C1-C2 alkoxy groups, C1-C2 haloalkyl groups, fluorine atoms or chlorine atoms;
a C3-C7 cycloalkyl group that may have 1 to 2 substituents which are selected from C1-C2 alkyl groups, C1-C2 alkoxy groups, fluorine atoms or chlorine atoms;
a C4-C6 cycloalkenyl group that may have 1 to 2 substituents which are selected from C1-C2 alkyl groups, C1-C2 alkoxy groups, fluorine atoms or chlorine atoms; or
a 4 to 6-membered saturated heterocyclic group that may have 1 to 3 substituents which are selected from C1-C2 alkyl groups, C1-C2 alkoxy groups, fluorine atoms or chlorine atoms.

**8.** The compound or a pharmaceutically acceptable salt thereof according to any one of claims 1 to 7, wherein the compound is selected from the group consisting of the following compounds:

O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-cyclopentyl carbonothioate;
O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-isopropyl carbonothioate;
O-((2R,3R,4S,5R)-5-(4-amino-5-iodo-7H-pyrrolo[2,3-d]pyrimidin-7-yl)-4-fluoro-2-(hydroxymethyl)tetrahydrofuran-3-yl) S-ethyl carbonothioate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl pentan-3-yl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate;
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopent-3-en-1-yl carbonate; and
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl (bicyclo[2.2.1]heptan-2-yl) carbonate.

**9.** The compound or a pharmaceutically acceptable salt thereof according to any one of claims 1 to 8, wherein the compound is selected from the group consisting of the following compounds:

(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl cyclopentyl carbonate; and
(2R,3R,4S,5R)-5-(4-amino-5-iodo-7H-pyrrolo[2,3-d]pyrimidin-7-yl)-4-fluoro-2-(hydroxymethyl)tetrahydrofuran-3-yl isopropyl carbonate.

**10.** An antitumor agent comprising the compound or a pharmaceutically acceptable salt thereof according to any one of claims 1 to 9, as an active ingredient.

**11.** A pharmaceutical composition comprising the compound or a pharmaceutically acceptable salt thereof according

to any one of claims 1 to 9 and a pharmaceutically acceptable carrier.

**12.** The antitumor agent according to claim 10 or the pharmaceutical composition according to claim 11, formulated as an oral preparation or an injection.

**13.** A method for preventing and/or treating a tumor, comprising administering the compound or a pharmaceutically acceptable salt thereof according to any one of claims 1 to 9 to a subject in need thereof.

**14.** A method for preventing and/or treating a tumor, comprising administering the compound or a pharmaceutically acceptable salt thereof according to any one of claims 1 to 9, formulated as an oral preparation or an injection, to a subject in need thereof.

**15.** The compound or a pharmaceutically acceptable salt thereof according to any one of claims 1 to 9 for use as a pharmaceutical composition.

**16.** The compound or a pharmaceutically acceptable salt thereof according to any one of claims 1 to 9 for use in prevention and/or treatment of a tumor.

**17.** The compound or a pharmaceutically acceptable salt thereof according to any one of claims 1 to 9, formulated as an oral preparation or an injection, for use in prevention and/or treatment of a tumor.

**18.** Use of the compound or a pharmaceutically acceptable salt thereof according to any one of claims 1 to 9, for producing an antitumor agent.

**19.** Use of the compound or a pharmaceutically acceptable salt thereof according to any one of claims 1 to 9, for producing a pharmaceutical composition.

**20.** Use of the compound or a pharmaceutically acceptable salt thereof according to any one of claims 1 to 9, for producing an antitumor agent, formulated as an oral preparation or an injection.

**21.** The antitumor agent according to claim 10, the method according to claim 13 or 14, the compound or a pharmaceutically acceptable salt thereof according to any one of claims 15 to 17, or the use according to any one of claims 18 to 20, wherein the tumor is selected from the group consisting of head and neck cancer (e.g. oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

**22.** The antitumor agent, method, use, compound or a pharmaceutically acceptable salt thereof according to claim 21, wherein the tumor is a brain tumor.

**23.** A compound represented by the following general formula (2)

[Formula 2]

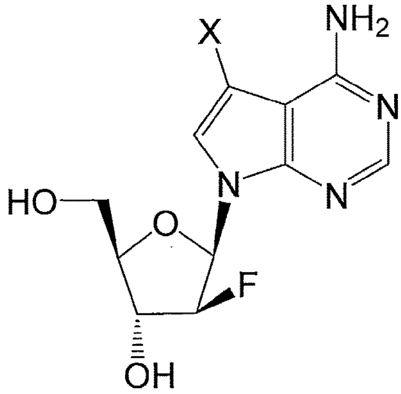

(2)

wherein
X represents a chlorine atom, a bromine atom or an iodine atom,
or a pharmaceutically acceptable salt thereof.

**24.** An antitumor agent or pharmaceutical composition comprising the compound or a pharmaceutically acceptable salt thereof according to claim 23, as active ingredients, and a pharmaceutically acceptable carrier.

**25.** The antitumor agent or pharmaceutical composition according to claim 24, formulated as an oral preparation or an injection.

**26.** A method for preventing and/or treating a tumor, comprising administering the compound or a pharmaceutically acceptable salt thereof according to claim 23.

**27.** The compound or a pharmaceutically acceptable salt thereof according to claim 23, for preventing and/or treating a tumor.

**28.** Use of the compound or a pharmaceutically acceptable salt thereof according to claim 23, for producing a pharmaceutical composition or an antitumor agent.

**29.** The antitumor agent or pharmaceutical composition according to claim 24 or 25, the method for preventing and/or treating a tumor according to claim 26, the compound or a pharmaceutically acceptable salt thereof according to claim 27 or the use according to claim 28, wherein the tumor is selected from the group consisting of head and neck cancer (e.g., oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

**30.** The antitumor agent or pharmaceutical composition, method for preventing and/or treating a tumor, compound or a pharmaceutically acceptable salt thereof or use according to claim 29, wherein the tumor is a brain tumor.

**31.** The antitumor agent or pharmaceutical composition consisting of the compound or a pharmaceutically acceptable salt thereof according to any one of claims 1 to 9 and 23, which is used in combination with an alkylating agent.

**32.** An enhancer of an antitumor effect comprising the compound or a pharmaceutically acceptable salt thereof according to any one of claims 1 to 9 and 23, for enhancing an antitumor effect of an alkylating agent.

**33.** An antitumor agent comprising the compound or a pharmaceutically acceptable salt thereof according to any one

of claims 1 to 9 and 23, for treating a cancer patient having received administration of an alkylating agent.

**34.** A method for preventing and/or treating a tumor, comprising administering the compound or a pharmaceutically acceptable salt thereof according to any one of claims 1 to 9 and 23, which is used in combination with an alkylating agent.

**35.** A method for enhancing an antitumor effect of an alkylating agent, comprising administering the compound or a pharmaceutically acceptable salt thereof according to any one of claims 1 to 9 and 23 to a patient.

**36.** A method for preventing and/or treating a tumor, comprising administering an antitumor agent consisting of the compound or a pharmaceutically acceptable salt thereof according to any one of claims 1 to 9 and 23, and an alkylating agent.

**37.** Use of the compound or a pharmaceutically acceptable salt thereof according to any one of claims 1 to 9 and 23, for treating a tumor, which is administered in combination with an alkylating agent.

**38.** Use of the compound or a pharmaceutically acceptable salt thereof according to any one of claims 1 to 9 and 23, for enhancing an antitumor effect of an alkylating agent, which is administered in combination with an alkylating agent.

**39.** Use of the compound or a pharmaceutically acceptable salt thereof according to any one of claims 1 to 9 and 23, for treating a tumor, which treats a cancer patient having received administration of an alkylating agent.

**40.** Use of the compound or a pharmaceutically acceptable salt thereof according to any one of claims 1 to 9 and 23, for enhancing an antitumor effect of an alkylating agent, which treats a cancer patient having received administration of an alkylating agent.

**41.** Use of the compound or a pharmaceutically acceptable salt thereof according to any one of claims 1 to 9 and 23, for producing an antitumor agent, wherein the antitumor agent is administered in combination with an alkylating agent.

**42.** Use of the compound or a pharmaceutically acceptable salt thereof according to any one of claims 1 to 9 and 23, for producing an enhancer of an antitumor effect of an alkylating agent, wherein the enhancer of an antitumor effect is administered in combination with an alkylating agent.

**43.** Use of the compound or a pharmaceutically acceptable salt thereof according to any one of claims 1 to 9 and 23, for producing an antitumor agent, wherein the antitumor agent is administered to a cancer patient having received administration of an alkylating agent or a cancer patient to receive administration of an alkylating agent.

**44.** Use of the compound or a pharmaceutically acceptable salt thereof according to any one of claims 1 to 9 and 23, for producing an enhancer of an antitumor effect of an alkylating agent, wherein the enhancer of an antitumor effect is administered to a cancer patient having received administration of an alkylating agent or a cancer patient to receive administration of an alkylating agent.

**45.** The antitumor agent according to claim 31 or 33, the enhancer of an antitumor effect according to claim 32, the method according to any one of claims 34 to 36, or the use according to any one of claims 37 to 44, wherein the alkylating agent is temozolomide.

**46.** The antitumor agent according to claim 31 or 33, the enhancer of an antitumor effect according to claim 32, the method according to any one of claims 34 to 36, or the use according to any one of claims 37 to 44, which is used in combination with a radiation therapy in addition to the alkylating agent.

**47.** An antitumor agent or a pharmaceutical composition consisting of the compound or a pharmaceutically acceptable salt thereof according to any one of claims 1 to 9 and 23, which is used in combination with a radiation therapy.

**48.** An enhancer of an antitumor effect comprising the compound or a pharmaceutically acceptable salt thereof according to any one of claims 1 to 9 and 23, for enhancing an antitumor effect of a radiation therapy.

**49.** An antitumor agent comprising the compound or a pharmaceutically acceptable salt thereof according to any one of claims 1 to 9 and 23, for treating a cancer patient having received a radiation therapy.

**50.** A method for preventing and/or treating a tumor, comprising the compound or a pharmaceutically acceptable salt thereof according to any one of claims 1 to 9 and 23, which is used in combination with a radiation therapy.

**51.** A method for enhancing an antitumor effect of a radiation therapy, comprising administering the compound or a pharmaceutically acceptable salt thereof according to any one of claims 1 to 9 and 23 to a patient.

**52.** A method for preventing and/or treating a tumor, comprising administering an antitumor agent consisting of the compound or a pharmaceutically acceptable salt thereof according to any one of claims 1 to 9 and 23 to a cancer patient having received a radiation therapy.

**53.** Use of the compound or a pharmaceutically acceptable salt thereof according to any one of claims 1 to 9 and 23, for treating a tumor, which is used in combination with a radiation therapy.

**54.** Use of the compound or a pharmaceutically acceptable salt thereof according to any one of claims 1 to 9 and 23, for enhancing an antitumor effect, which is used in combination with a radiation therapy.

**55.** Use of the compound or a pharmaceutically acceptable salt thereof according to any one of claims 1 to 9 and 23, for treating a tumor, which treats a cancer patient having received a radiation therapy.

**56.** Use of the compound or a pharmaceutically acceptable salt thereof according to any one of claims 1 to 9 and 23, for enhancing an antitumor effect, which treats a cancer patient having received a radiation therapy.

**57.** Use of the compound or a pharmaceutically acceptable salt thereof according to any one of claims 1 to 9 and 23, for producing an antitumor agent, wherein the antitumor agent is used in combination with a radiation therapy.

**58.** Use of the compound or a pharmaceutically acceptable salt thereof according to any one of claims 1 to 9 and 23, for producing an enhancer of an antitumor effect, wherein the enhancer of an antitumor effect is used in combination with a radiation therapy.

**59.** Use of the compound or a pharmaceutically acceptable salt thereof according to any one of claims 1 to 9 and 23, for producing an antitumor agent, wherein the antitumor agent is administered to a cancer patient having received a radiation therapy.

**60.** Use of the compound or a pharmaceutically acceptable salt thereof according to any one of claims 1 to 9 and 23, for producing an enhancer of an antitumor effect, wherein the enhancer of an antitumor effect is administered to a cancer patient having received a radiation therapy.

**61.** The antitumor agent according to claim 47 or 49, the enhancer of an antitumor effect according to claim 48, the method according to any one of claims 50 to 52 or the use according to any one of claims 53 to 60, which is used in combination with an alkylating agent in addition to the radiation therapy.

**62.** The antitumor agent, enhancer of an antitumor effect, method or use according to claim 61, wherein the alkylating agent is temozolomide.

Fig. 1

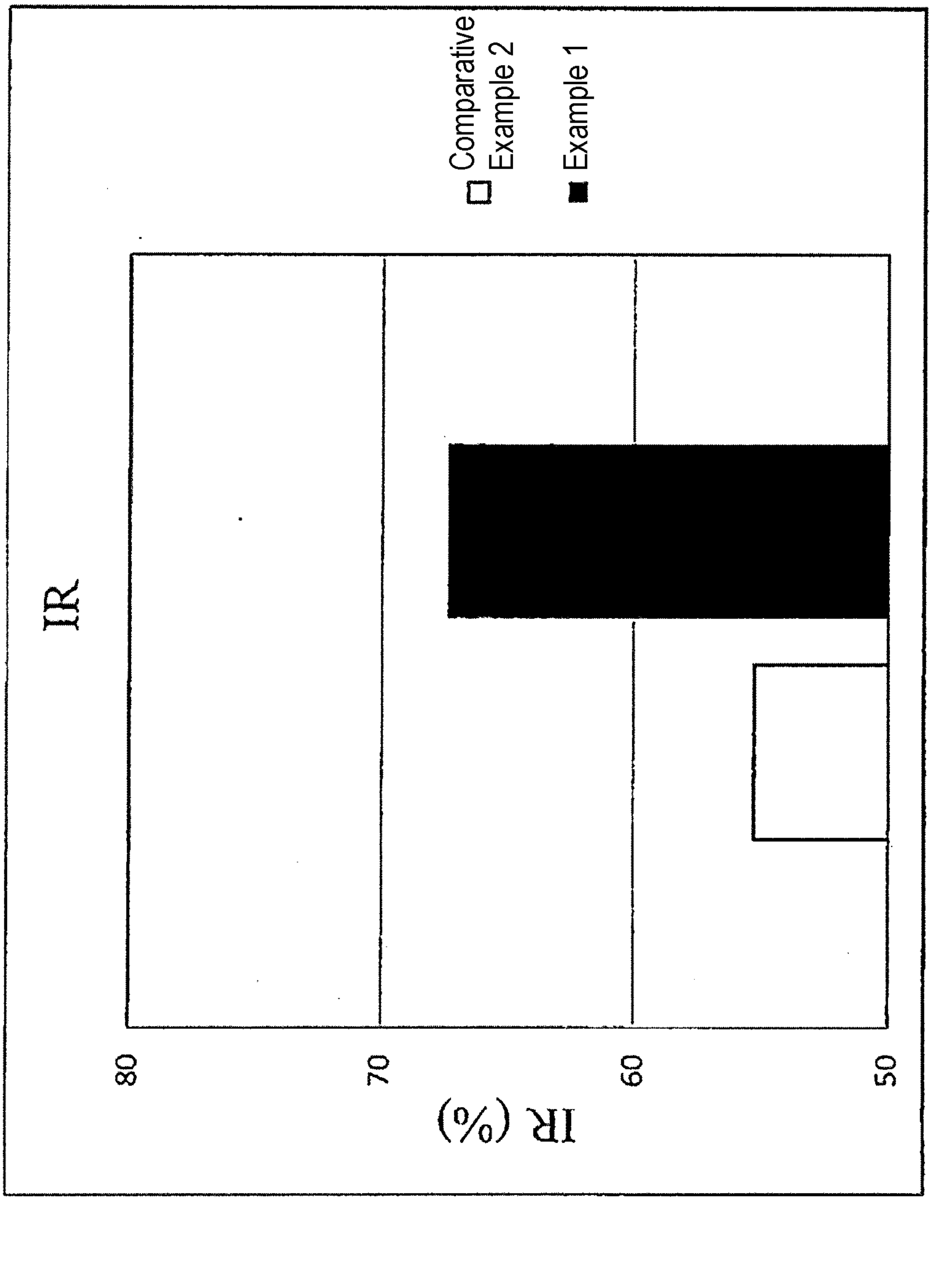

Fig. 2

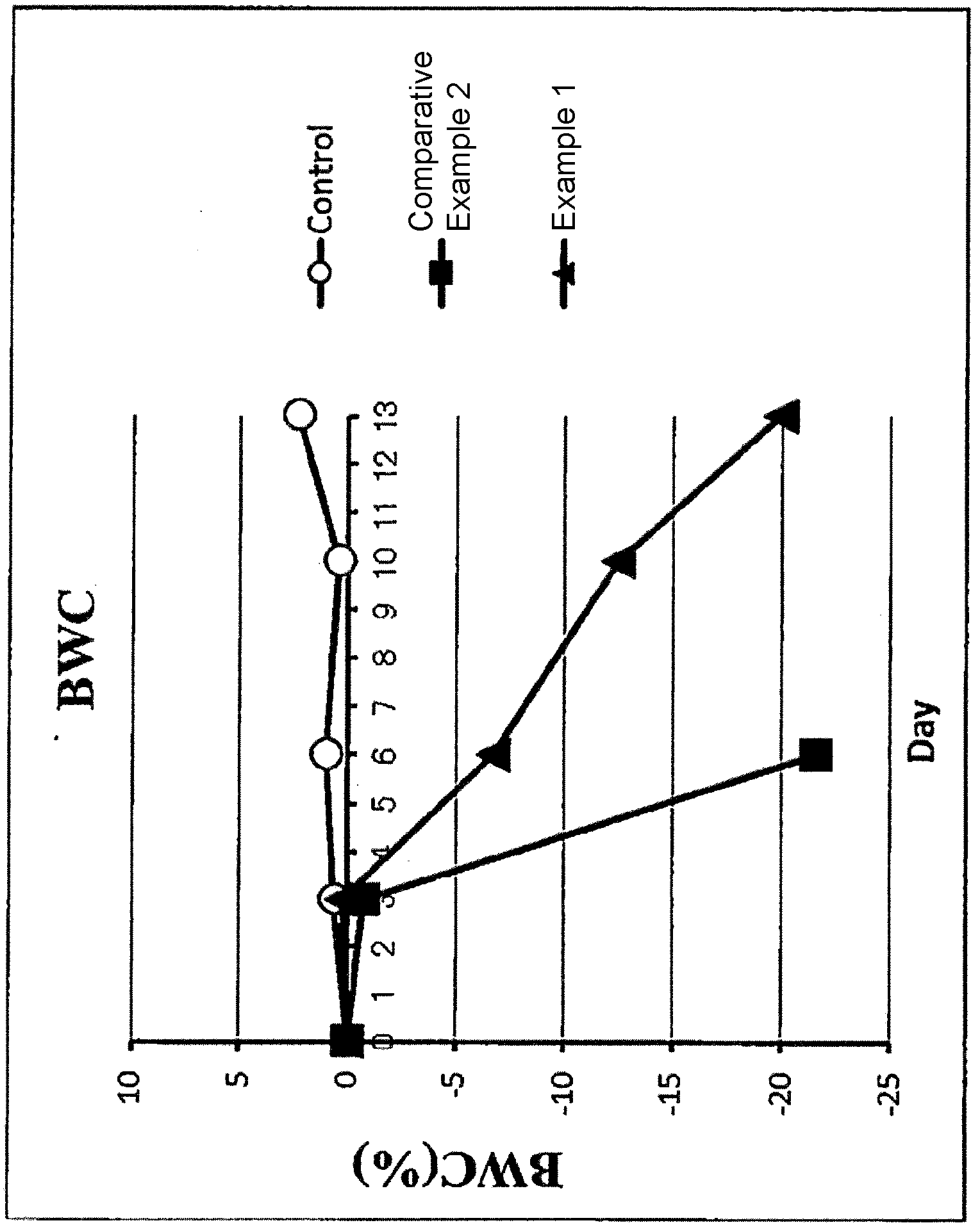
BWC
BWC(%)
10
5
0
-5
-10
-15
-20
-25
0 1 2 3 4 5 6 7 8 9 10 11 12 13
Day
Control
Comparative Example 2
Example 1

Fig. 3

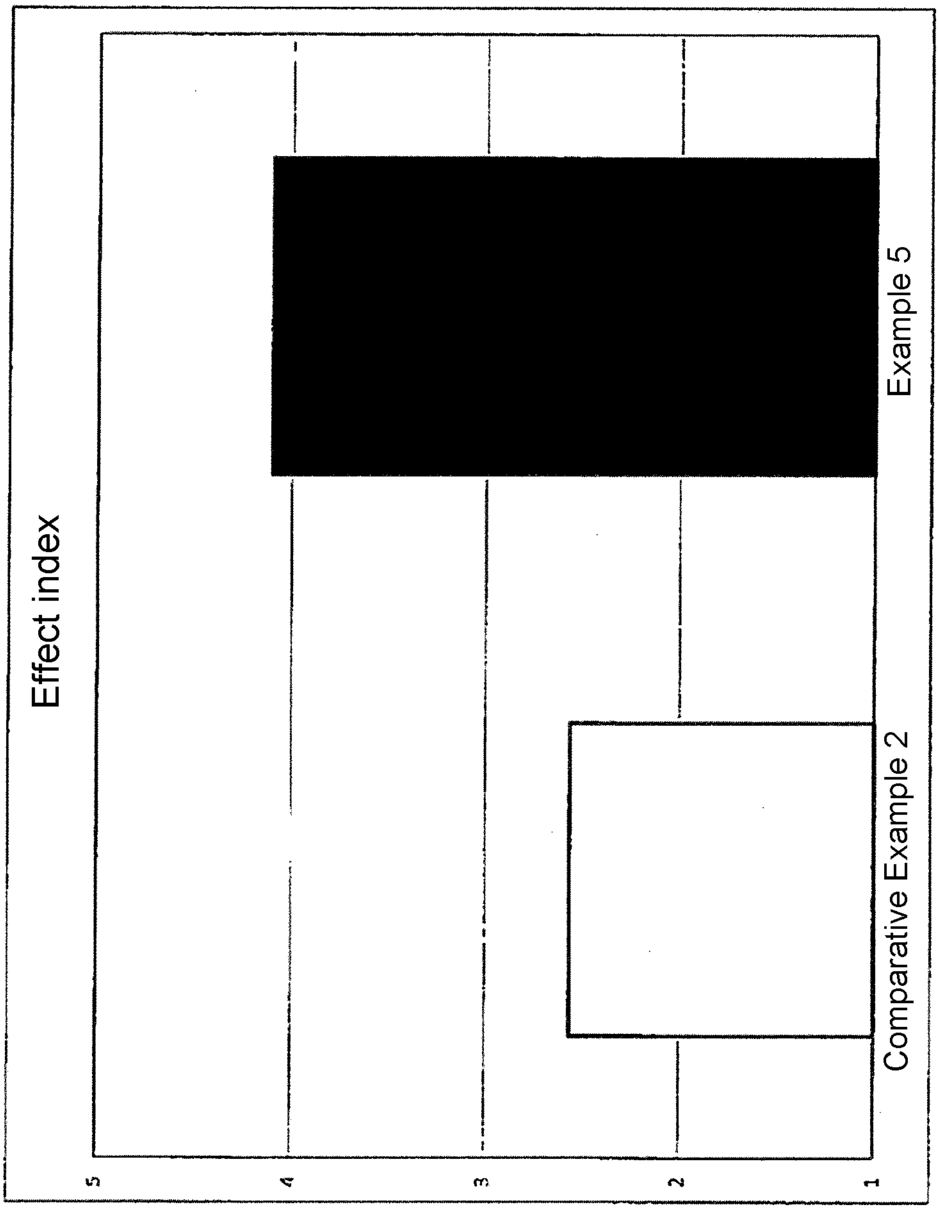

Fig. 4

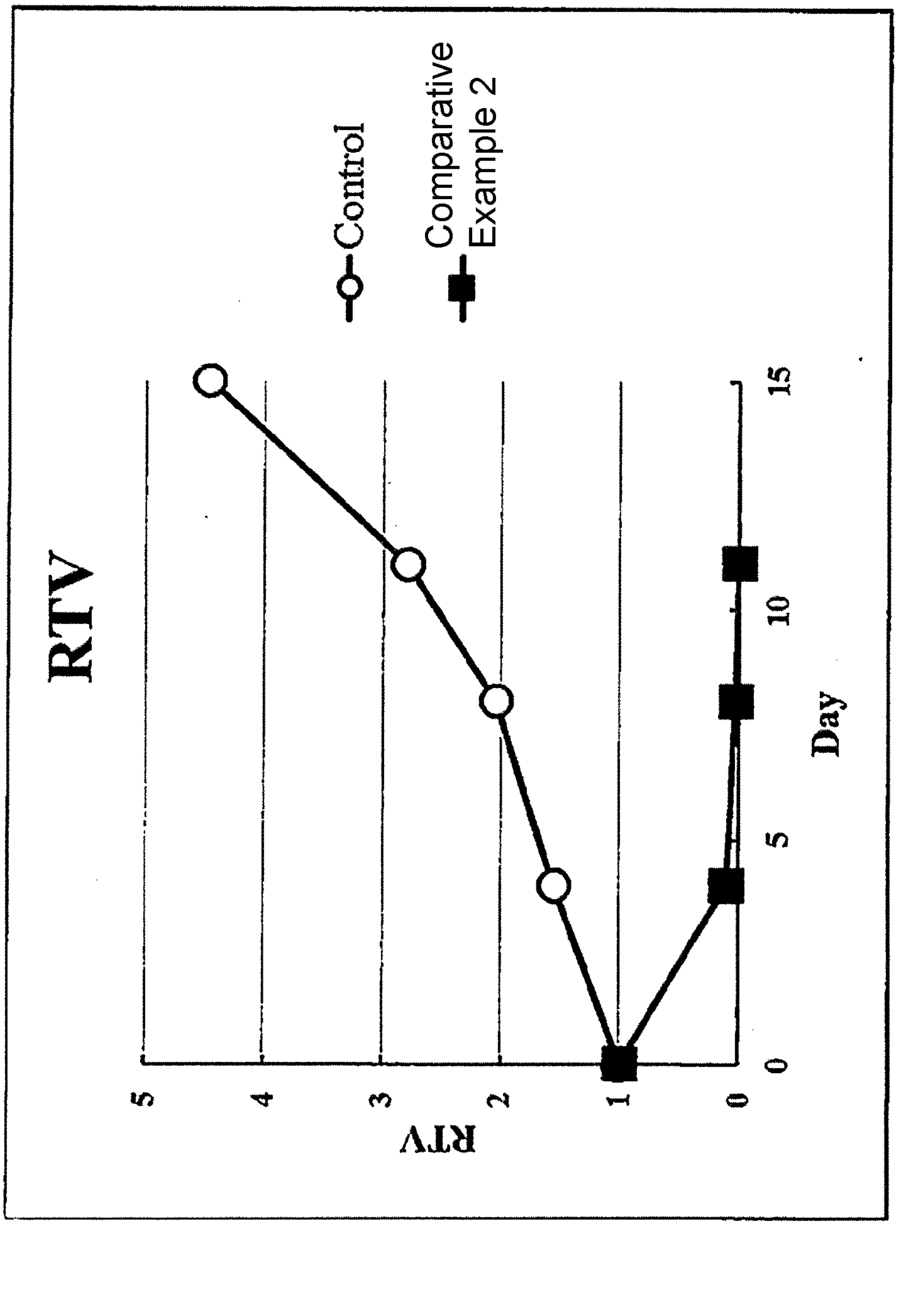

Fig. 5

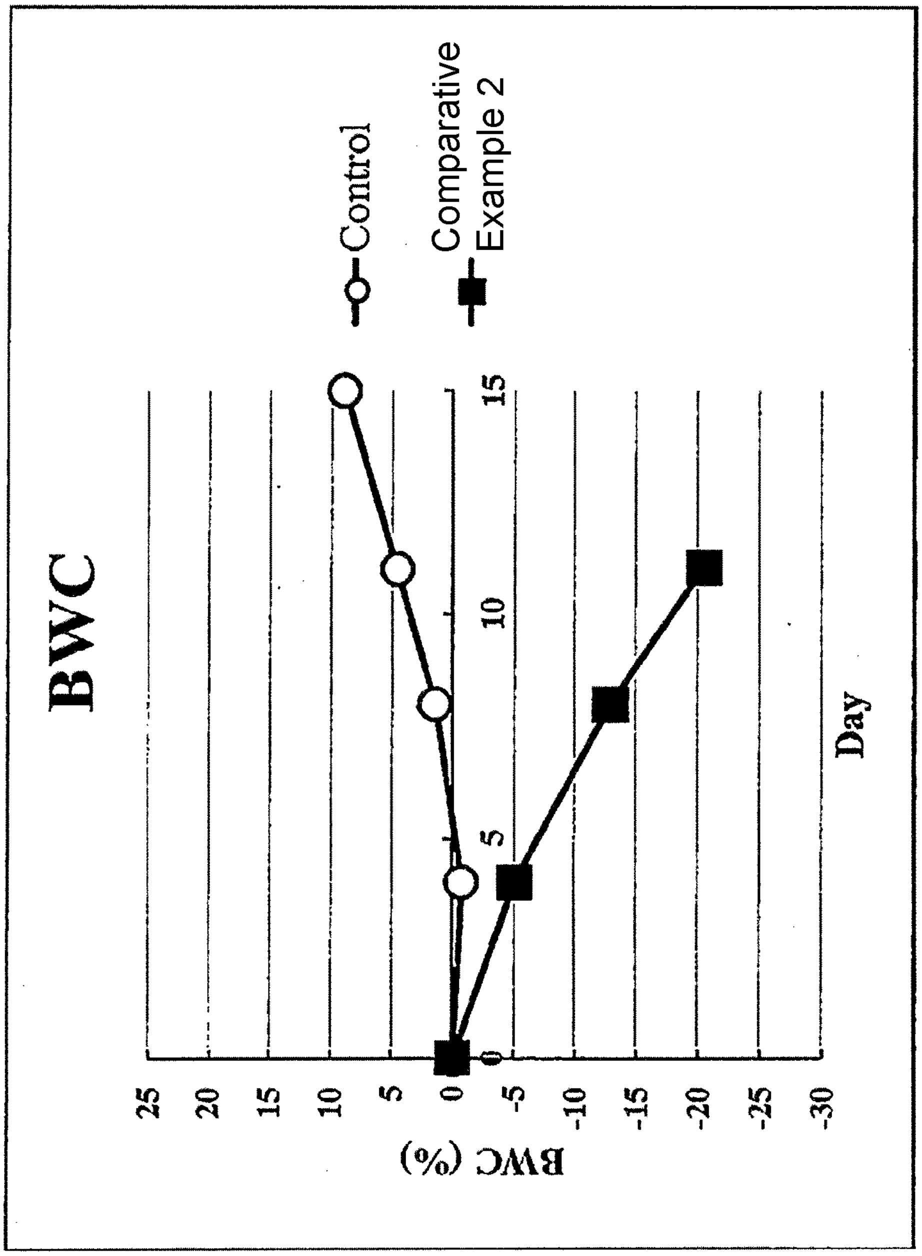
BWC
BWC (%)
25
20
15
10
5
0
-5
-10
-15
-20
-25
-30
0
5
10
15
Day
Control
Comparative Example 2

Fig. 6

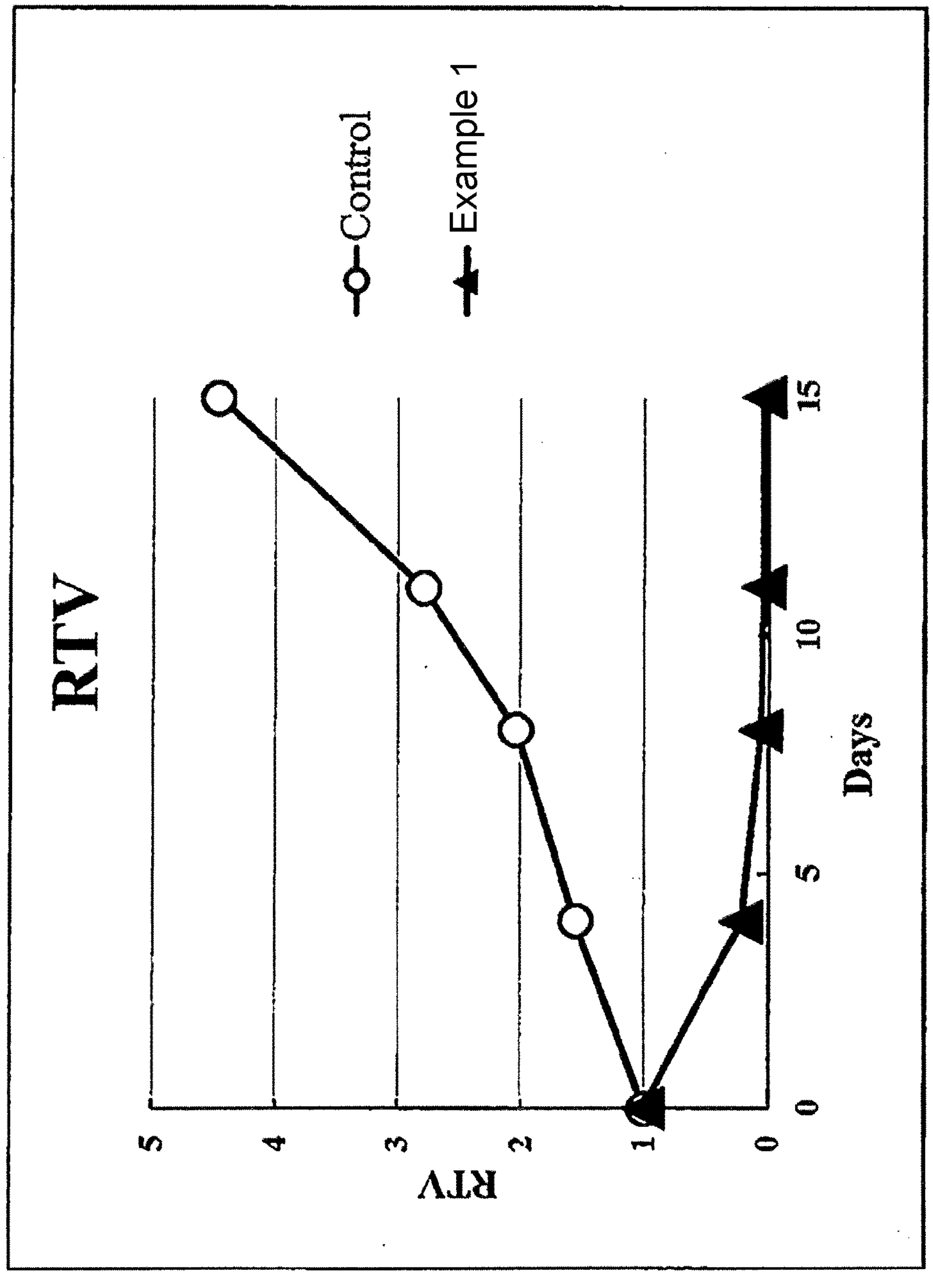

Fig. 7

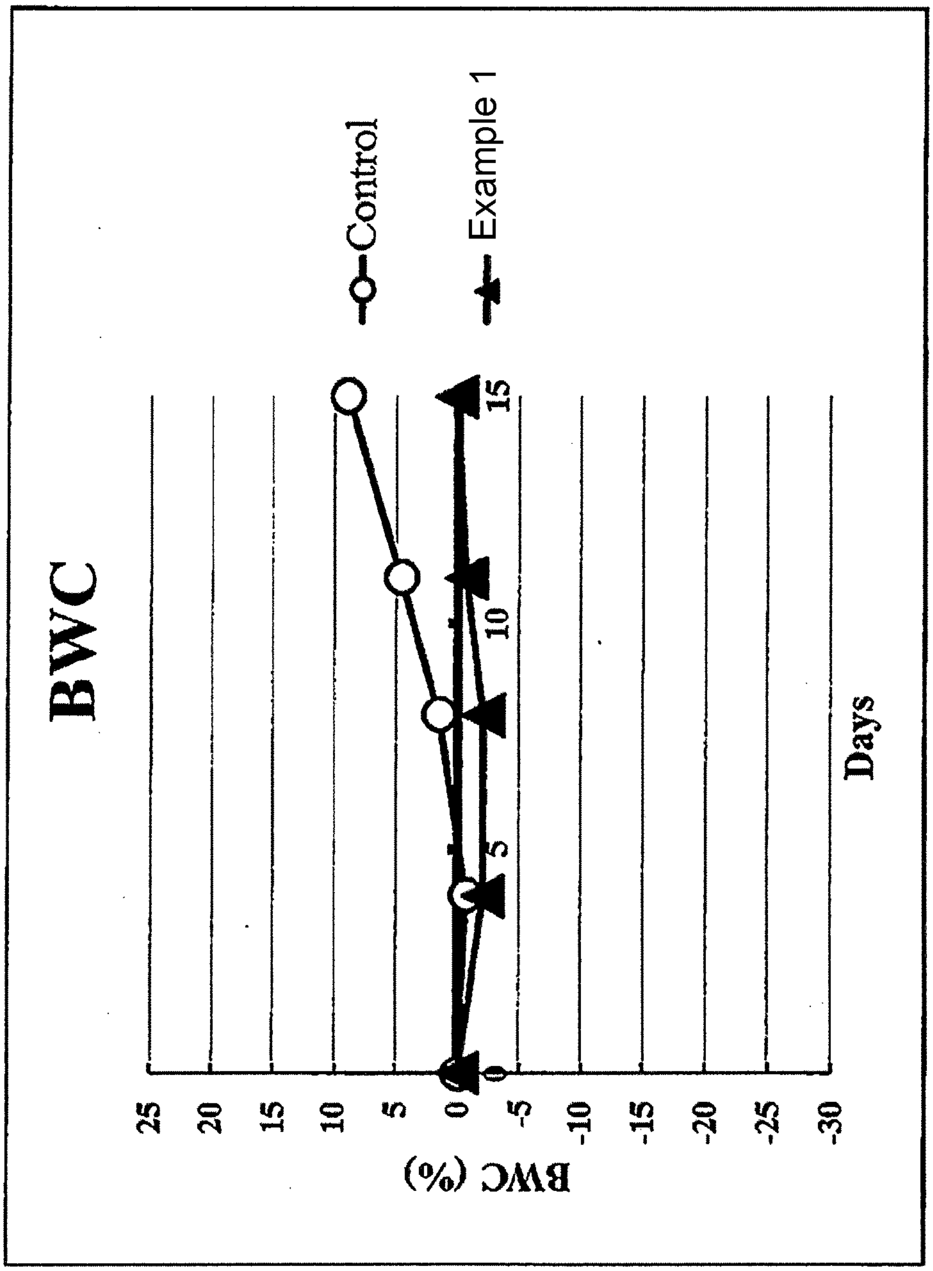
BWC
Control
Example 1
BWC (%)
25
20
15
10
5
0
-5
-10
-15
-20
-25
-30
0
5
10
15
Days

Fig. 8

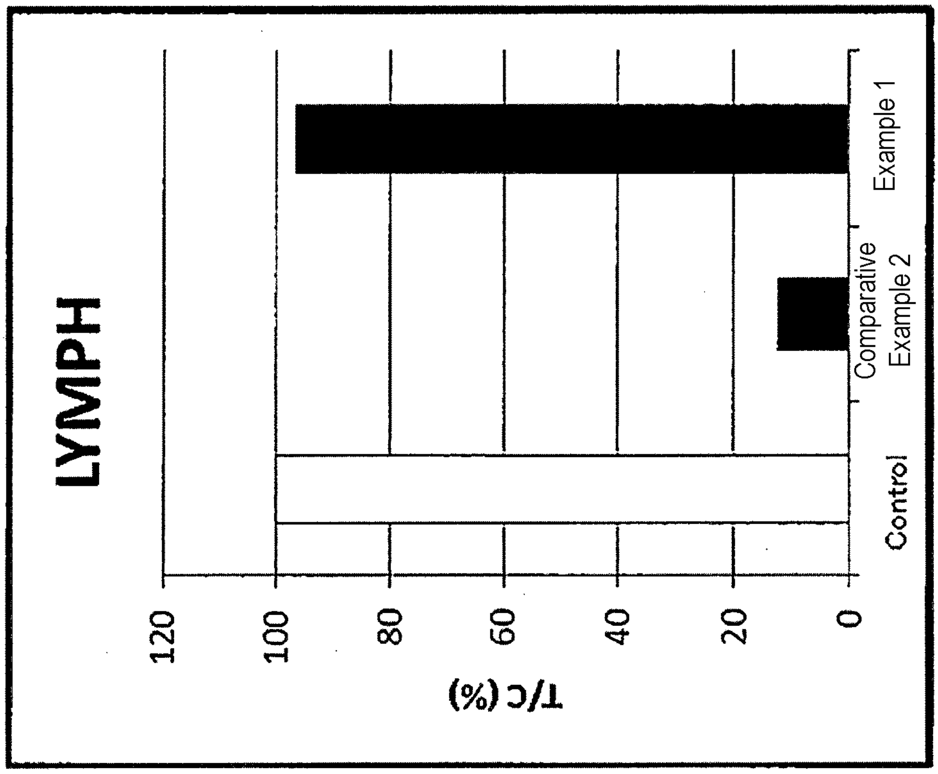
LYMPH
T/C (%)
120
100
80
60
40
20
0
Control
Comparative Example 2
Example 1

Fig. 9

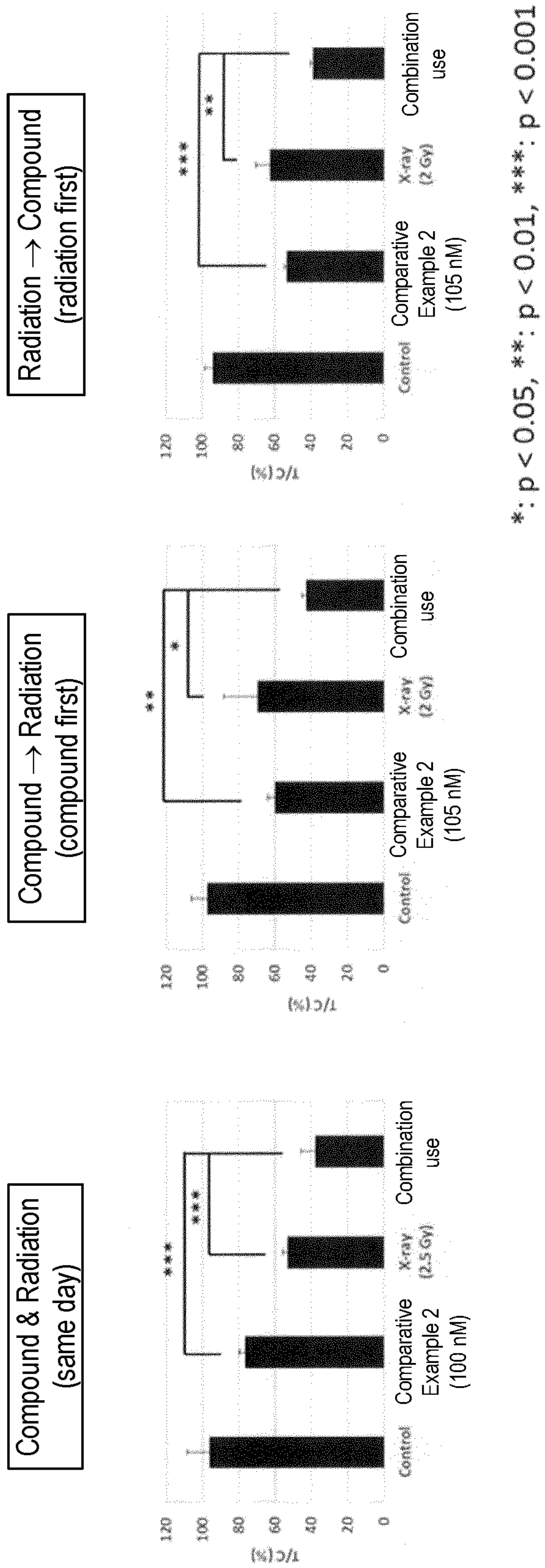
Radiation → Compound (radiation first)
Compound → Radiation (compound first)
Compound & Radiation (same day)
T/C (%)
120
100
80
60
40
20
0
Control
Comparative Example 2 (105 nM)
Comparative Example 2 (100 nM)
X-ray (2 Gy)
X-ray (2.5 Gy)
Combination use
*
**
***
*: p < 0.05, **: p < 0.01, ***: p < 0.001

Fig. 10

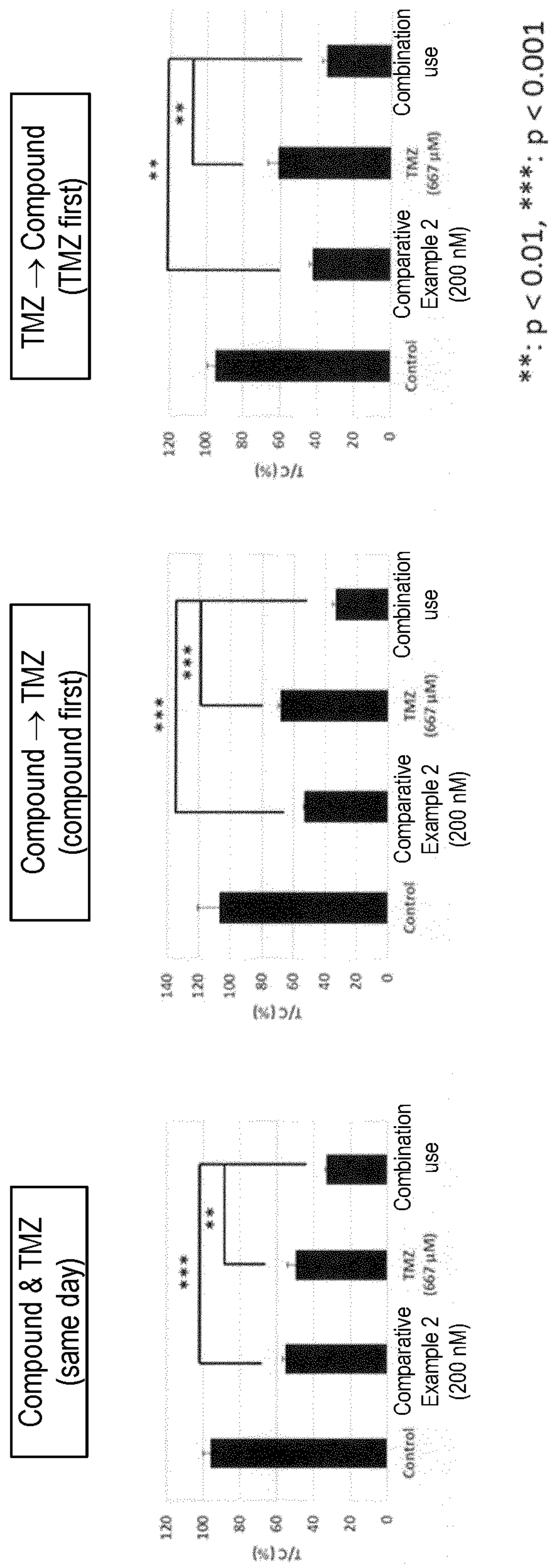
TMZ → Compound
(TMZ first)
Compound → TMZ
(compound first)
Compound & TMZ
(same day)
T/C (%)
Control
Comparative Example 2 (200 nM)
TMZ (667 μM)
Combination use
**: p < 0.01, ***: p < 0.001

## INTERNATIONAL SEARCH REPORT

International application No. PCT/JP2020/023661

A. CLASSIFICATION OF SUBJECT MATTER
A61P 35/00(2006.01)i; C07H 19/14(2006.01)i; A61K 31/7064(2006.01)i
FI: C07H19/14 CSP; A61P35/00; A61K31/7064
According to International Patent Classification (IPC) or to both national classification and IPC

B. FIELDS SEARCHED

Minimum documentation searched (classification system followed by classification symbols)
A61P35/00; C07H19/14; A61K31/7064

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | Synthesis, 2006, (12), 2005-2012, DOI 10.1055/s-2006-942400 in particular, compound 1b | 21<br>1-62 |
| X<br>Y | Acta Crystallographica, Section C: Crystal Structure Communications, 2006, C62(4), o231-o233, DOI 10.1107/S0108270106007633 in particular, compound I | 21<br>1-62 |
| X<br>Y | Acta Crystallographica, Section E: Structure Reports Online, 2014, 70(2), o120, sup-1 to 6, DOI 10.1107/S1600536813034995 in particular, Structure Report and Related literature | 21<br>1-62 |
| X<br>Y | Organic & Biomolecular Chemistry, 2008, 6(19), 3552-3560, DOI 10.1039/b806145a in particular, compound 9 | 21<br>1-62 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 August 2020 (24.08.2020) | 08 September 2020 (08.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/023661

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Bioorganic & Medicinal Chemistry, 2012, 20(17), 5202-5214, DOI 10.1016/j.bmc.2012.07.003 in particular, compound 11 | 23<br>1-62 |
| X<br>Y | Nucleosides, Nucleotides & Nucleic Acids, 2005, 24(5-7), 847-850, DOI 10.1081/NCN-200059181 in particular, compound 3b | 23<br>1-62 |
| X<br>Y | WO 2014/124430 A1 (EMORY UNIVERSITY) 14.08.2014 (2014-08-14) in particular, page 129 EFNS-01580 | 23<br>1-62 |
| Y<br>A | LARSEN, C. S. et al., Chapter 14 Design and application of prodrugs, Textbook of Drug Design and Discovery, Third edition, 2002, 460-514 in particular, page 479 | 1-22, 31-62<br>23-30 |
| Y<br>A | JP 2009-543884 A (ANADYS PHARMACEUTICALS, INC.) 10.12.2009 (2009-12-10) in particular, Title of invention, claims | 1-22, 31-62<br>23-30 |
| Y<br>A | WO 2018/085307 A1 (WU, L. I.) 11.05.2018 (2018-05-11) in particular, Title of invention, claims | 1-22, 31-62<br>23-30 |
| Y<br>A | WO 2004/041203 A2 (XENOPORT, INC.) 21.05.2004 (2004-05-21) in particular, page 72, compound 29 | 1-22, 31-62<br>23-30 |

Form PCT/ISA/210 (continuation of second sheet) (January 2015)

**INTERNATIONAL SEARCH REPORT**

International application No.
PCT/JP2020/023661

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

International application No.
PCT/JP2020/023661

<Continuation of Box No. III>

Document 1: Synthesis, 2006, (12), 2005-2012, DOI 10.1055/s-2006-942400
Document 2: Acta Crystallographica, Section C: Crystal Structure Communications, 2006, C62(4), o231-o233, DOI 10.1107/S0108270106007633
Document 3: Acta Crystallographica, Section E: Structure Reports Online, 2014, 70(2), o120, sup-1 to 6, DOI 10.1107/S1600536813034995

(Invention 1) Claims 1-22 and parts of claims 31-62 dependent on claims 1-9

Claims 1-22 have the special technical feature of a "compound represented by general formula (1) or a pharmaceutically acceptable salt thereof", and are thus classified as invention 1.

In addition, the parts of claims 31-62 which are dependent on claims 1-9 and pertain to the "compound represented by general formula (1) or a pharmaceutically acceptable salt thereof" are classified as invention 1.

(Invention 2) Claims 23-30 and parts of claims 31-62 dependent on claim 23

Claims 23-30 pertain to a "compound represented by general formula (2) or a pharmaceutically acceptable salt thereof", are common to claim 1 classified as invention 1 in terms of a predetermined nucleoside structure up to oxygen atoms at a 5'-position and a 3'-position, and can be said to share the common technical feature.

However, said technical feature does not make a contribution over the prior art in light of the disclosures of documents 1-3 and thus cannot be said to be a special technical feature. Moreover, there do not exist other identical or corresponding special technical features between these inventions.

Furthermore, claims 23-30 are not dependent on claim 1. Moreover, claims 23-30 are not substantially identical or equivalent to any of the claims classified as invention 1.

Therefore, claims 23-30 cannot be classified as invention 1.

In addition, the parts of claims 31-62 which are dependent on claim 23 and pertain to the "compound represented by general formula (2) or a pharmaceutically acceptable salt thereof" are classified as invention 2.

Accordingly, the inventions are classified into two as follows.

(Invention 1) Claims 1-22 and parts of claims 31-62 dependent on claims 1-9
(Invention 2) Claims 23-30 and parts of claims 31-62 dependent on claim 23

**INTERNATIONAL SEARCH REPORT**
Information on patent family members

International application No.
PCT/JP2020/023661

| Patent Documents referred in the Report | Publication Date | Patent Family | Publication Date |
|---|---|---|---|
| WO 2014/124430 A1 | 14 Aug. 2014 | (Family: none) | |
| JP 2009-543884 A | 10 Dec. 2009 | US 2008/0020989 A1<br>in particular, Title of invention, claims<br>WO 2008/011406 A2<br>EP 2040712 A2 | |
| WO 2018/085307 A1 | 11 May 2018 | JP 2019-533727 A<br>in particular, Title of invention, claims<br>EP 3529254 A1<br>CN 110325542 A | |
| WO 2004/041203 A2 | 21 May 2004 | US 2004/0142857 A1<br>in particular, page 31, compound 29<br>EP 1567169 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004041203 A
- WO 2013009735 A


### Non-patent literature cited in the description


- *Drug Metab Dispos.,* November 2002, vol. 30 (11), 1221-1229
- *J. Org. Chem.,* 1999, vol. 64 (22), 8319-8322
- *Acta Cryst.,* 2014, vol. E70, o120
- *ChemMedChem,* 2013, vol. 8, 832-846
- *Bioorg. Med. Chem,* 2012, vol. 20, 5202-5214
- *J Med Chem,* 2016, vol. 59 (14), 6860-6877
- *Synthesis,* 1992, vol. 5, 477-481
- **GREENE ; WUTS.** Protective Groups in Organic Synthesis. John Wiley & Sons Inc, 2014
- *LABIO,* October 2007, vol. 21 (30), 27